# EUROPEAN PATENT APPLICATION

(11) **EP 2 815 765 A1**
(43) Date of publication of application: **24.12.2014**
(21) Application number: 14166702.2
(22) Date of filing: 15.03.2012
(51) Int. Cl.: A61K 39/395, A61K 31/00, A61K 33/00, C07K 16/32

(54) **Overcoming resistance to ERBB pathway inhibitors**

(30) Priority: 15.03.2011 US 201161452976 P; 15.03.2011 US 201161452974 P
(62) Divisional of application: 12714108.3
(71) Applicant: Merrimack Pharmaceuticals, Inc., Cambridge, MA 02139 (US)
(72) Inventor: Garcia, Gabriela, Roslindale, MA 02131 (US); Kubasek, William, Belmont, MA 02478 (US); Lahdenranta, Maria Johanna, Cambridge, MA 02138 (US); Macbeath, Gavin, Wakefield, MA 01880 (US); McDonagh, Charlotte, Winchester, MA 01890 (US); Moyo, Victor, Ringoes, MA 08551 (US); Onsum, Matthew David, San Francisco, CA 94158 (US); Sevecka, Mark, Cambridge, MA 02139 (US); Wainszelbaum, Marisa, Boston, MA 02114 (US); Zhang, Bo, Lynnfield, MA 01940 (US); Schoeberl, Birgit, Cambridge, MA 02139 (US)
(74) Representative: Tuxworth, Pamela M.

(57) **Abstract**

Provided are methods for overcoming resistance to an ErbB pathway inhibitor, such as an EGFR inhibitor or a HER2 inhibitor. The resistance may be acquired resistance to an EGFR inhibitor, such as acquired resistance to gefitinib. In the methods provided, a subject exhibiting resistance to an ErbB pathway inhibitor is selected and both an ErbB 3 inhibitor and a second ErbB pathway inhibitor are administered to the subject, such as an EGFR inhibitor or a HER2 inhibitor. Also provided are methods for inhibiting the growth of a tumor comprising a T790M EGFR mutation by contacting the tumor with an ErbB3 inhibitor and an EGFR inhibitor. Compositions for overcoming resistance to an ErbB pathway inhibitor, comprising both an ErbB 3 inhibitor and a second ErbB pathway inhibitor, such as an EGFR inhibitor or a HER2 inhibitor, are also provided.

## Description

### Background

The ERBB family of receptor tyrosine kinases include EGFR (HER1), HER2 (ErbB2), ERBB3 (HER3), and ERBB4 (HER4). Over the past ten years, it has become evident that many epithelial cancers require EGFR or HER2 signaling for their growth and survival. Agents targeting EGFR have become widely used for the treatment of cancer such as lung cancer, colorectal cancer, head and neck cancer and, typically in combination with gemcitabine, pancreatic cancer. For example, small molecule tyrosine kinase inhibitors (TKIs) that downregulate the EGFR signaling pathway have been developed, such as gefitinib (Iressa®), which is indicated for the treatment of non-small cell lung cancer, erlotinib (Tarceva®), which is indicated for the treatment of non-small cell lung cancer and pancreatic cancer, and lapatinib (Tykerb®), which is indicated for the treatment of HER2-positive breast cancer. Furthermore, antibodies specific for EGFR have been developed, such as the humanized monoclonal antibody cetuximab (Erbitux®), which is indicated for the treatment of colorectal cancer and head and neck cancer. Agents targeting HER2 also have become widely used for the treatment of cancer such as breast cancer. An example of an agent targeting HER2 is the humanized anti-HER2 monoclonal antibody trastuzumab (Herceptin®), which is used around the world for the treatment of HER2 overexpressing breast cancer.

Recent studies have found that cancers that are sensitive to EGFR inhibitors are unique in that phosphoinositide 3-kinase (PI3K) signaling is under the sole control of EGFR. For the EGFR inhibitors to be effective, they must cause downregulation of the PI3K/AKT pathway (Bianco, R. et al. (2003) Oncogene 22:2812-2822). While patients with EGFR-driven cancers often initially respond well to EGFR-targeted therapies, over time many patients that were initially responsive suffer from recurrence and develop tumors refractory to the original treatment. Furthermore, certain EGFR-positive cancers exhibit a predisposition to resistance to EGFR-targeted therapies. One way in which such resistance has been observed to develop is through mutation of EGFR. The "T790M EGFR mutation" comprises and is identified by a change of a threonine, which is present at position 790 of wild-type EGFR, to a methionine. This mutation, which locates to the kinase domain of EGFR, has been described, *e.g*., by Kobayashi, S. et al. (2005) N. Engl. J. Med. 352:786-792; and Pao, W. et al. (2005) PLoS Med. 2:225-235.

Persistent activation of the PI3K signaling pathway through ErbB3 also has been associated with gefitinib resistance (Engelman *et al.* (2005), *supra*; Engelman *et al.* (2007) *supra*).

Resistance to HER2 inhibitors, such as trastuzumab and lapatinib, also has been reported. Similar to EGFR inhibitor resistance, persistent activation of the PI3K/AKT signaling pathway is at least one of the mechanisms reported for the acquired resistance to HER2 inhibitors.

Studies have identified ErbB3, which is a kinase-dead member of the ErbB family, as an activator of PI3K/AKT signaling in EGFR dependent cancers (Engelman, J.A. et al. (2005) Proc. Natl. Acad. Sci. USA 102:3788-3793; Engelman, J.A. et al. (2007) Science 316:1039-1045; In these cells, ErbB3 is tyrosine phosphorylated in an EGFR-dependent manner and then directly binds PI3K. Upon inhibition of EGFR, ErbB3 phosphorylation is lost, it no longer binds PI3K and there is loss of PI3K/AKT signaling (Engelman, J.A. et al. (2005) Proc. Natl. Acad. Sci. USA 102:3788-3793; Engelman, J.A. et al. (2007) Science 316:1039-1045). Furthermore, downregulation of ErbB3 using short hairpin RNA (shRNA) leads to a decrease in AKT phosphorylation in EGFR dependent cancers (Engelman, J.A. et al. (2005) Proc. Natl. Acad. Sci. USA 102:3788-3793). Similarly, ErbB3 is a major activator of PI3K in HER2 amplified breast cancers. Trastuzumab treatment leads to loss of ErbB3 phosphorylation, dissociation between ErbB3 and PI3K and loss of AKT phosphorylation in these cancers. Thus, signaling through ErbB3 is thought to be a major mechanism of PI3K/AKT activation in both EGFR and HER2 driven cancers.

There are also examples of resistance that implicate EGFR, HER2 and MET in reactivating ErbB3 (Engelman, J.A. et al. (2006) J. Clin. Invest. 116:2695-2706; Engelman, J.A. et al. (2007) Science 316:1039-1045; Ritter, C.A. et al. (2007) Clin. Cancer Res. 13:4909-4919; Sergina, N.V. et al. (2007) Nature 445:437-441). In addition, heregulin-induced activation of HER2-ErbB3 heterodimers has also been associated with resistance to EGFR inhibitors (Zhou, B.B. et al. (2006) Cancer Cell 10:39-50).

In view of the foregoing, compositions and methods for overcoming resistance to ErbB pathway inhibitors, including EGFR inhibitors (such as TKIs and anti-EGFR antibodies) and HER2 inhibitors (such as TKIs and anti-HER2 antibodies), are being actively sought, as they promise to extend or restore the effectiveness of targeted cancer therapies.

### Summary

Methods are provided for overcoming resistance to ErbB pathway inhibitors, as well as pharmaceutical compositions that can be used in the practice of such methods. The methods and compositions provided herein are based, at least in part, on the discovery by the inventors that use of an ErbB3 inhibitor in combination with an ErbB pathway inhibitor can overcome tumor resistance to an ErbB pathway inhibitor. For example, use of a bispecific anti-ErbB3, anti-ErbB2 antibody (or an anti-ErbB3 antibody) in combination with an anti-EGFR antibody has been demonstrated to overcome acquired resistance *in vivo* to the small molecule EGFR inhibitor gefitinib.

Accordingly, in one aspect, a method for overcoming or preventing resistance of a tumor to an ErbB pathway inhibitor in a subject is provided, the method comprising:
selecting a subject with a tumor exhibiting, or at risk for developing, resistance to an ErbB pathway inhibitor; and
administering to the subject (i) an ErbB3 inhibitor and (ii) an ErbB pathway inhibitor.

The ErbB pathway inhibitor administered to the subject does not need to be the same ErbB pathway inhibitor to which the subject is resistant, although typically, the ErbB pathway inhibitor administered to the subject will be directed against the same ErbB pathway as the ErbB pathway inhibitor to which the subject is resistant. For example, a subject who exhibits resistance to the EGFR pathway inhibitor gefitinib may be co-administered an ErbB3 inhibitor and an EGFR inhibitor, which EGFR inhibitor can be, for example, gefitinib or the anti-EGFR antibody cetuximab.

In certain embodiments, the resistance exhibited by the subject to the ErbB pathway inhibitor is acquired resistance. In one embodiment, the acquired resistance is acquired resistance to an EGFR inhibitor, such as acquired resistance wherein the EGFR in the tumor comprises tumor cells comprising an EGFR gene comprising a T790M EGFR mutation. In another embodiment the tumor comprises tumor cells comprising a KRAS gene comprising at least one KRAS mutation, e.g., a G12S, G12C, or G12V KRAS mutation or a Q61R KRAS mutation. In one embodiment, the acquired resistance is resistance to gefitinib. In another embodiment, the acquired resistance is acquired resistance to a HER2 inhibitor, such as acquired resistance to trastuzumab. In various embodiments, the resistance exhibited by the subject is associated with reactivation of PI3K/AKT signaling in tumor cells in the subject.

In one embodiment, the ErbB3 inhibitor administered to the subject is an anti-ErbB3 antibody. An exemplary anti-ErbB3 antibody is MM-121 (Ab #6), comprising V_{H} and V_{L} sequences as shown in SEQ ID NOs: 1 and 2, respectively. Alternately, the anti-ErbB3 antibody is an antibody that comprises the heavy and light chain CDRs of MM-121 (i.e., the anti-ErbB3 antibody comprises an antibody comprising V_{H} CDR1, 2 and 3 sequences as shown in SEQ ID NOs: 3-5, respectively, and V_{L} CDR1, 2 and 3 sequences as shown in SEQ ID NOs: 6-8, respectively). In another embodiment, the anti-ErbB3 antibody is an antibody having heavy and light chains comprising the amino acid sequences set forth in SEQ ID NOs 42 and 43, respectively. In other embodiments, the anti-ErbB3 antibody is Ab #3 (comprising V_{H} and V_{L} sequences as shown in SEQ ID NOs: 9 and 10, respectively), Ab #14 (comprising V_{H} and V_{L} sequences as shown in SEQ ID NOs: 17 and 18, respectively), Ab #17 (comprising V_{H} and V_{L} sequences as shown in SEQ ID NOs: 25 and 26, respectively) or Ab #19 (comprising V_{H} and V_{L} sequences as shown in SEQ ID NOs: 33 and 34, respectively). In still other embodiments, the anti-ErbB3 antibody is selected from mAb 1B4C3 or mAb 2D1D12 or humanized versions thereof, mAb 205.10 (e.g., mAb 205.10.1, mAb 205.10.2, or mAb 205.10.3) (Roche-Glycart), AMG-888 (U3-1287 -- U3 Pharma AG and Amgen), AV-203 (Aveo Pharmaceuticals) and humanized mAb 8B8. Typically, the ErbB3 inhibitor inhibits PI3K/AKT signaling.

In one embodiment, the ErbB3 inhibitor administered to the subject is a bispecific anti-ErbB3, anti-ErbB2 antibody such as MM-111, which comprises two scFvs in a Human Serum Albumin (HSA) conjugate as set forth in SEQ ID NO:44. MM-111 abrogates heregulin binding to ErbB2/ErbB3 and inhibits heregulin activation of ErbB2/ErbB3 without significantly affecting ErbB2 biological activity. A number of bispecific anti-ErbB2/antiErbB3 antibodies that are scFv HSA conjugates, including MM-111 (also referred to as B2B3-1), as well as A5-HSA-ML3.9, A5-HSA-B1D2, B12-HSA-B1D2, A5-HSA-F5B6H2, H3-HSA-F5B6H2, and F4-HSA-F5B6H2, are described in co-pending U.S. patent application Publication No. 20110059076, and PCT publication number WO2009/126920. Other suitable bispecific anti-ErbB2/antiErbB3 antibodies are disclosed and claimed in US Patent Nos. 7,332,580 and 7,332,585. MM-111 is currently undergoing clinical trials, including an open-label Phase 1-2 and pharmacologic study of MM-111 in patients with advanced, refractory HER2 positive cancers, and an open-label Phase 1-2 trial of MM-111 in combination with trastuzumab (Herceptin®) in patients with advanced HER2 positive breast cancer. In certain embodiments, the ErbB3 inhibitor inhibits PI3K/AKT signaling.

In one embodiment, the ErbB pathway inhibitor administered to the subject is an EGFR inhibitor. For example, the EGFR inhibitor can be an anti-EGFR antibody such as cetuximab. Other exemplary anti-EGFR antibodies are MM-151, Sym004, matuzumab, panitumumab, nimotuzumab and mAb 806.

In another embodiment, the EGFR inhibitor administered to the subject is a small molecule inhibitor of EGFR signaling such as gefitinib. Other exemplary small molecule inhibitors of EGFR signaling are afatinib, lapatinib, canertinib, erlotinib HCL, pelitinib, PKI-166, PD158780, and AG 1478.

In another embodiment, the ErbB pathway inhibitor administered to the subject is a HER2 inhibitor. For example, the HER2 inhibitor can be an anti-HER2 antibody such as trastuzumab. Alternatively, the HER2 inhibitor administered to the subject can be a small molecule inhibitor of HER2 signaling such as lapatinib.

In another aspect, a method of inhibiting growth of a tumor comprising cells comprising a T790M EGFR mutation cells is disclosed, the method comprising contacting the tumor with (i) an EGFR inhibitor; and (ii) an ErbB3 inhibitor. In a one embodiment, the ErbB3 inhibitor comprises ananti-ErbB3 antibody, such as one or more of the anti-ErbB3 antibodies set forth above. In another embodiment, the ErbB3 inhibitor comprises a bispecific anti-ErbB3, anti-ErbB2 antibody, such as one or more of the bispecific antibodies set forth above. In one embodiment, the ErbB3 inhibitor inhibits PI3K/AKT signaling. In another embodiment, the EGFR inhibitor comprises an anti-EGFR antibody, such as one or more of the antibodies set forth above. In yet another embodiment, the EGFR inhibitor comprises a small molecule inhibitor of EGFR signaling, such as one or more of the small molecule inhibitors set forth above.

In yet another aspect, a method of treating a tumor in a subject is provided, the method comprising
selecting a subject with a tumor that, by biopsy tests positive for a T790M EGFR mutation, and
administering to the subject (i) an EGFR inhibitor; and (ii) an ErbB3 inhibitor. In certain embodiments, the ErbB3 inhibitor administered to the subject comprises an anti-ErbB3 antibody, such as one or more of the anti-ErbB3 antibodies set forth above. In certain embodiments, the ErbB3 inhibitor administered to the subject comprises a bispecific anti-ErbB3, anti-ErbB2 antibody, such as one or more of the bispecific antibodies set forth above. Exemplary ErbB3 inhibitors inhibit PI3K/AKT signaling. In another embodiment, the EGFR inhibitor administered to the subject comprises an anti-EGFR antibody, such as one or more of the antibodies set forth above. In yet another embodiment, the EGFR inhibitor administered to the subject comprises a small molecule inhibitor of EGFR signaling, such as one or more of the small molecule inhibitors set forth above.

The compositions and methods provided herein can be used to inhibit growth, invasiveness or metastasis of a tumor, or treat a subject carrying a tumor that is resistant toErbB pathway inhibition. In one embodiment, the tumor is a lung cancer tumor, such as a non-small cell lung cancer (NSCLC) tumor, e.g., an adenocarcinoma NSCLC, a squamous cell carcinoma NSCLC, or a large cell carcinoma NSCLC. In other embodiments, the tumor can be a colorectal cancer, head and neck cancer, pancreatic cancer or breast cancer tumor.

In another aspect, pharmaceutical compositions for overcoming resistance to an ErbB pathway inhibitor are provided. The pharmaceutical compositions comprise one or more of an ErbB3 inhibitor as described above and an ErbB pathway inhibitor as described above. In one embodiment, the ErbB3 inhibitor and the ErbB pathway inhibitor are formulated with a pharmaceutical carrier into a single composition. In another embodiment, the ErbB3 inhibitor is formulated with a first pharmaceutical carrier to form a first composition, the ErbB pathway inhibitor is formulated with a second pharmaceutical carrier to form a second composition and the first and second composition are optionally packaged together.

### Brief Description of the Drawings

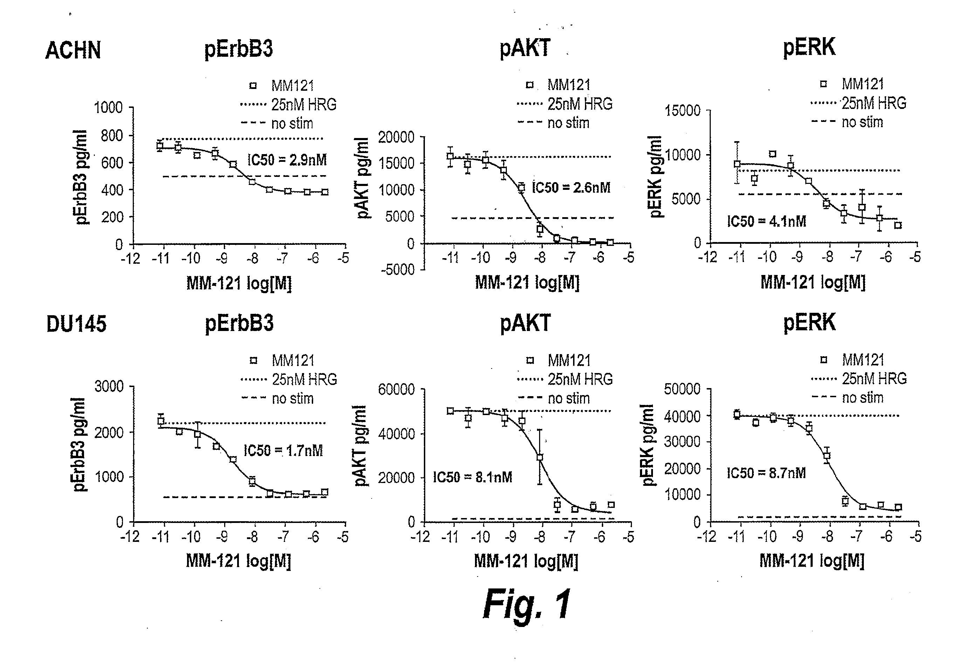

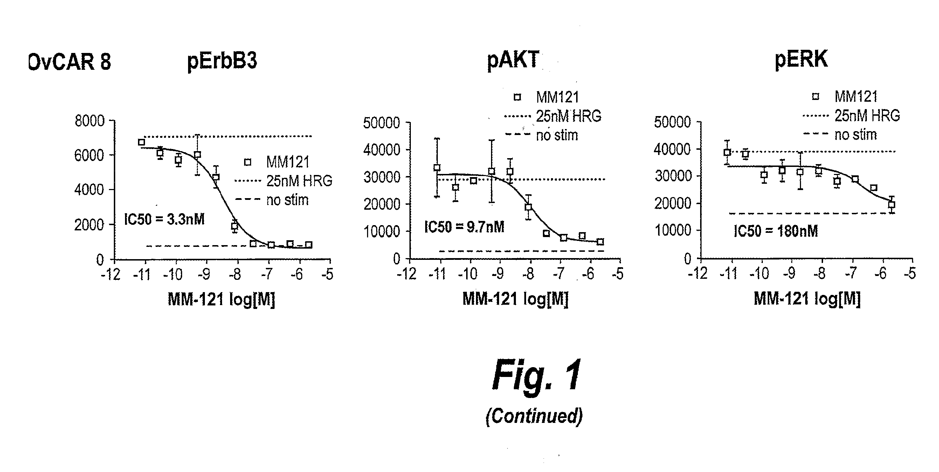

Figure 1 is a series of graphs of ELISA assay results, showing inhibition of heregulin-induced phosphorylation of ErbB3 (pErbB3), AKT (pAKT) and ERK (pERK) in the ACHN, DU145 and OvCAR8 cell lines by the anti-ErbB3 antibody MM-121. Data represent the mean ± SD of two separate experiments.

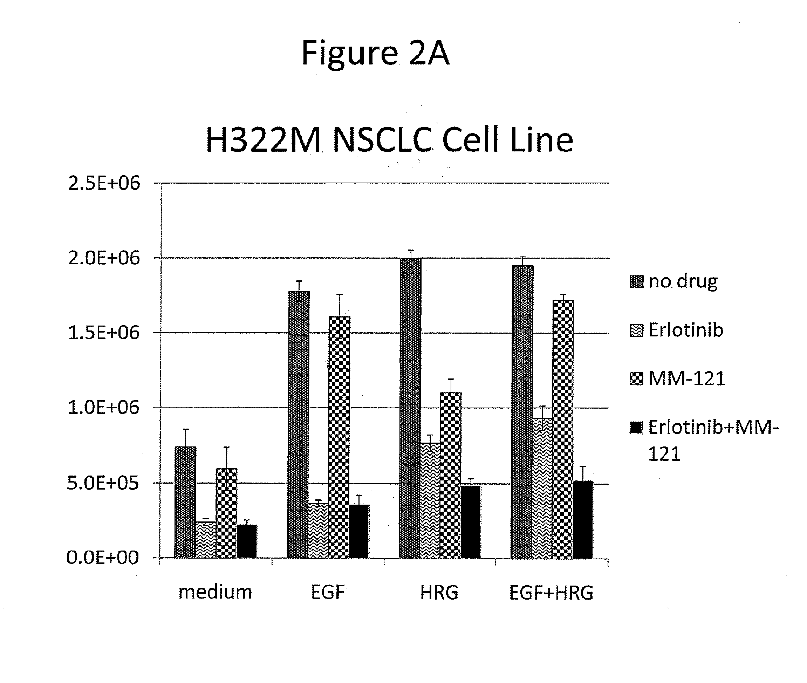

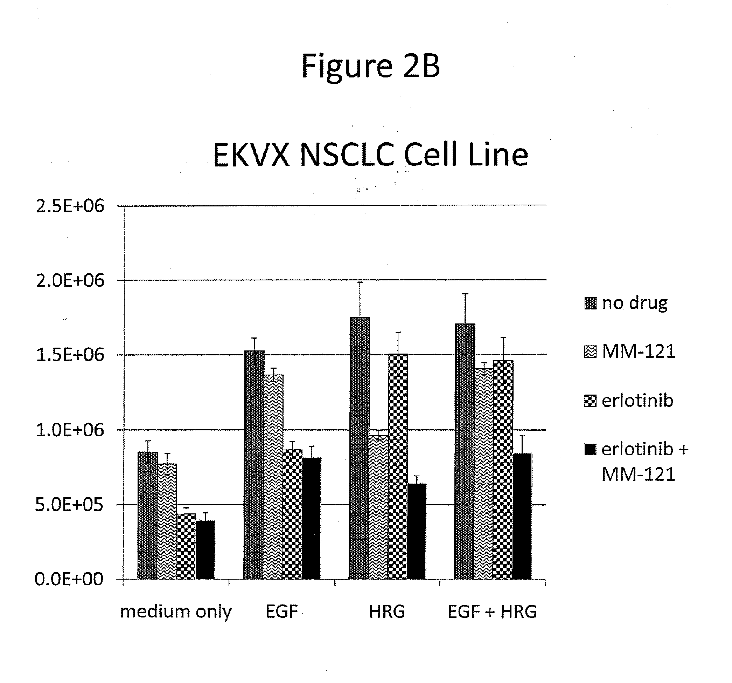

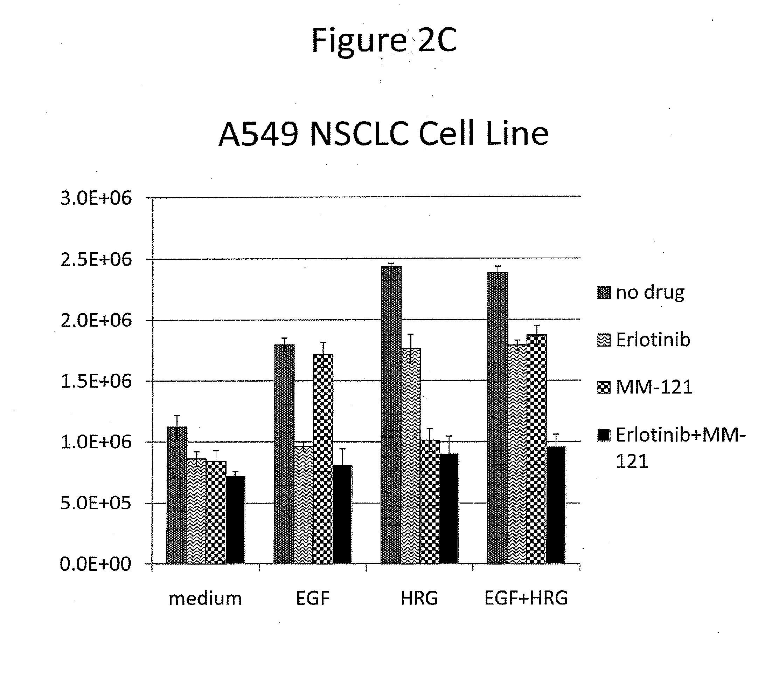

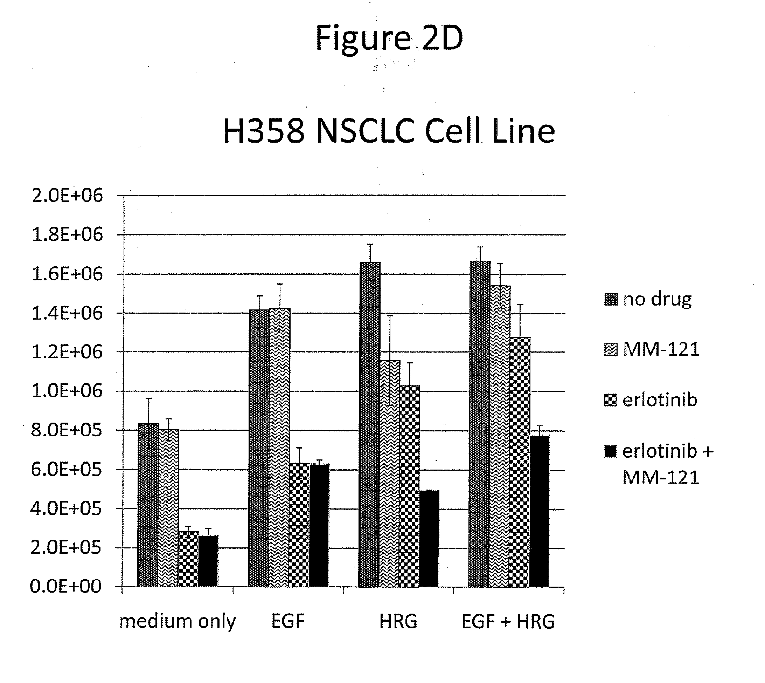

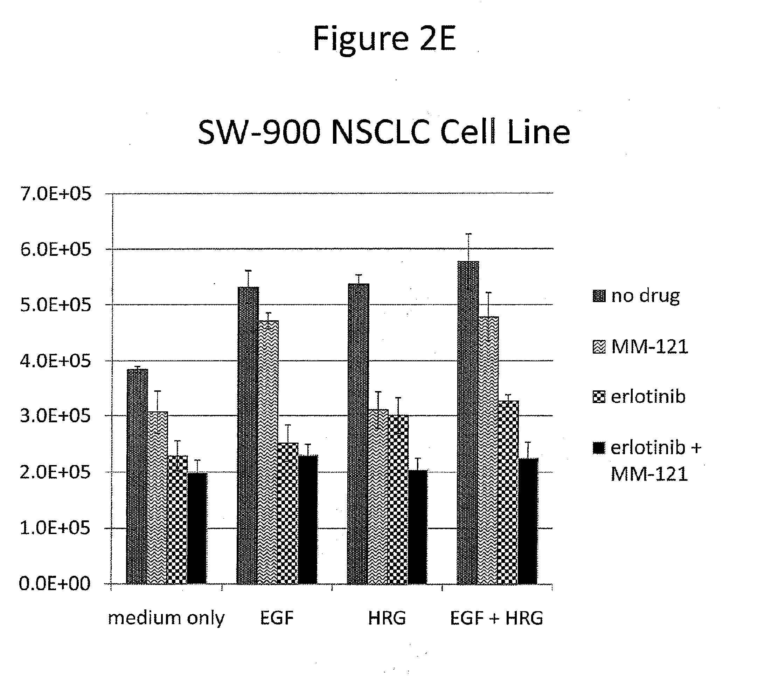

Figure 2 is a series of graphs showing spheroid assays using EGFR wild-type NSCLC cell lines. Spheroid cell cultures were treated with EGF (10 nM), heregulin1-β1 (HRG) (10 nM), both EGF and HRG (10 nM), or no exogenous ligands, as well as with erlotinib (1 µM), MM-121 (1 µM), a combination of the two (1 µM each), or in the absence of drugs. Cell lines tested include adenocarcinoma cell lines NCI-H322M, Figure 2A; EKVX, Figure 2B; A549, Figure 2C; H358, Figure 2D, and squamous cell line SW-900, Figure 2E. The y-axes represent relative live cell density.

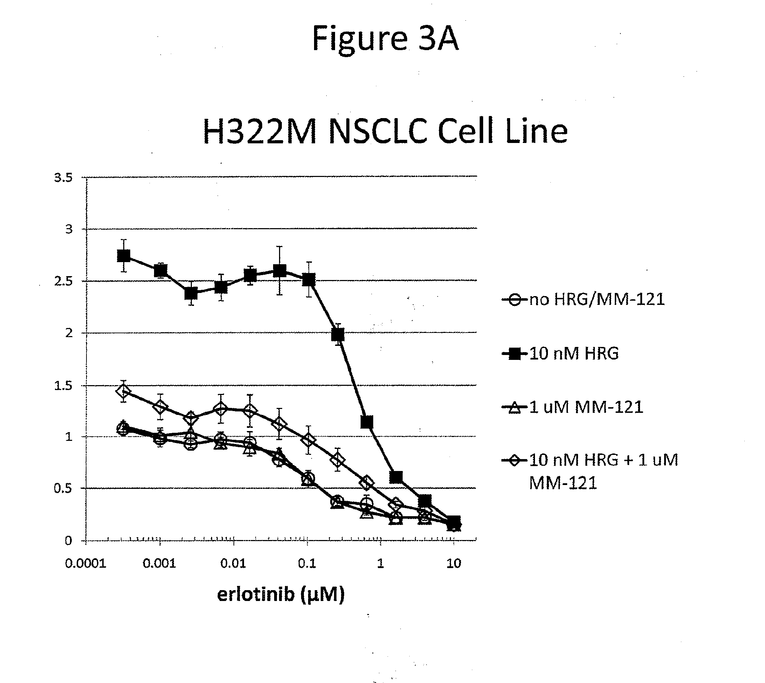

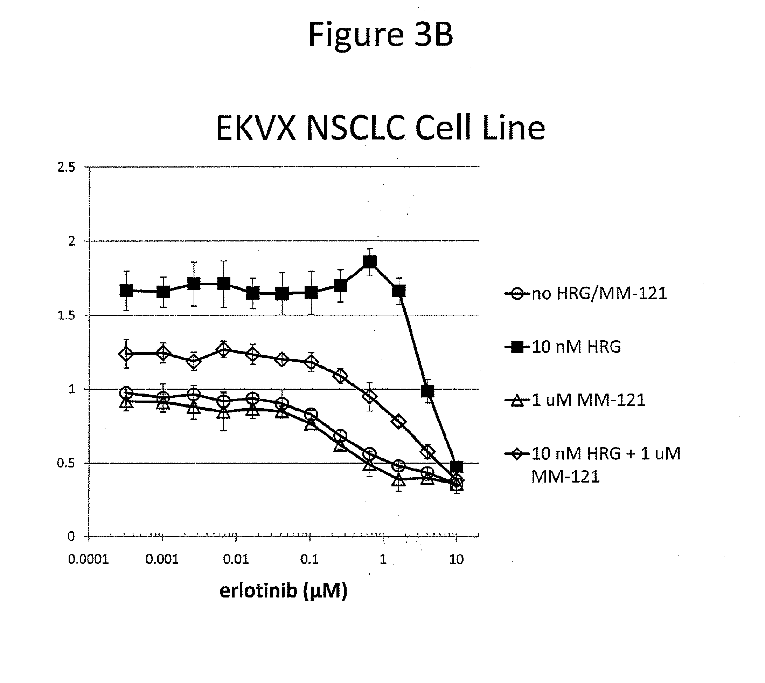

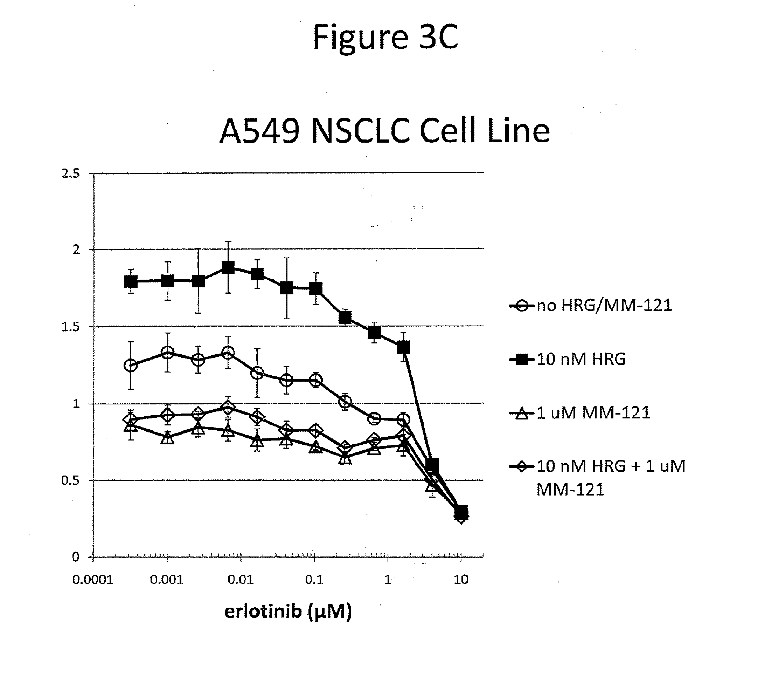

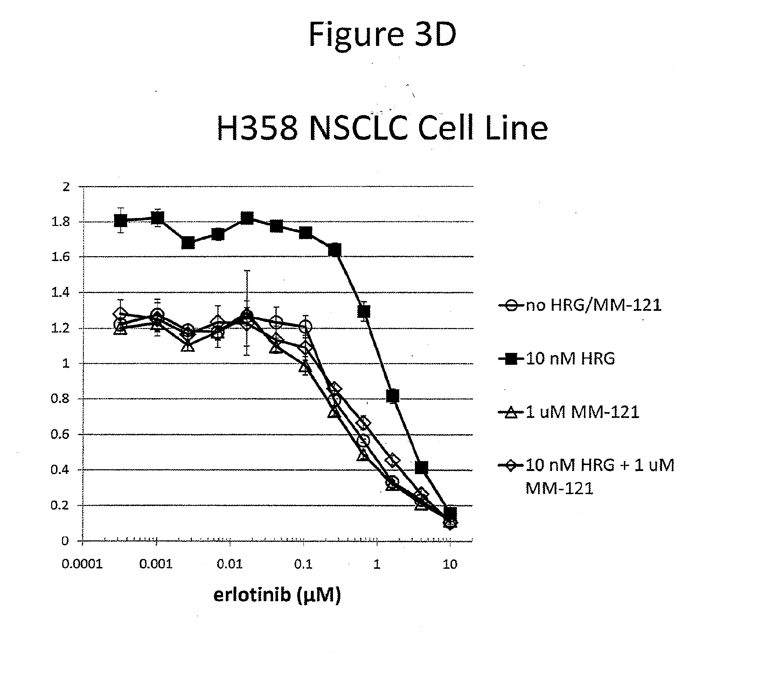

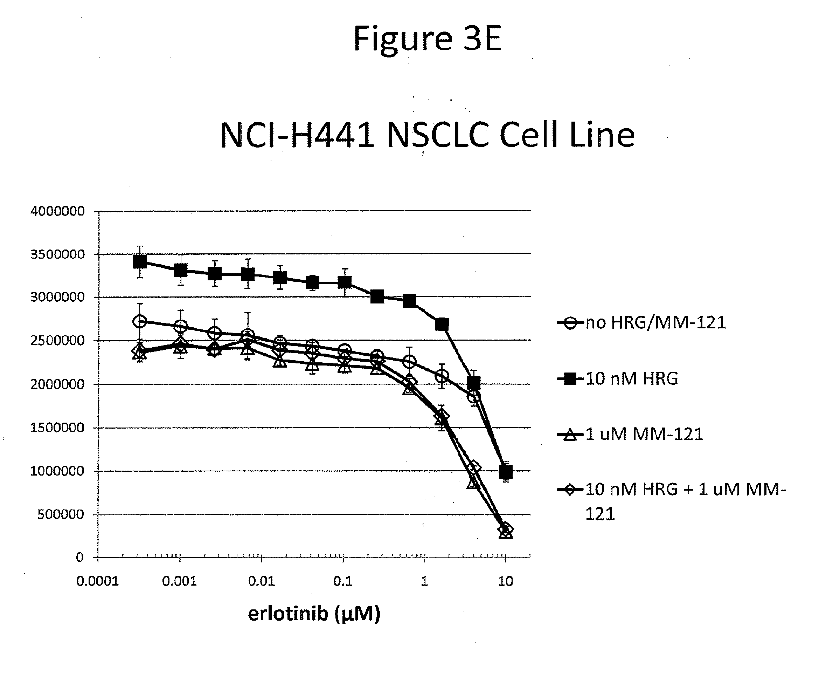

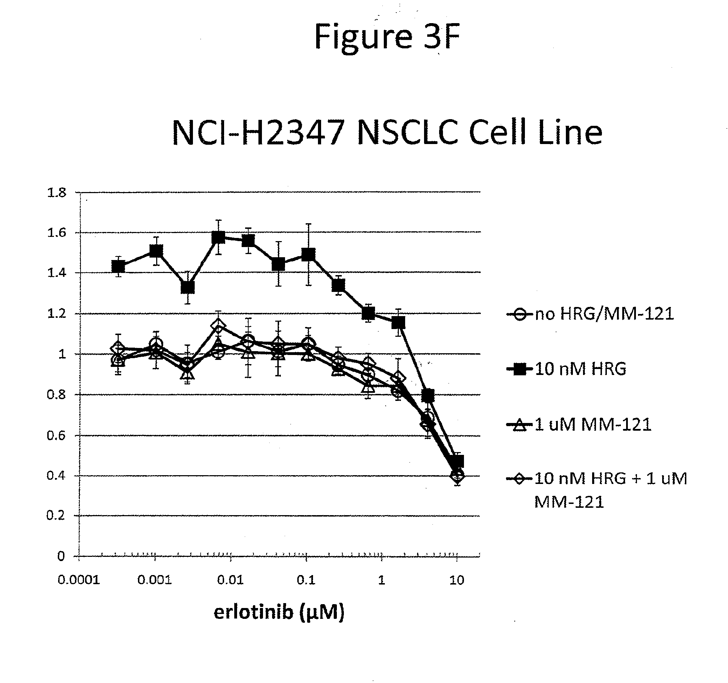

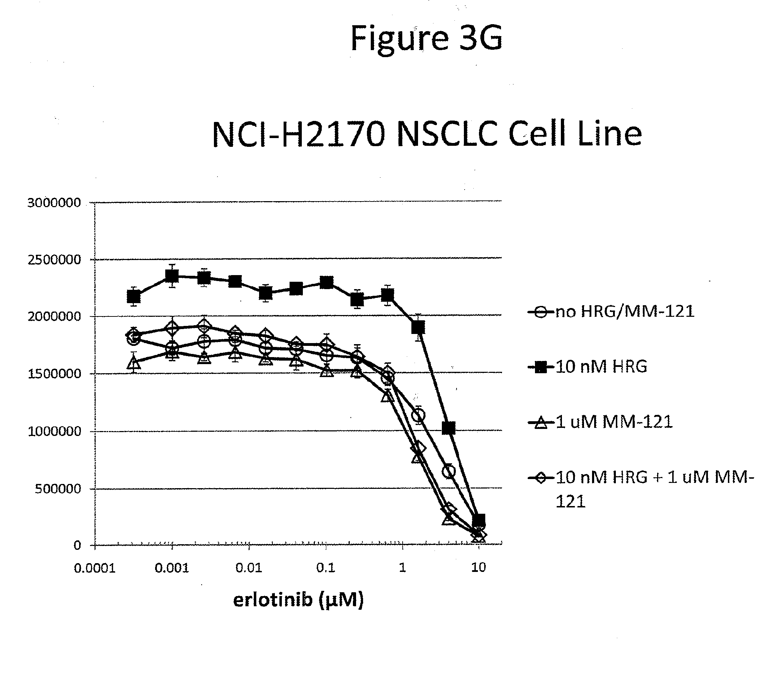

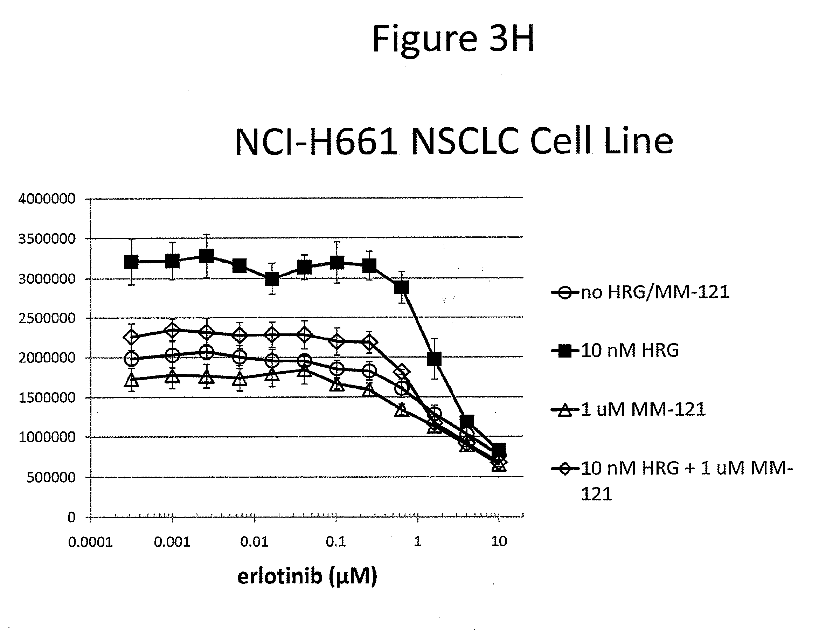

Figure 3 is a series of graphs showing spheroid assays using EGFR wild-type NSCLC cell lines. Spheroid cell cultures were treated with doses of erlotinib ranging from 0 to 10 µM, in either the absence or presence of heregulin-1β1(HRG), and in either the absence or presence of MM 121. Cells tested include the adenocarcinoma cell lines NCI-H322M (Figure 3A); EKVX (Figure 3B); A549 (Figure 3C); NCI-H358 (Figure 3D); NCI-H441 (Figure 3E), NCI-H2347 (Figure 3F); the squamous cell carcinoma cell line NCI-H2170 (Figure 3G), and the large cell carcinoma cell line NCI-H661 (Figure 3H). The y-axes represent relative live cell density.

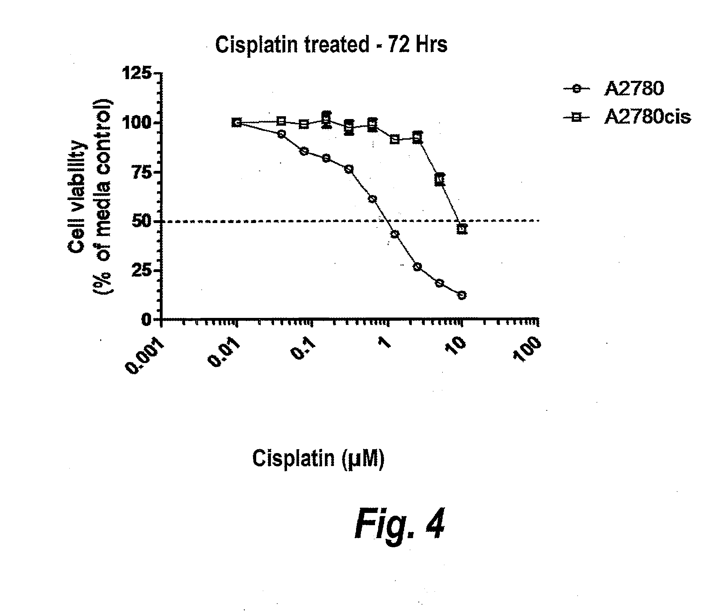

Figure 4 is a graph showing cell viability of cisplatin sensitive (A2780) and resistant (A2780cis) cells after treatment with cisplatin. Cell viability (Y-axis) is given as % viability of media control and is plotted against drug concentration in log µM.

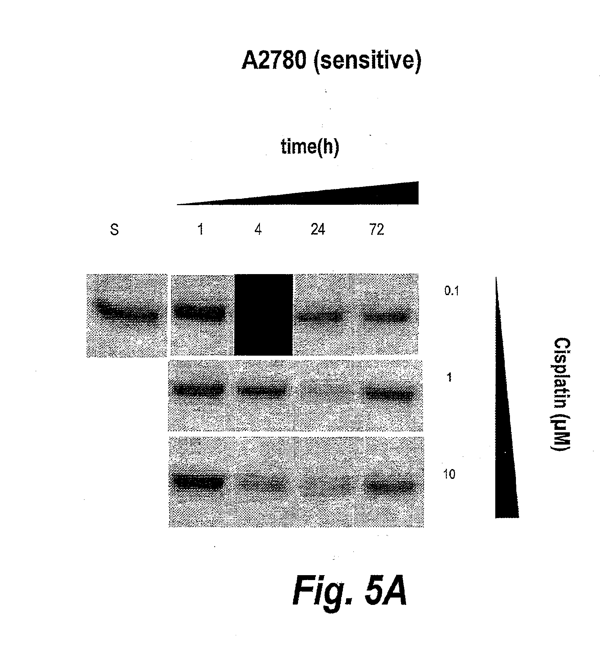

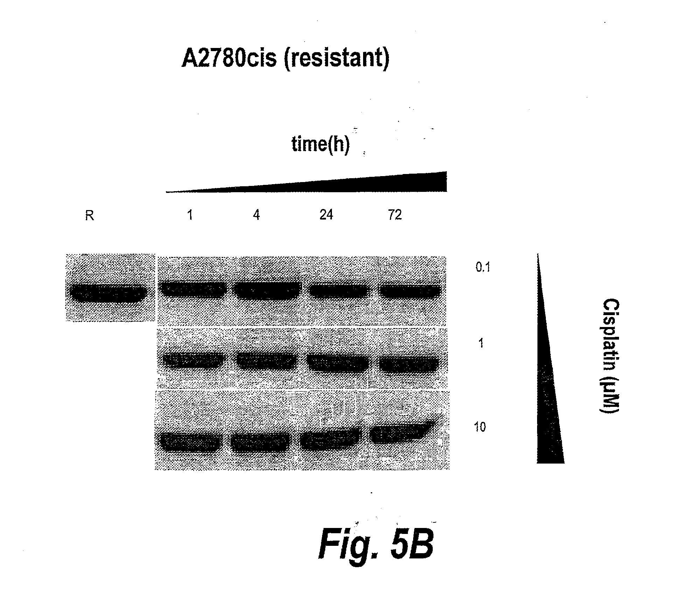

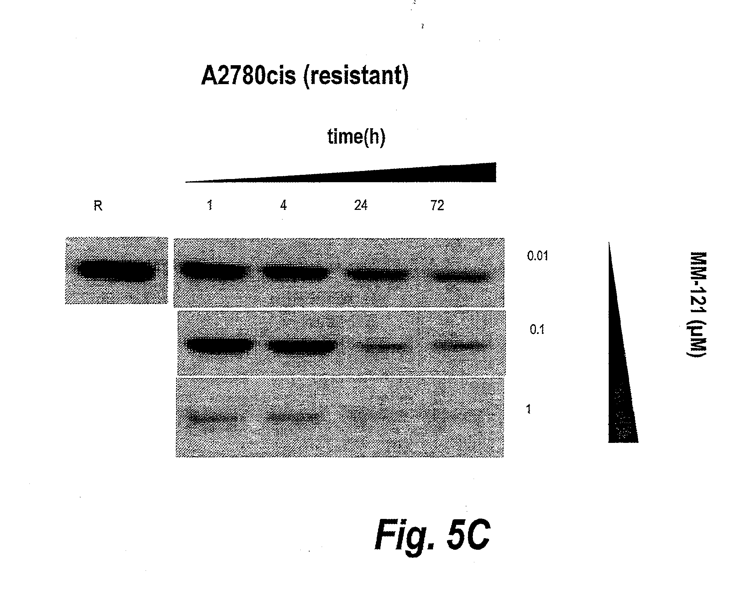

Figure 5 shows images of three western blots probed with anti-pAKT. Shown are pAKT levels in lysate from A2780 cells treated with cisplatin (Figure 5A), A2780cis cells treated with cisplatin (Figure 5B), and A2780cis cells treated with MM-121 (Figure 5C). S indicates untreated sensitive cell control and R indicates untreated resistant cell control. The X-axes indicate the time the lysates were harvested in hours post drug treatment and the Y-axes indicate drug concentration in µM.

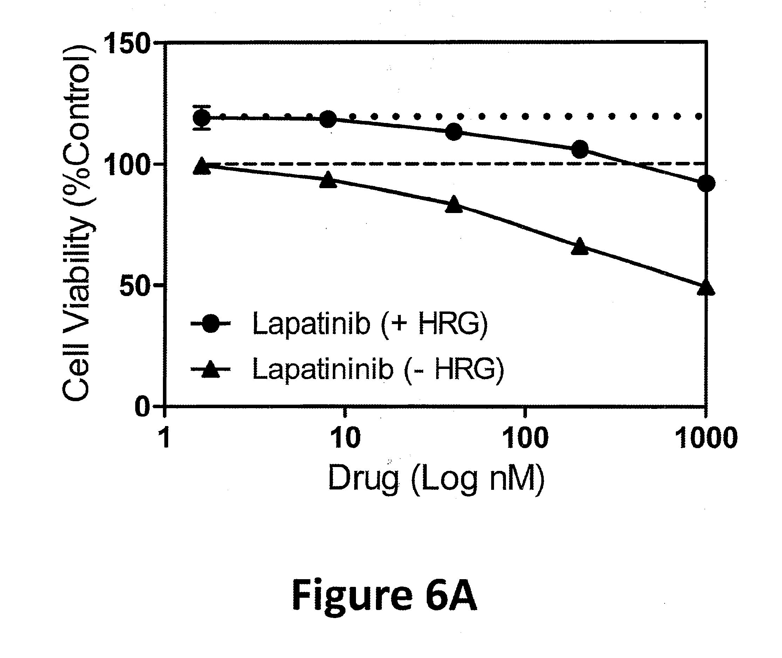

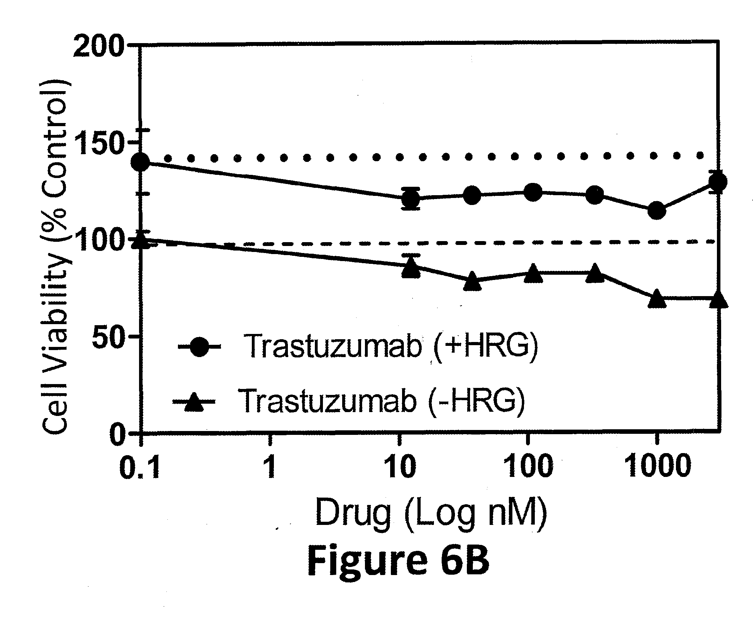

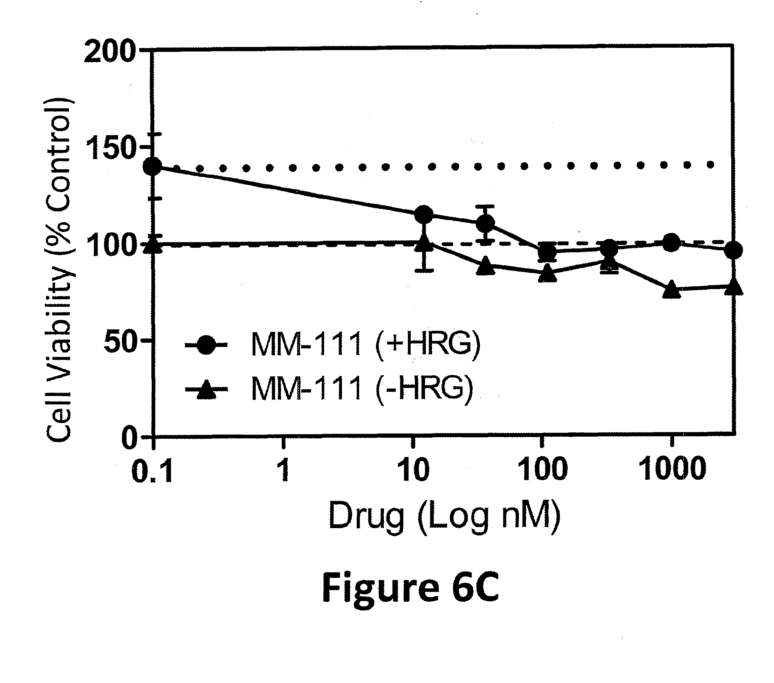

Figure 6 comprises a series of graphs showing cell viability (as % control) of BT474-M3 cells *in vitro* (Y-axis) after treatment with: Figure 6A, lapatinib, Figure 6B trastuzumab, or Figure 6C, MM-111. For each the X-axis indicates drug concentration in nM.

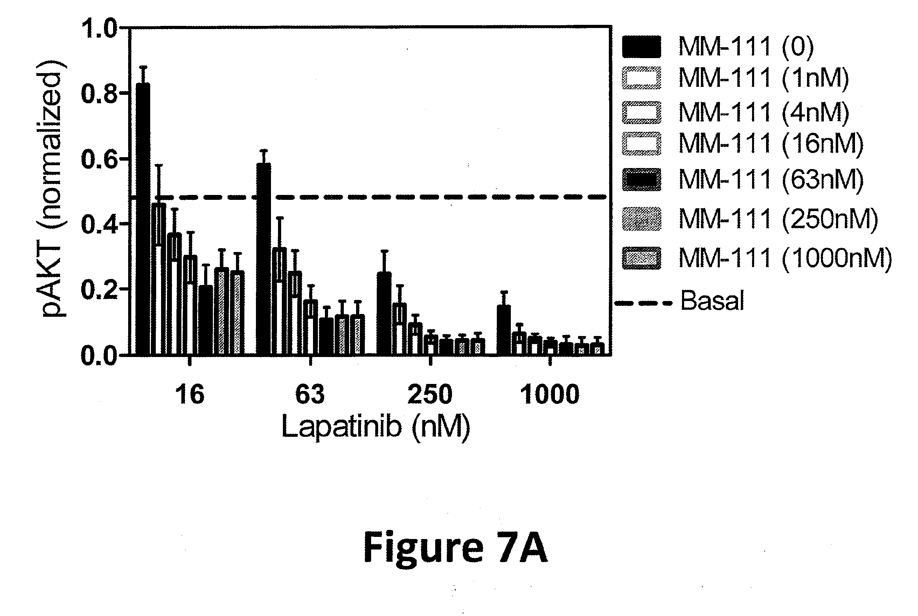

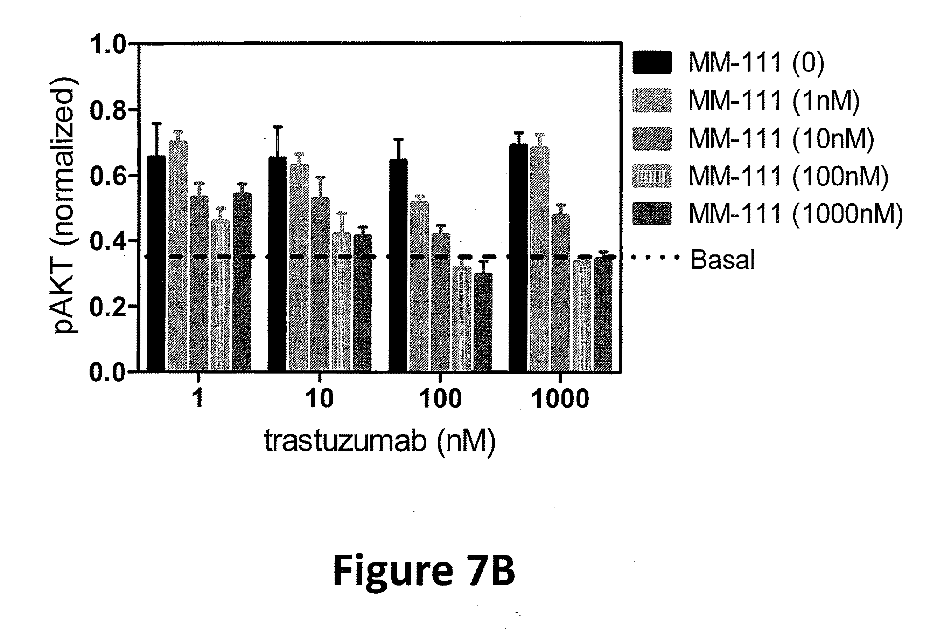

Figure 7 comprises two graphs showing inhibition of AKT activation in heregulin-stimulated BT474-M3 cells (Y-axes, normalized amounts of pAKT) after treatment with: Figure 7A lapatinib, Figure 7B, trastuzumab. For each the X-axis shows ErbB pathway inhibitor concentrations as indicated. For each indicated lapatinib concentration along the X-axis in Figure 7A the grouped bars, reading from left to right, correspond to MM-111 concentrations of 0nM, 1nM, 4nM, 16nM, 63nM, 250nM, and 1000nM. For indicated trastuzumab concentration along the X-axis in Figure 7B the grouped bars, reading from left to right, correspond to trastuzumab concentrations of 0nM, 1nM, 10nM, 100nM, and 1000nM. The lines marked "basal" indicate the pAKT level in cells that were not stimulated with heregulin and were not treated with MM-111, lapatinib, or trastuzumab.

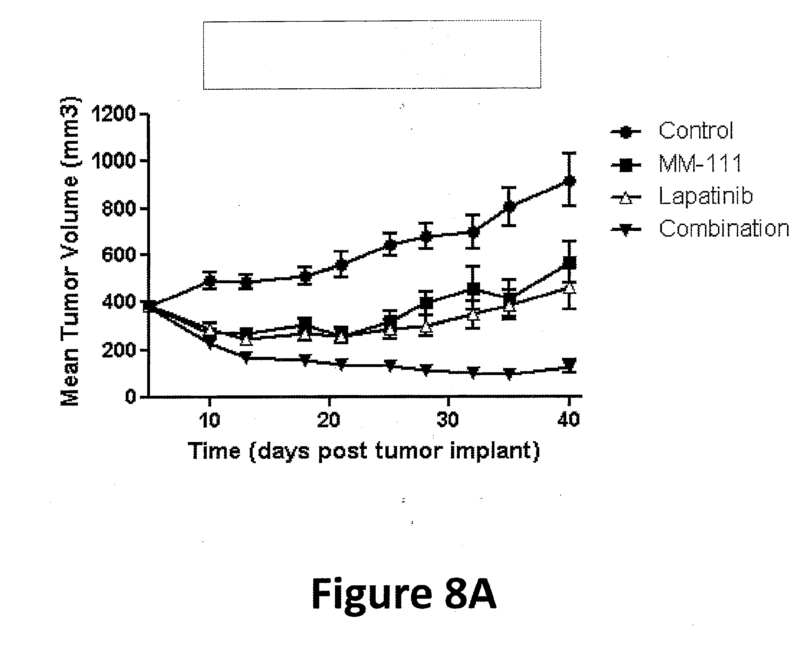

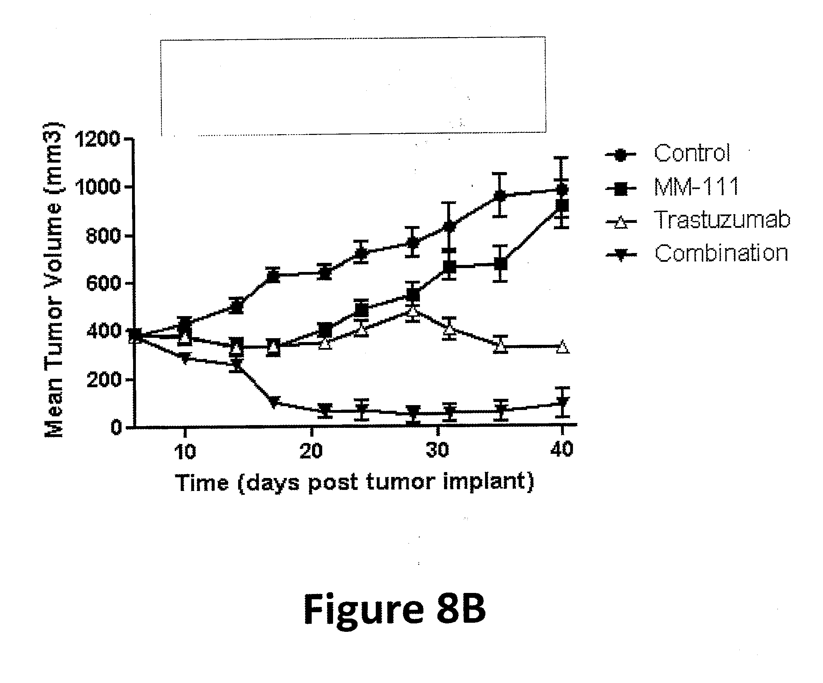

Figure 8 comprises two graphs showing reductions in tumor volumes in a BT474-M3 xenograft breast cancer xenograft model after treatment with: Figure 8A, MM-111, lapatinib, or a combination of MM-111 and lapatinib; Figure 8B MM-111, trastuzumab, or a combination of MM-111 and trastuzumab. The Y-axes represent mean tumor volume in mm³ and the x-axes represent time in days post tumor implant.

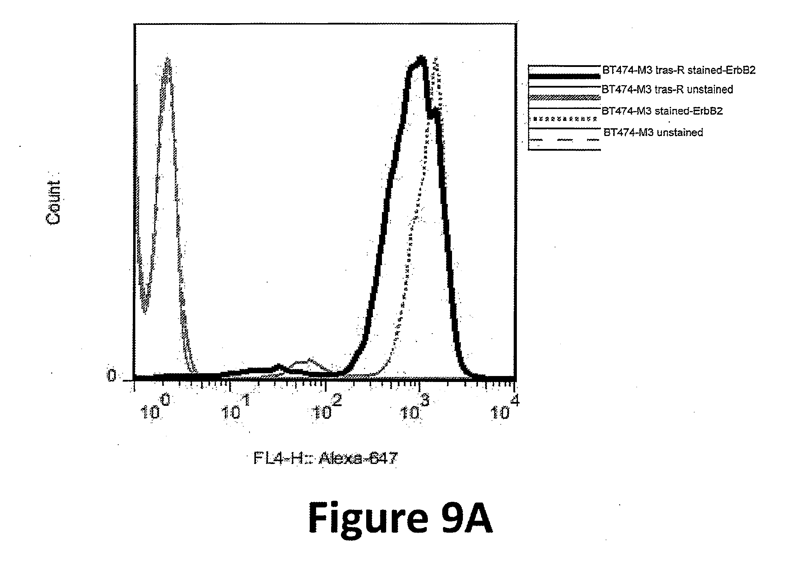

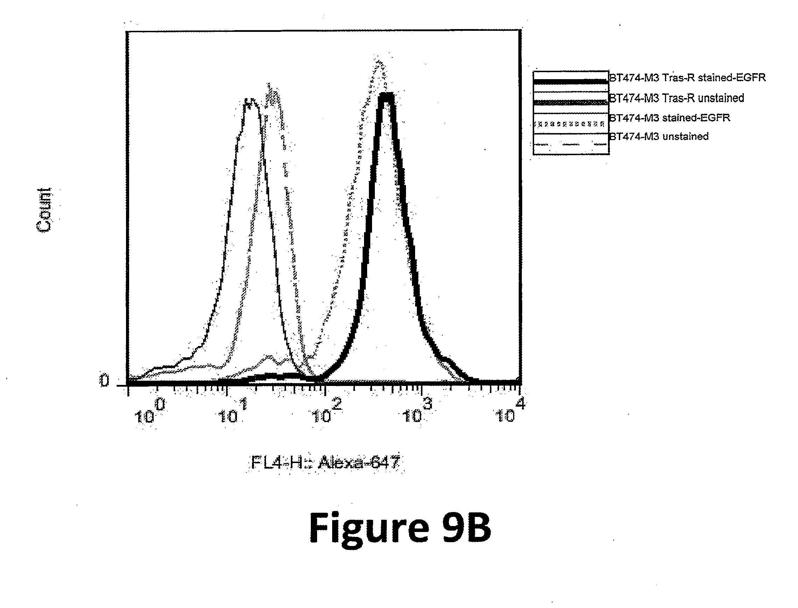

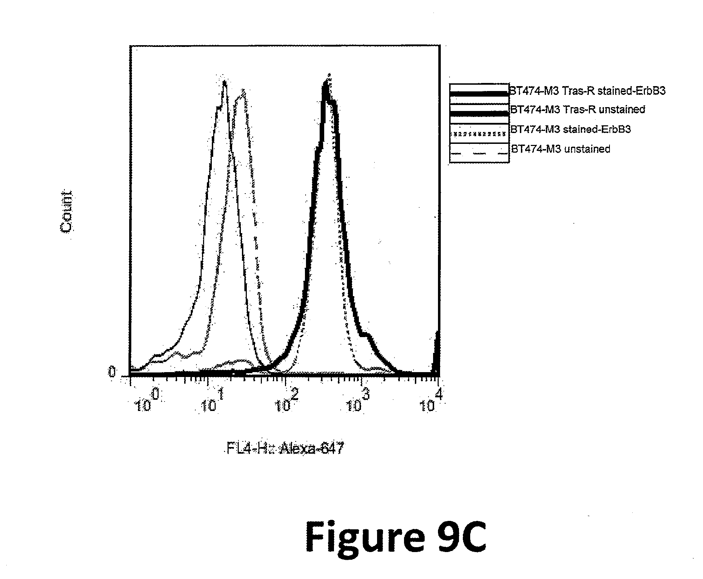

Figure 9 comprises three plots showing FACS analysis of ErbB receptors in trastuzumab-resistant BT474-M3 cells. For each, cell count (Y-axis) is plotted against fluorochrome emission intensity (X-axis) for: Figure 9A, ErbB2 receptor status - thick solid black line = trastuzumab-resistant cells stained for ErbB2, thinner solid gray line = trastuzumab-resistant cells, unstained, dotted line = non-resistant cells, stained for ErbB2, dashed line = non-resistant cells, unstained; Figure 9B, EGFR status - thick solid black line = trastuzumab-resistant cells stained for EGFR, thinner solid gray line = trastuzumab-resistant cells, unstained, dotted line = non-resistant cells, stained for EGFR, dashed line = non-resistant cells, unstained; Figure 9C, ErbB3 status ErbB3 receptor status, thick solid black line = trastuzumab-resistant cells stained for ErbB3, thinner solid gray line = trastuzumab-resistant cells, unstained, dotted line = non-resistant cells, stained for ErbB3, dashed line = non-resistant cells, unstained.

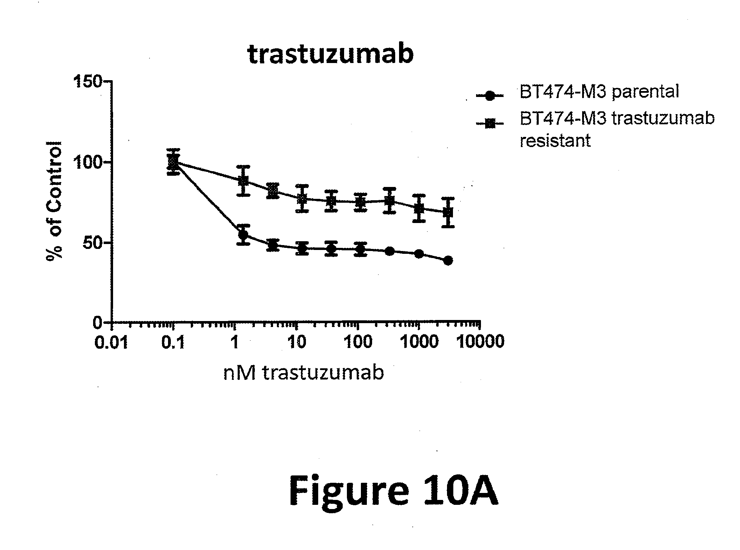

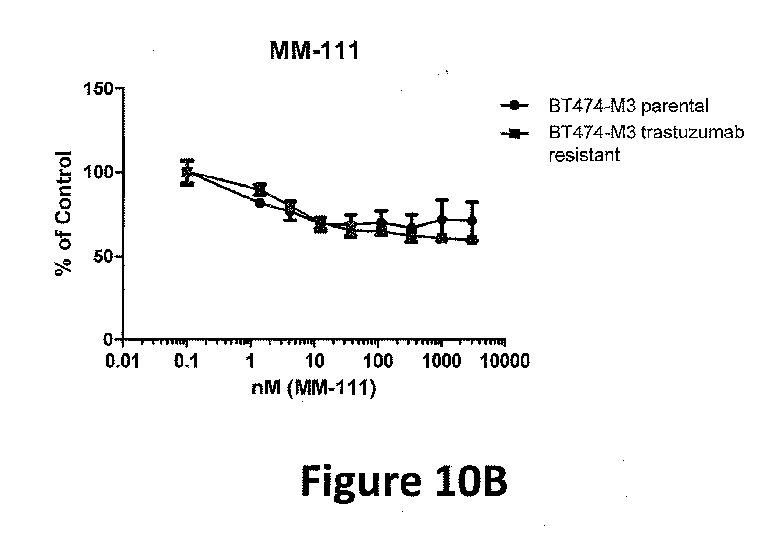

Figure 10 comprises two graphs comparing the ability of: Figure 10A, trastuzumab and Figure 10B, MM-111, to inhibit cell growth in trastuzumab-resistant BT474-M3 cells. For each, the growth of parental (non-resistant) and trastuzumab-resistant BT474-M3 cells is shown as % of control (no trastuzumab or MM-111 added) cells (Y-axis) after treatment with a dose range of trastuzumab (A) or MM-111 (B) (X-axes, in nM trastuzumab or MM-111).

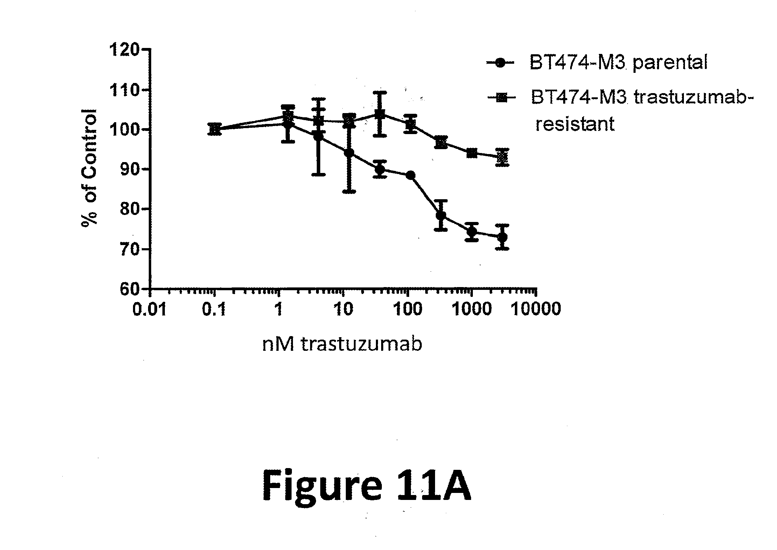

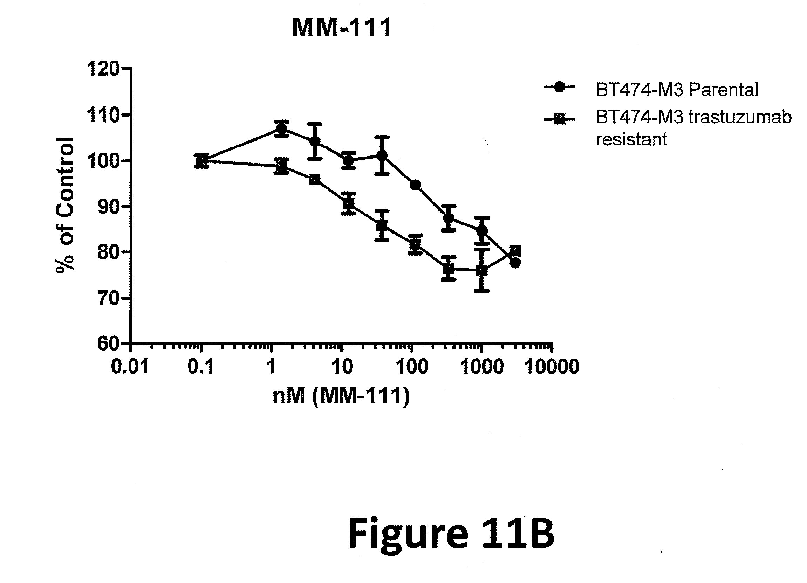

Figure 11 comprises two graphs comparing the ability of: Figure 11A, trastuzumab, and Figure 11B, MM-111, to inhibit cell growth in trastuzumab-resistant BT474-M3 cell spheroids. The growth of parental (non-resistant) and trastuzumab-resistant BT474-M3 cell spheroids is shown as % of control (no trastuzumab or MM-111 added) cells (Y-axes) after treatment with a dose range of drug (X-axes, in nM trastuzumab or MM-111).

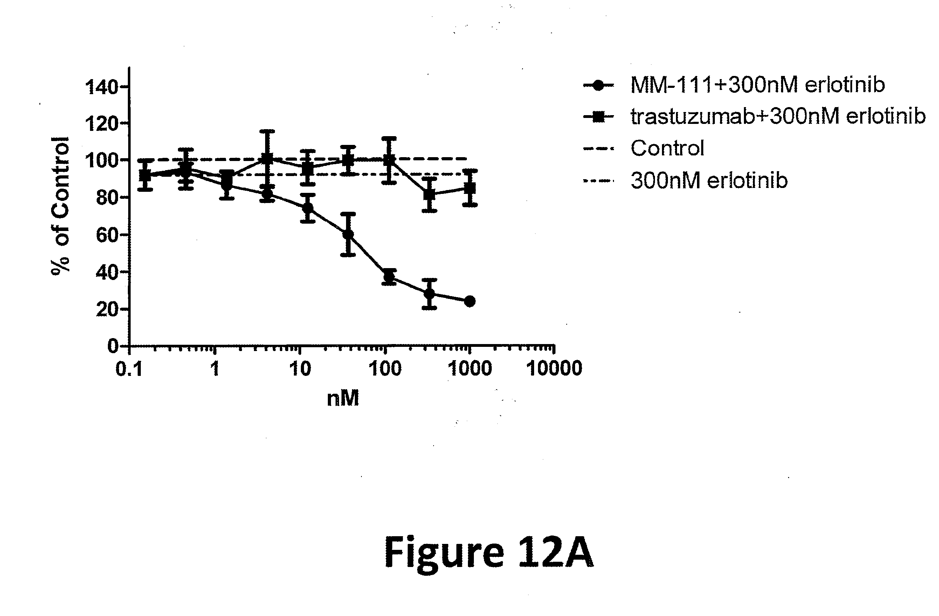

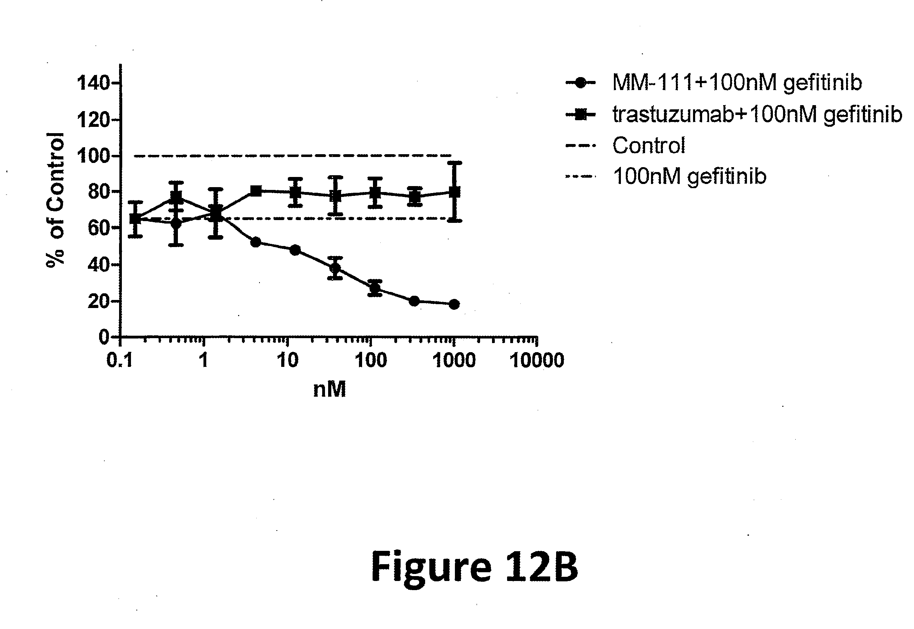

Figure 12 is two graphs comparing the effect of MM-111 or trastuzumab on cell growth of trastuzumab-resistant BT474-M3 cell spheroids when in combination with 300nM erlotinib (Figure 12A) or 100nM gefitinib (Figure 12B). The growth of the cell spheroids is shown as % of control (dashed line, no drug added). The growth of cell spheroids treated with erlotinib alone or gefitinib alone is shown by dot-dash lines. The x-axes are a log scale of each of MM-111 and /or trastuzumab concentration in nM and the y axis is spheroid size as % of control spheroid size.

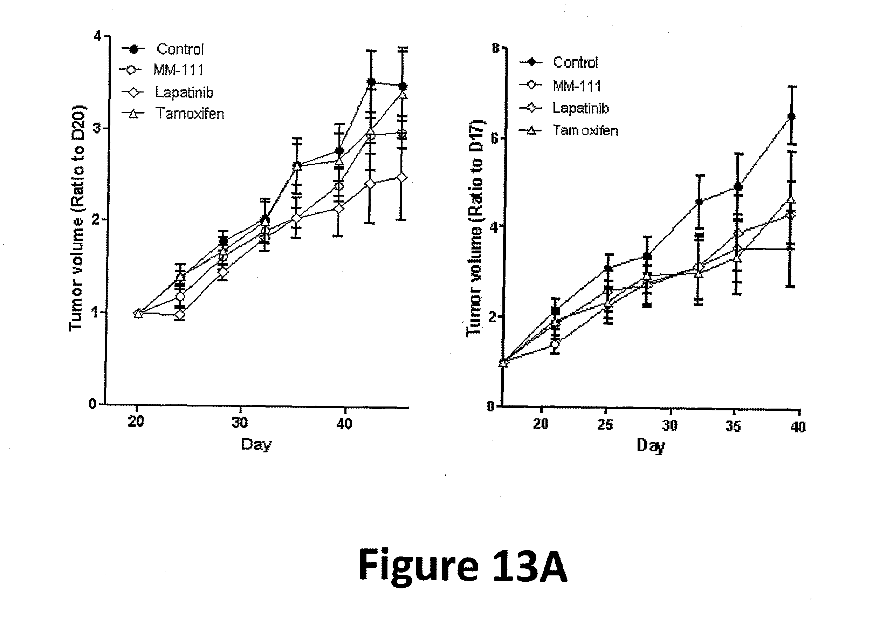

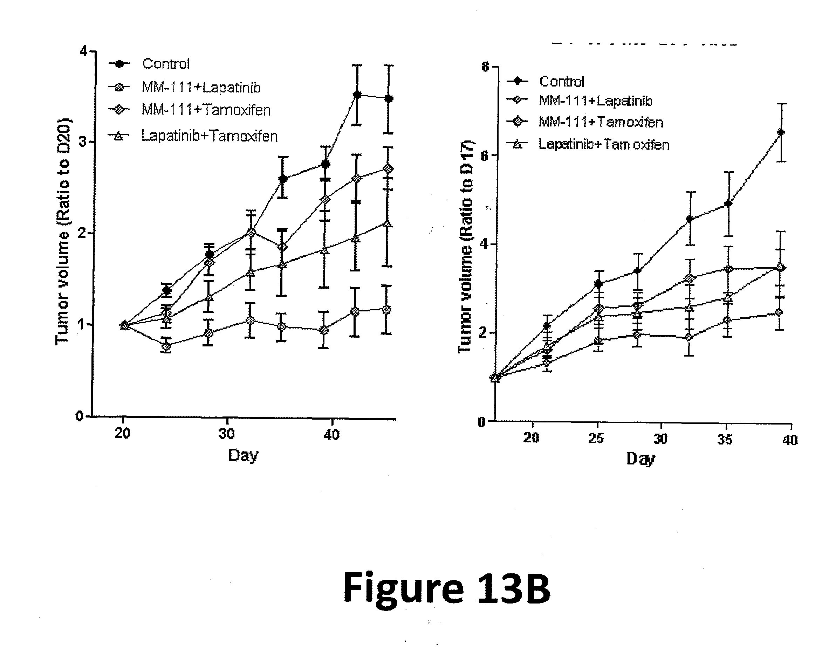

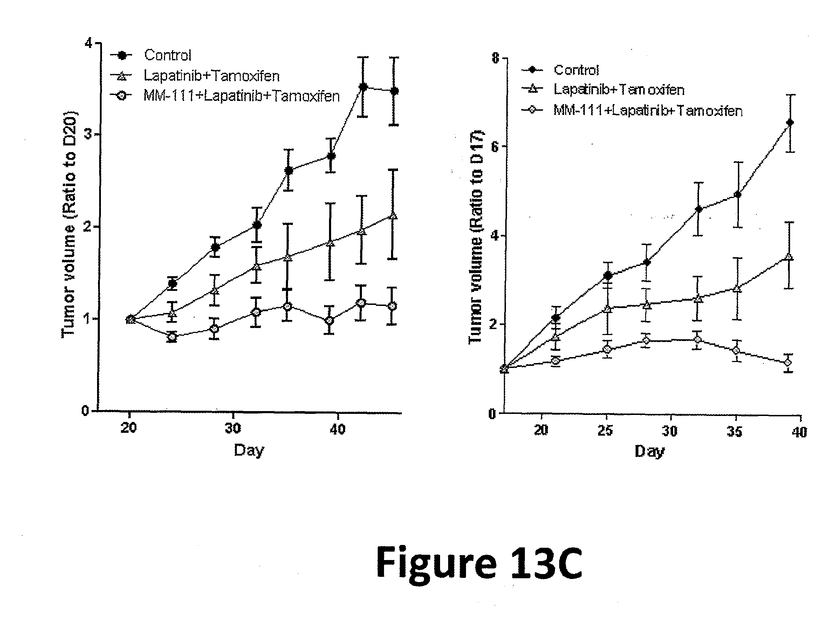

Figure 13 is a series of graphs showing the effect of MM-111, lapatinib, and tamoxifen on tumor growth inhibition in a BT474-M3 xenograft model. In Figures 13A, 13B, and 13C, the left panel shows tumor volume of xenografts of BT474-M3 cells that have been engineered to express green fluorescent protein and the right panel shows tumor volume in xenografts of the BT474-M3 cells that have been engineered to express GFP and heregulin 1. Figure 13A shows the tumor growth curves of BT474-M3-GFP and BT474-M3-GFP-HRG tumors wherein the mice were treated with MM-111 (48 mpk), lapatinib (150 mpk) and tamoxifen (5 mg). Figure 13B shows the tumor growth curves of BT474-M3-GFP and BT474-M3-GFP-HRG tumors wherein the mice were treated with MM-111 (48 mpk) + lapatinib (150 mpk), MM-111 + tamoxifen (5 mg), and lapatinib + tamoxifen combination therapies. Figure 13C shows the tumor growth curves of BT474-M3-GFP and BT474-M3-GFP-HRG tumors wherein the mice were treated with lapatinib + tamoxifen and MM-111 + lapatinib + tamoxifen combination therapies. Control mice received no treatment. The x-axes are time in days and the y-axes are tumor volume in relation to the tumor volume at the start of treatment on day 17 or day 20 after inoculation ("Ratio to D17" or "Ratio to D20").

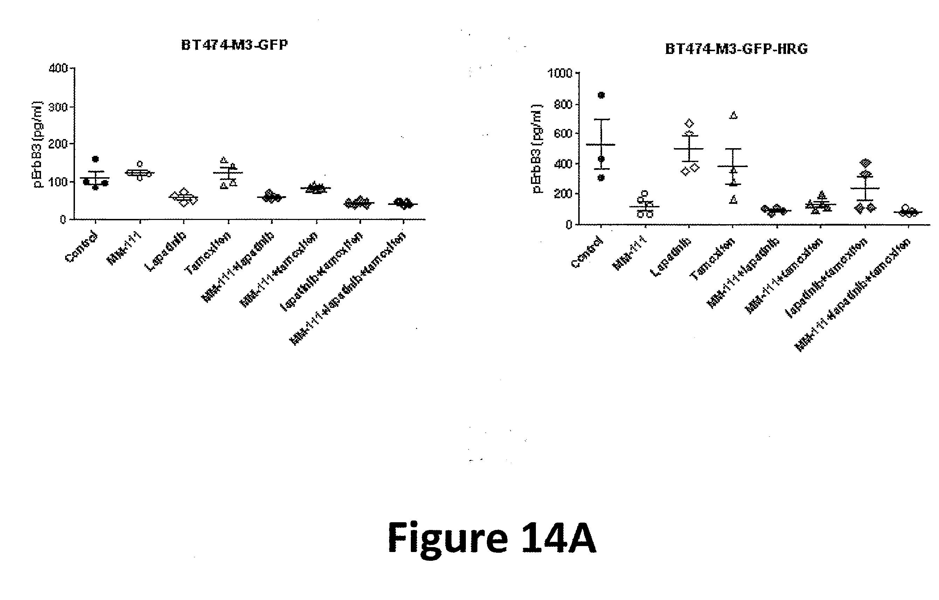

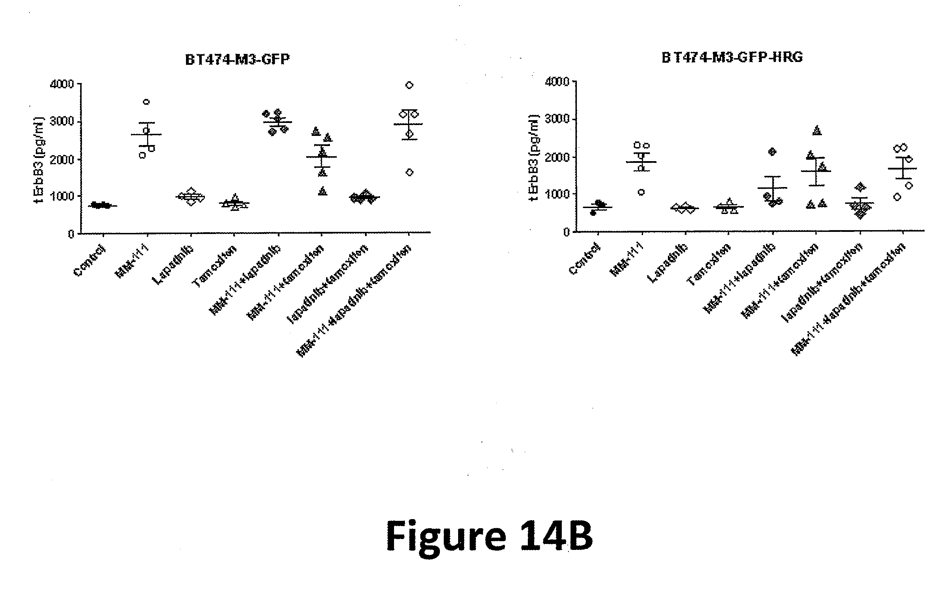

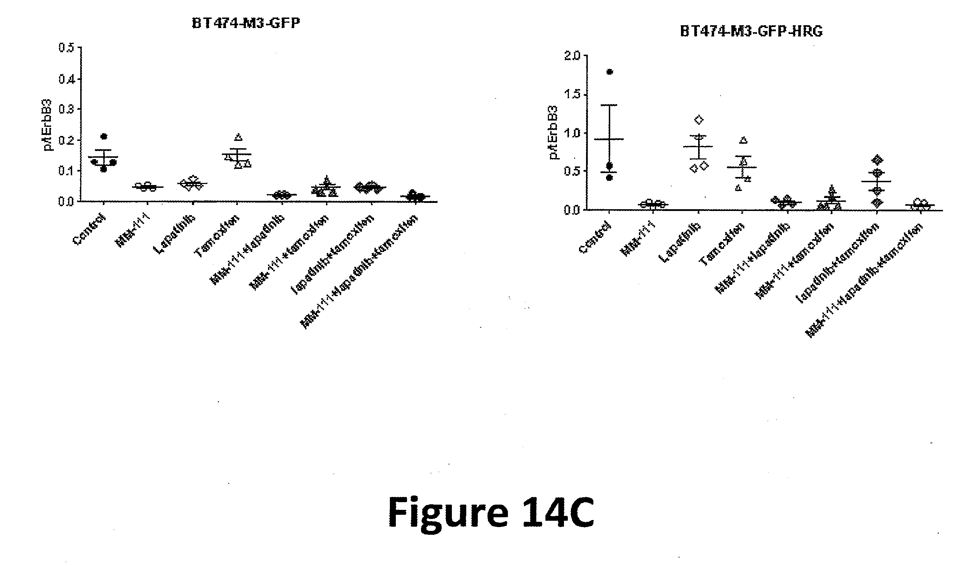

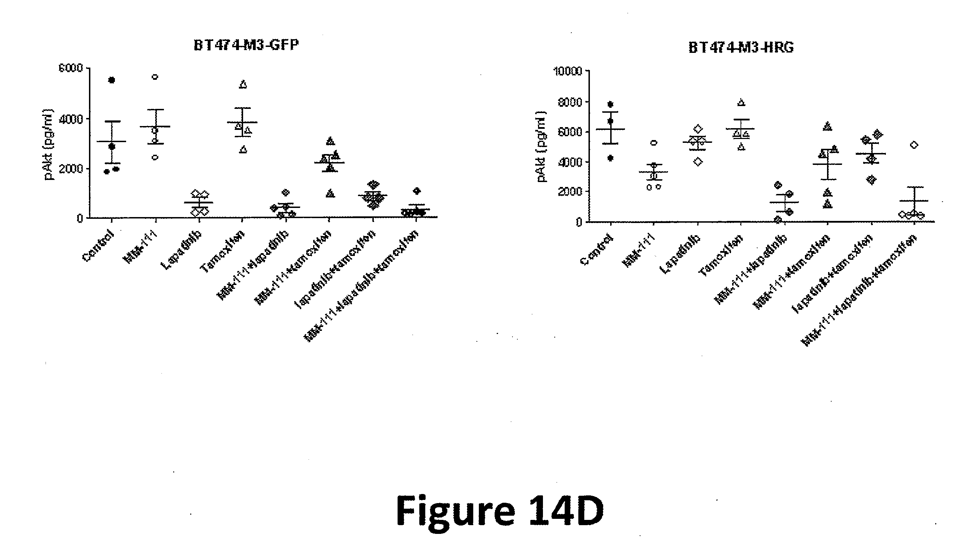

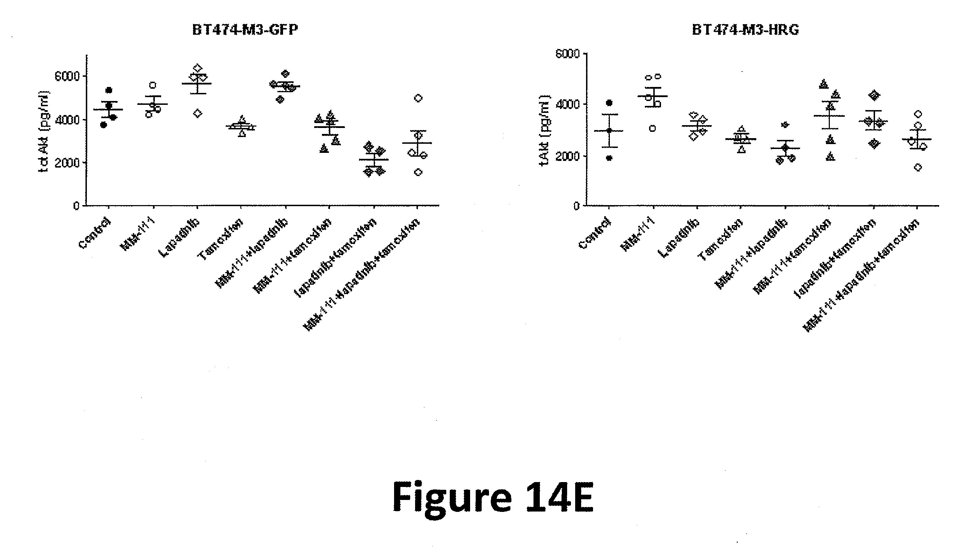

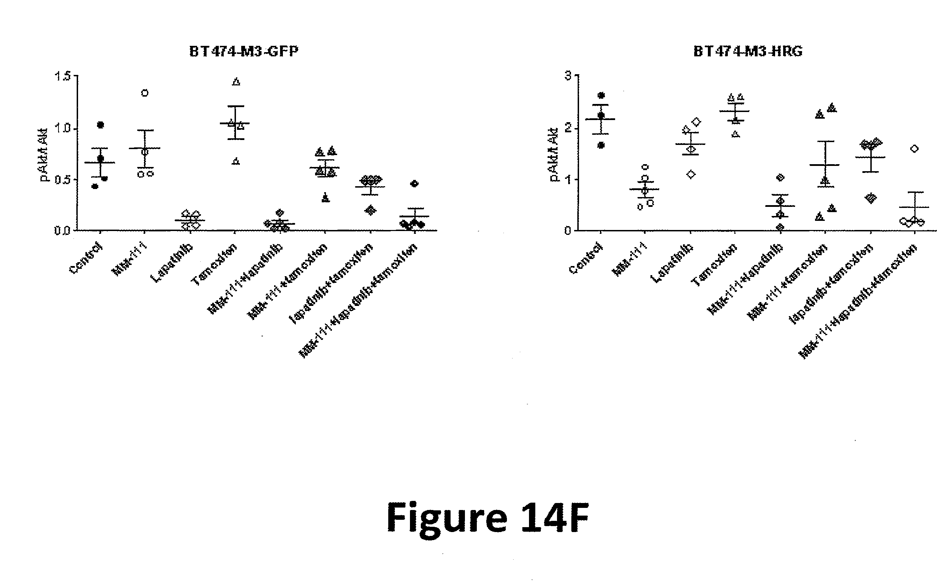

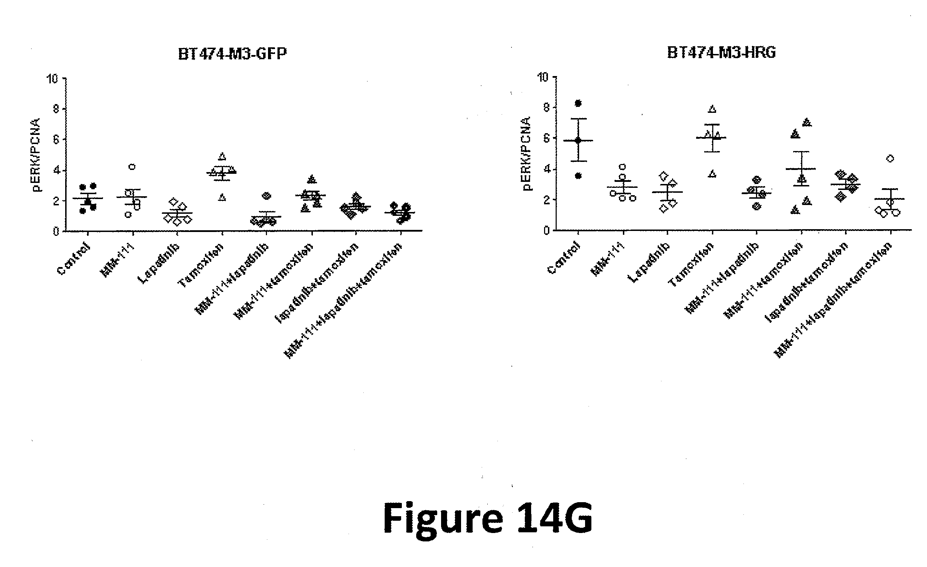

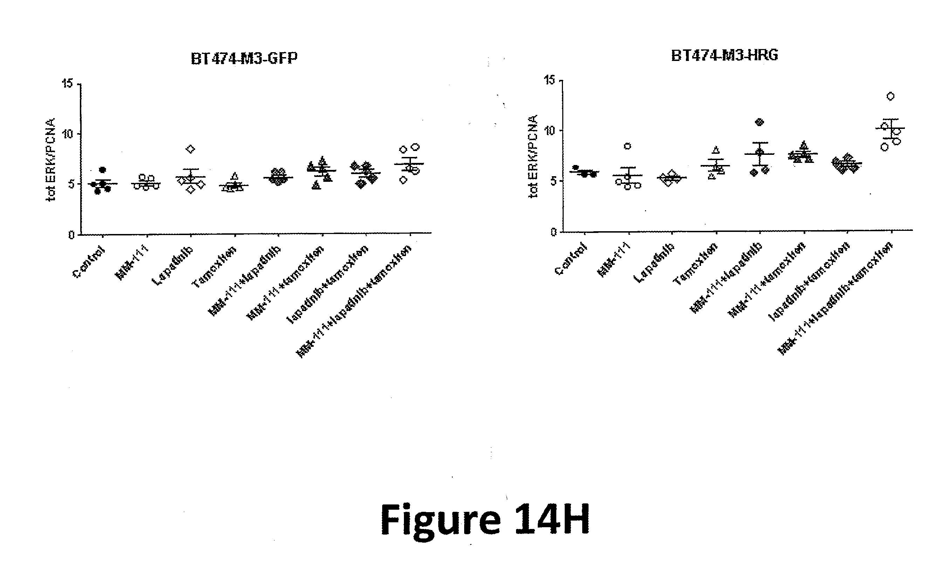

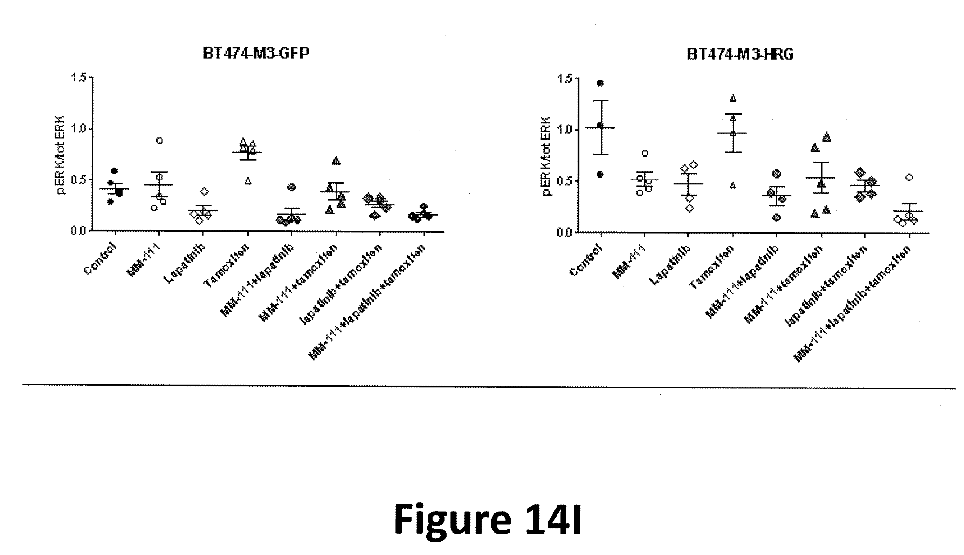

Figure 14 is a series of graphs showing the effect of MM-111, lapatinib and tamoxifen mono- and combination therapies on HRG-induced signaling in BT474-M3-GFP (left panels) and BT474-M3-GFP-HRG (right panels) tumors. Figure 14A shows pErbB3 levels in pg/ml; Figure 14B shows total ErbB3 levels (tErbB3) in pg/ml; Figure 14C shows the ratio of phospho-ErbB3 to total ErbB3; Figure 14D shows phospho-Akt (pAkt) levels in pg/ml; Figure 14E shows total Akt levels (tAkt) in pg/ml; Figure 14F shows the ratio of pAkt to tAkt; Figure 14G shows phospho-ERK1/2 levels (pERK) normalized to the levels of proliferating cell nuclear antigen (PCNA) ; and Figure 14H shows total ERK1/2 levels (totERK) normalized to the levels of PCNA; Figure 14I shows the ratio of pERK and totERK levels. The x-axes represent the therapy the tumor-bearing mice received. Control mice received no treatment.

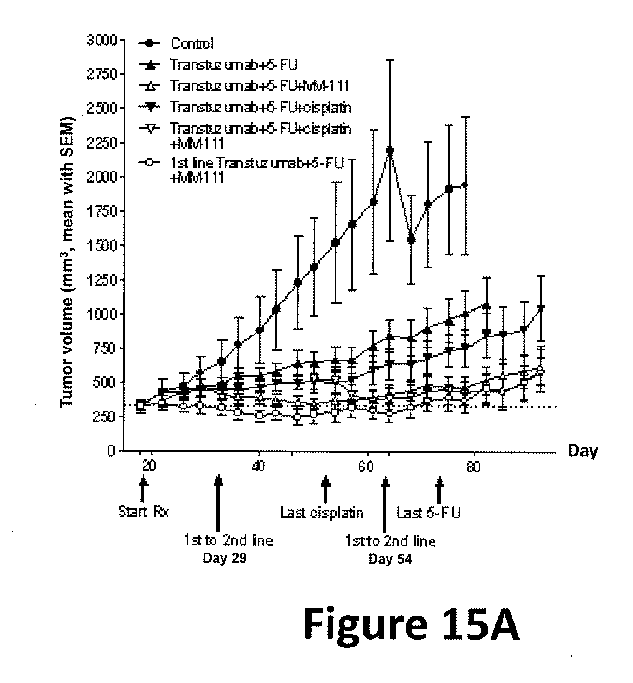

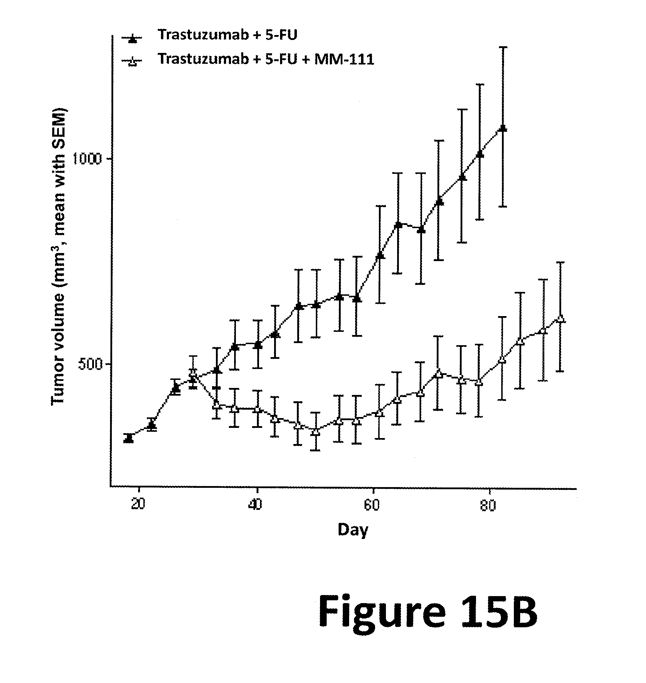

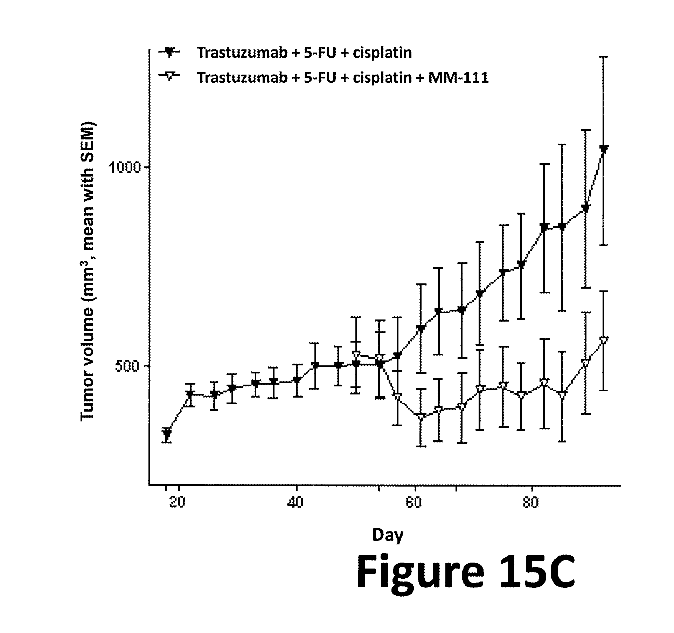

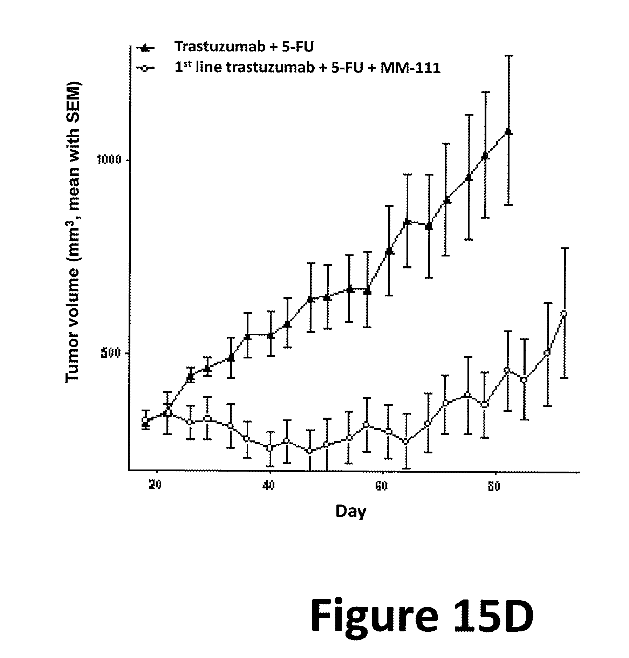

Figure 15 is a series of graphs showing tumor growth curves in an NCI-N87 xenograft model. Figure 15A shows tumor bearing mice either untreated (control) or treated with trastuzumab (3.5 mpk) + 5-fluorouracil (5-FU; 12 mpk 5 days/week), trastuzumab + 5-FU + cisplatin (5 mpk), 1^{st} line MM-111 (96 mpk) + trastuzumab + 5-FU, 2^{nd} line MM-111 + trastuzumab + 5-FU and 2^{nd} line MM-111 + trastuzumab + 5-FU + cisplatin. The switch to 1^{st} to 2^{nd} line treatments as well as discontinuation of the chemotherapeutics is indicated with arrows. Figure 15B shows the tumor growth curves of NCI-N87 -tumors treated with trastuzumab + 5-FU and 2^{nd} line MM-111 + trastuzumab + 5-FU. Figure 15C shows the tumor growth curves of NCI-N87 -tumors treated with trastuzumab + 5-FU + cisplatin and 2^{nd} line MM-111 + trastuzumab + 5-FU + cisplatin. The x-axes are time in days and the y-axes are tumor volume in mm³.

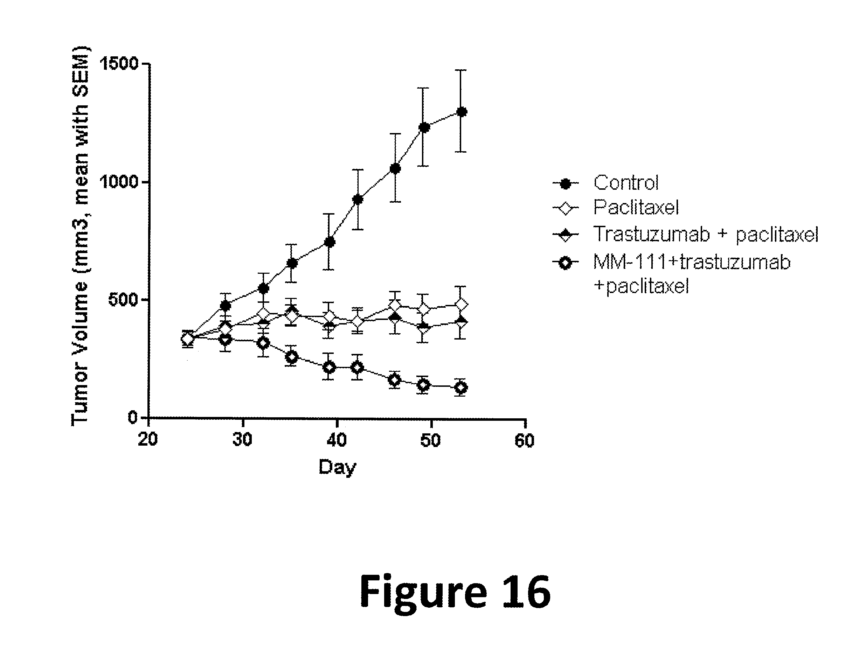

Figure 16 is a series of graphs showing the effect of MM-111, lapatinib and tamoxifen mono- and combination therapies on HRG-induced signaling in BT474-M3-GFP (left panels) and BT474-M3-GFP-HRG (right panels) tumors. Tumor-bearing mice were treated with MM-111, lapatinib and trastuzumab monotherapies (Figure 16A); MM-111 +lapatinib, MM-111 + trastuzumab, and lapatinib + trastuzumab combination therapies (Figure 16B); or lapatinib + trastuzumab and MM-111 + lapatinib + trastuzumab combination therapies (Figure 16C). The x-axes are time in days and the y-axes are tumor volume in relation to the tumor volume at the start of treatment on day 17 after inoculation ("Ratio").

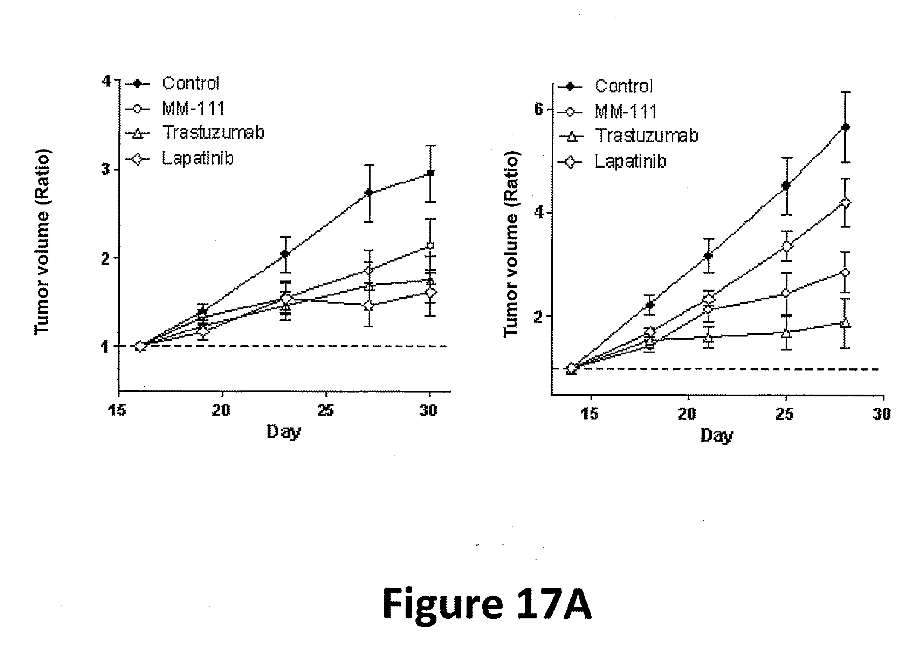

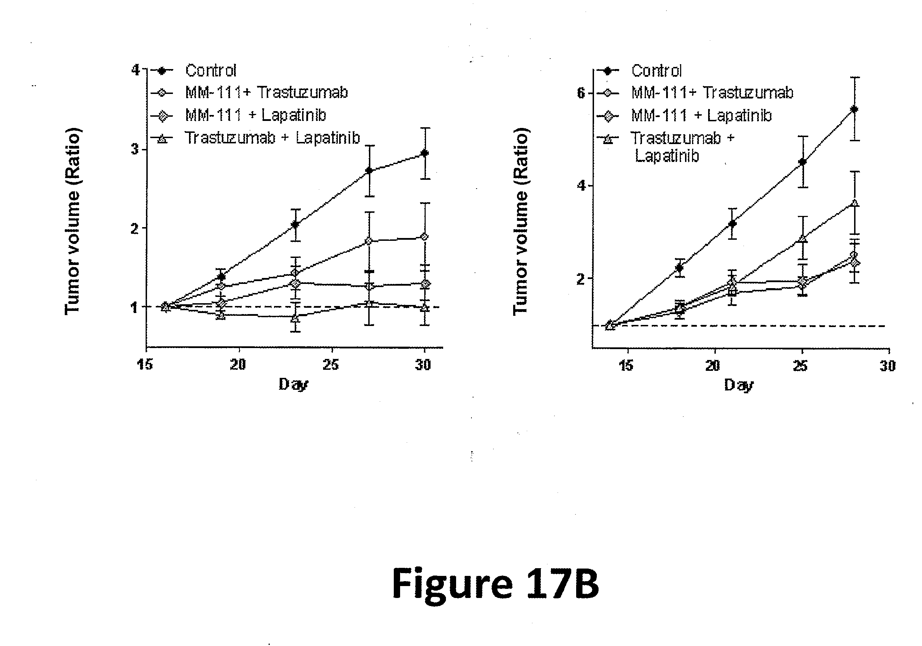

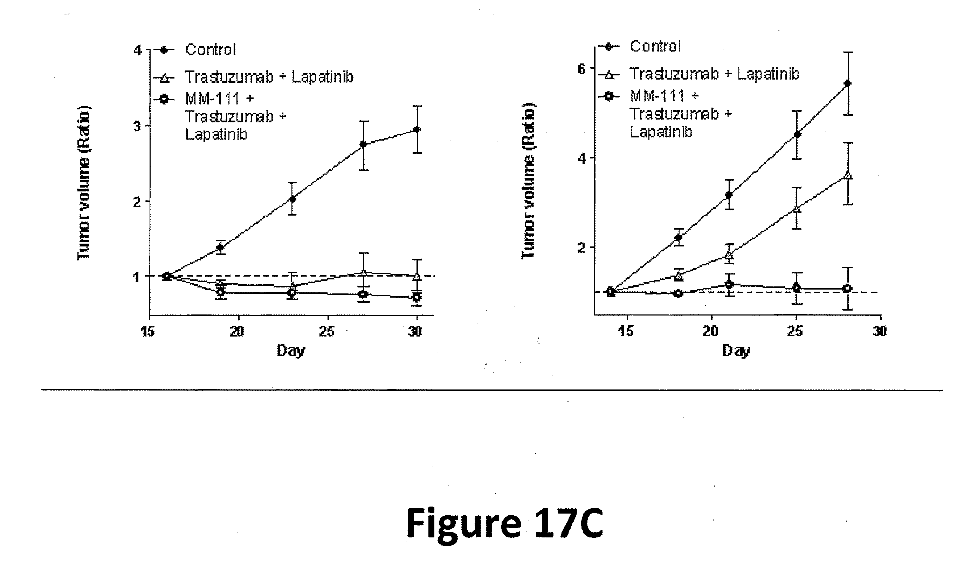

Figure 17 is a graph showing tumor growth curves in an NCI-N87 xenograft model. Tumor bearing mice were either untreated (control) or treated with paclitaxel (20 mpk), trastuzumab (3.5 mpk) + paclitaxel, or MM-111 (48 mpk) + trastuzumab + paclitaxel. The x-axis is time in days and the y-axis is tumor volume in mm³.

### Detailed Description

Methods for overcoming resistance to ErbB pathway inhibitors are provided, as well as pharmaceutical compositions that can be used in such methods. As described further in the Examples, it has now been demonstrated that an ErbB3 inhibitor, e.g., an anti-ErbB3 antibody or a bispecific anti-ErbB3, anti-ErbB2 antibody, is able to overcome ligand-induced (e.g., heregulin-induced) resistance to an ErbB pathway-targeted therapy (e.g., an EGFR-targeted therapy) both in vitro and in vivo. Accordingly, provided herein are methods for overcoming resistance to ErbB pathway inhibitors by combining the use of an ErbB pathway inhibitor with an ErbB3 inhibitor.

Although not intended to be limited by mechanism or bound by any theory of operation, the ability of the ErbB3 inhibitors described herein to overcome resistance to ErbB pathway inhibitors is thought to be due, at least in part, to the ability of the ErbB3 inhibitor to block ligand-dependent reactivation of ErbB3 signaling that leads to reactivation of PI3K/AKT signaling.

So that this disclosure may be more readily understood, certain terms are first defined.

As used herein, the term "inhibitor" indicates therapeutic agents that inhibit, downmodulate, suppress or downregulate activity of a receptor or other signal transduction protein, including signaling mediated thereby. The term encompasses small molecule inhibitors (e.g., small molecule tyrosine kinase inhibitors) and antibodies, interfering RNA (shRNA, siRNA), soluble receptors and the like.

An "ErbB pathway inhibitor" is an inhibitor that acts on one or more proteins of one or more ErbB signaling pathways, such as the EGFR (ErbB1/HER1) signaling pathway or the HER2 (ErbB2, neu) signaling pathway.

An "EGFR inhibitor" acts on EGFR.

A "HER2 inhibitor" acts on HER2 (ErbB2).

An "ErbB3 inhibitor" acts on ErbB3.

An "antibody" is a whole antibody or any antigen binding fragment (i.e., "antigen-binding portion") or single chain thereof. The term "antibody" encompasses: (i) monoclonal antibodies; (ii) recombinant antibodies (*i.e*., antibodies that are prepared, expressed, created or isolated by recombinant means); (iii) chimeric antibodies (*i.e.,* antibodies in which the variable domain(s) are from one species and the constant domain(s) are from another); (iv) humanized antibodies (i.e., antibodies in which only the CDRs are from a donor species and the rest of the antibody structure is human, although additional FR substitutions, either donor substitutions or non-donor/non-acceptor substitutions, may be incorporated); (v) fully human antibodies (*i.e*., antibodies in which the variable region CDRs and FRs are derived from human germline immunoglobulin sequences); and (vi) bispecific and multispecific antibodies (i.e., antibodies having two or more binding sites with different antigen-binding specificities). The term "antigen-binding portion" of an antibody (or simply "antibody portion"), as used herein, refers to one or more fragments of an antibody that retain the ability to specifically bind to an antigen (*e.g.,* ErbB3). Examples of binding fragments encompassed within the term "antigen-binding portion" of an antibody include (i) a Fab fragment, a monovalent fragment consisting of the V_{L}, V_{H}, CL and CH1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) an Fv fragment consisting of the V_{L} and V_{H} domains of a single arm of an antibody, (v) a dAb including V_{H} and V_{L} domains; (vi) a dAb fragment, which consists of a V_{H} domain; (vii) a dAb which consists of a V_{H} or a V_{L} domain; and (viii) a combination of two or more isolated CDRs which are joined, e.g., by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form a monovalent molecule (known as a single chain Fv (scFv). Such single chain antibodies are also intended to be encompassed within the term "antigen-binding portion" of an antibody. These antibody fragments may be obtained using conventional techniques known to those with skill in the art, and the fragments may be screened for utility in the same manner as are intact antibodies. Antigen-binding portions can be produced by recombinant DNA techniques, or by enzymatic or chemical cleavage of intact immunoglobulins.

The term "resistance to an ErbB pathway inhibitor" (such as in "exhibiting resistance to an ErbB pathway inhibitor") refers to the property of a cell (e.g., a cancer cell) in which the cell displays reduced, diminished or a lack of responsiveness to an ErbB pathway inhibitor (e.g., as measured by the ability of the inhibitor to inhibit cell growth or proliferation), as compared to the same cell at an earlier time point or as compared to other cells of the same type that respond to the ErbB pathway inhibitor. The cell can be within tissue, e.g., tumor tissue. Furthermore, the tissue, e.g., tumor tissue, can be in a subject, in which case the subject is referred to as exhibiting resistance to the ErbB pathway inhibitor.

As used herein, the term "acquired resistance" refers to resistance to an ErbB pathway inhibitor that develops in a cell over time, typically during the course of treatment with an ErbB pathway inhibitor, such that the responsiveness of the cell to the ErbB pathway inhibitor diminishes over time as compared to the responsiveness of such a cell to the inhibitor at the start of treatment. This "acquired resistance", which develops over time in previously responsive cells, is in contrast to cells that are "predisposed to resistance" to an ErbB pathway inhibitor, which refers to an inherent lack of, or significantly reduced, responsiveness of the cells to treatment with an ErbB pathway inhibitor as compared to cells that exhibit responsiveness to (i.e., cells whose growth and/or proliferation is significantly inhibited by) the ErbB pathway inhibitor.

As used herein, the terms "overcoming resistance" and "overcome resistance" to an inhibitor, e.g., an ErbB pathway inhibitor, refers to the phenomenon in which the level or amount or degree of resistance to an inhibitor in a previously-resistant cell is diminished, reduced or reversed such that the cell exhibits a measurable degree of responsiveness (or increased responsiveness) to the same inhibitor, or another inhibitor that inhibits the same signaling pathway, as compared to the cell in its resistant state. For example, "overcoming resistance to an EGFR pathway inhibitor" in a cell, wherein the inhibitor for which resistance has been demonstrated is, for example, gefitinib, results in a cell that exhibits a measurable degree of responsiveness to gefitinib or to another EGFR pathway inhibitor (such as the anti-EGFR antibody cetuximab) as compared to the cell in its resistant state.

As used herein, the term "subject" includes any human or nonhuman mammal having a disease or disorder for which resistance to an ErbB pathway inhibitor can be addressed using a method provided herein, such as a subject or patient with a tumor exhibiting such resistance

### I. ErbB3 Inhibitors

As described in further detail herein, the methods and compositions provided herein involve the use of one or more ErbB3 inhibitors.

MM-121 is a fully human anti-ErbB3 antibody currently undergoing Phase II clinical trials. MM-121 (also referred to as "Ab #6") and related human anti-ErbB3 antibodies are described in detail in U.S. Patent No. 7,846,440, U.S. Patent Publication Nos. 20090291085, 20100056761, and 20100266584, and PCT Publication No. WO 2008/100624. Other anti-ErbB3 antibodies that may be used in a disclosed combination include any of the other anti-ErbB3 antibodies described in U.S. Patent No. 7,846,440, such as Ab #3, Ab #14, Ab #17 or Ab #19 or an antibody that competes with Ab #3, Ab #14, Ab #17 or Ab #19 for binding to ErbB3. Additional examples of anti-ErbB3 antibodies that may be administered in accordance with the methods disclosed herein include antibodies disclosed in U.S. Patents and Patent Publications Nos. 7,285,649, 20100310557, and 20100255010, as well as antibodies 1B4C3 (cat # sc-23865, Santa Cruz Biotechnology) and 2D1D12 (U3 Pharma AG), both of which are described in, e.g., U.S. Patent Publication No. 20040197332 and are produced by hybridoma cell lines DSM ACC 2527 or DSM ACC 2517 (deposited at DSMZ) anti-ErbB3 antibodies disclosed in U.S. Patent No. 7,705,130 including but not limited to the anti-ErbB3 antibody referred to as AMG888 (U3-1287 -- U3 Pharma AG and Amgen), described in, e.g., U.S. Patent No. 7,705,130; the anti-ErbB3 antibody referred to as AV-203 (Aveo Pharmaceuticals) which is described in U.S. Patent Publication No. 20110256154, the monoclonal antibodies (including humanized versions thereof), such as 8B8 (ATCC® HB-12070™), described in U.S. Patent No. 5,968,511, and the anti-ErbB3 antibodies mAb 205.10 (e.g., mAb 205.10.1, mAb 205.10.2, or mAb 205.10.3) (Roche-Glycart), described in, e.g., U.S. Patent Publication No. 20110171222. Other such examples include anti-ErbB3 antibodies that are multi-specific antibodies and comprise at least one anti-ErbB3 antibody (e.g., one of the aforementioned anti-ErbB3 antibodies) linked to at least a second therapeutic antibody or to an additional therapeutic agent. Yet other suitable anti-ErbB3 antibodies comprise either: 1) variable heavy (VH) and/or variable light (VL) regions encoded by the nucleic acid sequences set forth in SEQ ID NOs:45 and 46, respectively, or 2) VH and/or VL regions comprising the amino acid sequences set forth in SEQ ID NOs: 1 and 2, respectively, or 3) CDRH1, CDRH2, and CDRH3 sequences comprising the amino acid sequences set forth in SEQ ID NO:3(CDRH1) SEQ ID NO:4(CDRH2) and SEQ ID NO: 5(CDRH3), and/or CDRL1, CDRL2, and CDRL3 sequences comprising the amino acid sequences set forth in SEQ ID NO: 6 (CDRL1) SEQ ID NO: 7 (CDRL2) and SEQ ID NO: 8 (CDRL3) as well as an antibody that binds to human ErbB3 and has at least 90% variable region sequence identity with the above-mentioned antibodies 1), 2), or 3). In another embodiment, the antibody competes for binding with and/or binds to the same epitope on human ErbB3 as any one of the above-mentioned antibodies. When the antibody is MM-121, the epitope typically comprises residues 92-104 of human ErbB3 (SEQ ID NO: 41). In other embodiments, the antibody is a fully human monoclonal antibody that binds to ErbB3 and, in living cells and either a) inhibits ErbB2/ErbB3 complex formation or b) prevents intracellular phosphorylation of ErbB3induced by any of the forms of each of the following: heregulin, EGF, TGFα, betacellulin, heparin-binding epidermal growth factor, biregulin, epigen, epiregulin, and amphiregulin, or does both a) and b).

Anti-ErbB3 antibodies described above, can be generated, e.g., in prokaryotic or eukaryotic cells, using methods well known in the art, e.g., in a cell line capable of glycosylating proteins, such as CHO cells.

MM-111 (also referred to as B2B3-1), is described in co-pending U.S. Patent Application Serial No. 12/757,801, and PCT Publication No. WO2009/126920. Also disclosed therein are other bispecific anti-ErbB2/antiErbB3 antibodies that are scFv HSA conjugates and that are suitable for use in the methods and compositions provided herein, including A5-HSA-ML3.9, A5-HSA-B1D2, B12-HSA-B1D2, A5-HSA-F5B6H2, H3-HSA-F5B6H2, and F4-HSA-F5B6H2. Other bispecific anti-ErbB2/antiErbB3 antibodies that are suitable for use in the methods and compositions provided herein include ALM and H3 x B1D2, each comprising an anti-ErbB3 antibody linked to an anti-ErbB2 antibody, which are described further in U.S. Patent Nos. 7,332,585 and 7,332,580, as well as in PCT Application Serial No. PCT/US2007/024287.

In yet another embodiment, the bispecific anti-ErbB3, anti-ErbB2 antibody can comprise a mixture, or cocktail, of two or more bispecific anti-ErbB3, anti-ErbB2 antibodies, each of which binds to a different epitope on ErbB3. In one embodiment, the mixture, or cocktail, comprises three bispecific anti-ErbB3, anti-ErbB2 antibodies, each of which binds to a different epitope on ErbB3.

In another embodiment, the ErbB3 inhibitor comprises a nucleic acid molecule, such as an RNA molecule, that inhibits the expression or activity of ErbB3. RNA antagonists of ErbB3 have been described (see e.g., U.S. Patent Publication No. 20080318894). Moreover, interfering RNAs specific for ErbB3, such as shRNAs or siRNAs that specifically inhibit the expression and/or activity of ErbB3, have been described (see e.g., Sergina, N.V. et al. (2007) Nature 445:437-441; Liu, B. et al. (2007) Int. J. Cancer 120:1874-1882; Frolov, A. et al. (2007) Cancer Biol. Ther. 6:548-554; Sithanandam, G. and Anderson, L.M. (2008) Cancer Gene Ther. 15:413-418; Lee-Hoeflich, S.J. et al. (2008) Cancer Res. 68:5878-5887).

In yet another embodiment, the ErbB3 inhibitor comprises a soluble form of the ErbB3 receptor that inhibits signaling through the ErbB3 pathway. Such soluble ErbB3 molecules have been described in the art (see e.g., U.S. Patent No. 7,390,632, U.S. Patent Publication No. 20080274504 and U.S. Patent Publication No. 20080261270, each by Maihle et al., and U.S. Patent Publication No. 20080057064 by Zhou).

In another embodiment, the ErbB3 inhibitor comprises a nucleic acid molecule, such as an RNA molecule, that inhibits the expression or activity of ErbB3. RNA antagonists of ErbB3 have been described (see *e.g.,* U.S. Patent Publication No. 20080318894). Moreover, interfering RNAs specific for ErbB3, such as shRNAs or siRNAs that specifically inhibit the expression and/or activity of ErbB3, have been described (see *e.g*., Sergina, N.V. et al. (2007) Nature 445:437-441; Liu, B. et al. (2007) Int. J. Cancer 120:1874-1882; Frolov, A. et al. (2007) Cancer Biol. Ther. 6:548-554; Sithanandam, G. and Anderson, L.M. (2008) Cancer Gene Ther. 15:413-418; Lee-Hoeflich, S.J. et al. (2008) Cancer Res. 68:5878-5887).

In yet another embodiment, the ErbB3 inhibitor comprises a soluble form of the ErbB3 receptor that inhibits signaling through the ErbB3 pathway. Such soluble ErbB3 molecules have been described in the art (see e.g., U.S. Patent No. 7,390,632, U.S. Patent Publication No. 20080274504 and U.S. Patent Publication No. 20080261270, each by Maihle *et al.,* and U.S. Patent Publication No. 20080057064 by Zhou).

### II. ErbB Pathway Inhibitors

As described in further detail herein, the methods and compositions provided herein involve the use of one or more ErbB pathway inhibitors.

In one embodiment, the ErbB pathway inhibitor is an EGFR inhibitor (i.e., an inhibitor that inhibits EGFR and thereby inhibits EGFR pathway signaling).

In one embodiment, the EGFR inhibitor comprises an anti-EGFR antibody. One A useful anti-EGFR antibody is cetuximab (Erbitux®, ImClone). Other examples of anti-EGFR antibodies include MM-151 (further described in Bukhalid et al., co-pending commonly assigned U.S. Patent Application Serial No. 61/504,633, filed on July 5, 2011), Sym004 (Symphogen, Pederson et al., Cancer Research January 15, 2010 70; 588, also see U.S. Patent Serial No. 7,887,805), matuzumab (EMD72000), panitumumab (Vectibix®; Amgen); nimotuzumab (TheraCIM) and mAb 806 (Mishima, K. et al. (2001) Cancer Res. 61:5349-5354).

In another embodiment, the EGFR inhibitor comprises a small molecule inhibitor of the EGFR signaling pathway, such as a tyrosine kinase inhibitor (TKI) that inhibits the EGFR signaling pathway. An example of a small molecule inhibitor of the EGFR signaling pathway is gefitinib (Iressa®, AstraZeneca and Teva). Other examples of small molecule inhibitors of EGFR include erlotinib HCL (OSI-774; Tarceva®; OSI Pharma), lapatinib (Tykerb®, GlaxoSmithKline), canertinib (PD183805; Pfizer), PKI-166 (Novartis); PD158780; pelitinib; and AG 1478 (4-(3-Chloroanillino)-6,7-dimethoxyquinazoline).

In another embodiment, the ErbB pathway inhibitor is a HER2 inhibitor (i.e., an inhibitor that inhibits HER2 pathway signaling).

In one embodiment, the HER2 inhibitor comprises an anti-HER2 antibody. An example of an anti-HER2 antibody is trastuzumab (Herceptin®). Herceptin is commercially available from Genentech, Inc. Another example of an anti-HER2 antibody is pertuzumab (Omnitarg®; Genentech).

In another embodiment, the HER2 inhibitor comprises a small molecule inhibitor of the HER2, such as a tyrosine kinase inhibitor (TKI) that inhibits HER2 signaling. Non-limiting examples of small molecule inhibitors of HER2 signaling include lapatinib (Tykerb®, GlaxoSmithKline), CI-1033 (PD 183805; Pfizer), PKI-166 (Novartis) and pelitinib EKB-569.

### III. Methods

In one aspect, a method is provided for overcoming resistance to an ErbB pathway inhibitor in a subject, the method comprising:
selecting a subject exhibiting resistance to an ErbB pathway inhibitor; and
administering to the subject (i) an ErbB3 inhibitor and (ii) an ErbB pathway inhibitor.

The ErbB pathway inhibitor administered to the subject does not need to be the same ErbB pathway inhibitor to which the subject has been demonstrated to be resistant, although typically, the ErbB pathway inhibitor administered to the subject will be directed against the same ErbB pathway as the ErbB pathway inhibitor to which the subject has been demonstrated to be resistant. For example, a subject who exhibits resistance to the EGFR pathway inhibitor gefitinib may be co-administered an ErbB3 inhibitor and an EGFR inhibitor, which EGFR inhibitor can be, for example, gefitinib or the anti-EGFR antibody cetuximab.

The ErbB3 inhibitor and the ErbB pathway inhibitor can be co-administered to the subject or, alternatively, the ErbB3 inhibitor can be administered prior to administration of the ErbB pathway inhibitor. The ErbB3 inhibitor and the ErbB pathway inhibitor can be administered to the subject by any route suitable for the effective delivery of the inhibitor to the subject. For example, many small molecule inhibitors are suitable for oral administration. Antibodies and other biologic agents typically are administered intravenously, intraperitoneally or intramuscularly.

Identification of a subject exhibiting resistance to an ErbB pathway inhibitor can be achieved through standard methods well known in the art. For example, the inability (or reduced ability) of the ErbB pathway inhibitor to inhibit the growth and/or proliferation of tumor cells in the subject (or a sample of tumor cells from the subject cultured *in vitro*) can be indicative of resistance.

In some embodiments, the resistance to the ErbB pathway inhibitor is acquired resistance, e.g., acquired resistance to an EGFR inhibitor, e.g., acquired resistance to gefitinib. In another embodiment, the acquired resistance to an EGFR inhibitor is associated with a T790M EGFR mutation in the subject, e.g., in a tumor in the subject. In another embodiment, the acquired resistance is to a HER2 inhibitor.

The resistance exhibited by the subject may be resistance associated with reactivation of PI3K/AKT signaling and the ErbB3 inhibitors inhibit PI3K/AKT signaling. Methods for assessing the ability of an ErbB3 inhibitor to inhibit PI3K/AKT signaling are known in the art, such as the assays described in detail in the Examples. For example, the ability of the ErbB3 inhibitor to inhibit phosphorylation of AKT can be assessed using standard techniques known in the art.

In another embodiment, the ErbB3 inhibitor administered to the subject is an anti-ErbB3 antibody. A useful anti-ErbB3 antibody comprises MM-121, comprising V_{H} and V_{L} sequence as shown in SEQ ID NOs: 1 and 2, respectively, or an antibody comprising VH CDR1, 2 and 3 sequences as shown in SEQ ID NOs: 3-5, respectively, and V_{L} CDR1, 2 and 3 sequences as shown in SEQ ID NOs: 6-8, respectively (i.e., the V_{H} and V_{L} CDRs of MM-121). In another embodiment, the anti-ErbB3 antibody is an antibody having heavy and light chains comprising the amino acid sequences set forth in SEQ ID NOs 42 and 43, respectively. Additional non-limiting exemplary anti-ErbB3 antibodies and other forms of ErbB3 inhibitors are described in detail in Subsection I above.

In another embodiment, the ErbB pathway inhibitor administered to the subject is an EGFR inhibitor. A useful EGFR inhibitor is an anti-EGFR antibody, e.g., cetuximab. Additional non-limiting exemplary anti-EGFR antibodies are described in detail in Subsection II above.
In another embodiment, the ErbB pathway inhibitor administered to the subject is a small molecule inhibitor of EGFR signaling as described in Subsection II above. A useful small molecule inhibitor of EGFR signaling is gefitinib. Additional non-limiting exemplary small molecule inhibitors of EGFR signaling are described in detail in Subsection II above.

In yet another embodiment, the ErbB pathway inhibitor administered to the subject is a HER2 inhibitor. Useful HER2 inhibitors include lapatinib and anti-HER2 antibodies, e.g., trastuzumab. Additional non-limiting exemplary anti-HER2 inhibitors are described in detail in Subsection II above.

In another aspect, a method of inhibiting growth, invasiveness or metastasis of a tumor is provided, wherein the tumor comprises cells comprising a T790M EGFR mutation, the method comprising contacting the tumor (or cells thereof) with (i) an EGFR inhibitor; and (ii) an ErbB3 inhibitor.

In certain embodiments, the ErbB3 inhibitor inhibits PI3K/AKT signaling. Methods for assessing the ability of an ErbB3 inhibitor to inhibit PI3K/AKT signaling are known in the art, such as the assays described in detail in the Examples. For example, the ability of the ErbB3 inhibitor to inhibit phosphorylation of AKT can be assessed using standard techniques known in the art.

In one embodiment, the tumor (or cells thereof) is contacted with an ErbB3 inhibitor comprising an anti-ErbB3 antibody as described in detail in Subsection I above.

In another embodiment, the tumor (or tumor cells) is contacted with an EGFR inhibitor comprising an anti-EGFR antibody as described in detail in Subsection II above. In another embodiment, the tumor (or tumor cells) is contacted with an EGFR inhibitor comprising a small molecule inhibitor of EGFR signaling, as described in detail in Subsection II above.

In yet another aspect, a method of treating a tumor in a subject is provided, the method comprising
selecting a subject with a tumor comprising a T790M EGFR mutation, and
administering to the subject (i) an EGFR inhibitor; and (ii) an ErbB3 inhibitor.

The ErbB3 inhibitor and the EGFR inhibitor can be co-administered to the subject or, alternatively, the ErbB3 inhibitor can be administered prior to administration of the EGFR inhibitor. The ErbB3 inhibitor and the EGFR inhibitor can be administered to the subject by any route suitable for the effective delivery of the inhibitor to the subject. For example, many small molecule inhibitors are suitable for oral administration. Antibodies and other biologic agents typically are administered intravenously, intraperitoneally or intramuscularly.

Identification of a subject with a tumor comprising a T790M EGFR mutation can be achieved using methods well known in the art, such as by analysis (e.g., by PCR and sequencing) of genomic DNA in, or cDNA encoding EGFR prepared from, a sample of tumor cells (e.g., a biopsy) from the subject.

A useful ErbB3 inhibitor is one that inhibits PI3K/AKT signaling. Methods for assessing the ability of an ErbB3 inhibitor to inhibit PI3K/AKT signaling are well known in the art, such as the assays described in detail in the Examples.

The ErbB3 inhibitor administered to the subject may comprise an anti-ErbB3 antibody, e.g., MM-121, which comprises V_{H} and V_{L} sequences as shown in SEQ ID NOs: 1 and 2, respectively, or the ErbB3 inhibitor may be an antibody comprising V_{H} CDR1, 2 and 3 sequences as shown in SEQ ID NOs: 3-5, respectively, and V_{L} CDR1, 2 and 3 sequences as shown in SEQ ID NOs: 6-8, respectively (i.e., the V_{H} and V_{L} CDRs of MM-121). Additional non-limiting exemplary anti-ErbB3 antibodies and other forms of ErbB3 inhibitors are described in detail in Subsection I above.

In another embodiment, the EGFR inhibitor administered to the subject comprises an anti-EGFR antibody as described in detail in Subsection II above. In another embodiment, the EGFR inhibitor administered to the subject comprises a small molecule inhibitor of EGFR signaling as described in detail in Subsection II above.

The methods disclosed herein are suitable for use in overcoming resistance to ErbB pathway inhibitors in essentially any diseases or disorders in which such resistance is observed, although the disease for which the methods are used is typically a cancer. In situations in which the resistance is to an EGFR inhibitor, the cancer typically is selected from the group consisting of lung cancer (e.g., non-small cell lung cancer), colorectal cancer, head and neck cancer and pancreatic cancer. In situations in which the resistance is to a HER2 inhibitor, the cancer typically is breast cancer.

### IV. Pharmaceutical Compositions

In another aspect, pharmaceutical compositions are provided that can be used in the methods provided herein, i.e., pharmaceutical compositions for overcoming resistance to an ErbB pathway inhibitor. The pharmaceutical compositions typically comprise an ErbB3 inhibitor (as described in detail in Section I above), an ErbB pathway inhibitor (as described in detail in section II above) and a pharmaceutical carrier. The ErbB3 inhibitor and the ErbB pathway inhibitor can be formulated with the pharmaceutical carrier into a single composition. Alternatively, the ErbB3 inhibitor can be formulated with a pharmaceutical carrier to form a first composition, the ErbB pathway inhibitor can be formulated with a pharmaceutical carrier to form a second composition, and the first and second composition can be packaged together. Additionally, the pharmaceutical composition can include, for example, instructions for use of the composition to overcome resistance to an ErbB pathway inhibitor.

As used herein, "pharmaceutically acceptable carrier" includes any and all solvents, dispersion media, coatings, antibacterial and antifungal agents, isotonic and absorption delaying agents, buffers, and other excipients that are physiologically compatible. Preferably, the carrier is suitable for parenteral, oral, or topical administration. Depending on the route of administration, the active compound, e.g., small molecule or biologic agent, may be coated in a material to protect the compound from the action of acids and other natural conditions that may inactivate the compound.

Pharmaceutically acceptable carriers include sterile aqueous solutions or dispersions and sterile powders for the extemporaneous preparation of sterile injectable solutions or dispersion, as well as conventional excipients for the preparation of tablets, pills, capsules and the like. The use of such media and agents for the formulation of pharmaceutically active substances is known in the art. Except insofar as any conventional media or agents are incompatible with the active compound, use thereof in the pharmaceutical compositions disclosed herein is contemplated. Supplementary active compounds can also be incorporated into the compositions.

A pharmaceutically acceptable carrier can include a pharmaceutically acceptable antioxidant. Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Examples of suitable aqueous and nonaqueous carriers which may be employed in the pharmaceutical compositions disclosed herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In many cases, it will be useful to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

These compositions may also contain functional excipients such as preservatives, wetting agents, emulsifying agents and dispersing agents.

Therapeutic compositions typically must be sterile, non-pyrogenic, and stable under the conditions of manufacture and storage. The composition can be formulated as a solution, microemulsion, liposome, or other ordered structure suitable to high drug concentration.

Sterile injectable solutions can be prepared by incorporating the active compound in the required amount in an appropriate solvent with one or a combination of ingredients enumerated above, as required, followed by sterilization, *e.g*., by microfiltration. Generally, dispersions are prepared by incorporating the active compound into a sterile vehicle that contains a basic dispersion medium and the required other ingredients from those enumerated above. In the case of sterile powders for the preparation of sterile injectable solutions, exemplary methods of preparation are vacuum drying and freeze-drying (lyophilization) that yield a powder of the active ingredient plus any additional desired ingredient from a previously sterile-filtered solution thereof.

Prevention of presence of microorganisms may be ensured both by sterilization procedures, supra, and by the inclusion of various antibacterial and antifungal agents, for example, paraben, chlorobutanol, phenol sorbic acid, and the like.

Pharmaceutical compositions disclosed herein can also be administered combination with other agents besides the ErbB3 inhibitors and ErbB pathway inhibitors described herein. For example, the combination therapy can include a combination of an ErbB3 inhibitor and an ErbB pathway inhibitor and at least one or more additional therapeutic agents, such as one or more chemotherapeutic agents known in the art. The pharmaceutical compositions and combinations thereof disclosed herein can also be administered in conjunction with radiation therapy and/or surgery.

Dosage regimens are adjusted to provide the optimum desired response (*e.g.*, a therapeutic response). For example, a single bolus may be administered, several divided doses may be administered over time or the dose may be proportionally reduced or increased as indicated by the exigencies of the therapeutic situation.

It is especially advantageous to formulate parenteral compositions disclosed herein in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms are dictated by and directly dependent on (a) the unique characteristics of the active compound and the particular therapeutic effect to be achieved, and (b) the limitations inherent in the art of compounding such an active compound for the treatment of sensitivity in individuals.

Actual dosage levels of the active ingredients in the pharmaceutical compositions provided herein may be varied so as to obtain an amount of the active ingredient which is effective to achieve the desired therapeutic response for a particular patient, composition, and mode of administration, without being toxic to the patient.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

The active compound may be mixed under sterile conditions with a pharmaceutically acceptable carrier, and with any preservatives, buffers, or propellants which may be required.

When compounds provided herein are administered as pharmaceuticals, to humans or animals, they can be given alone or as a pharmaceutical composition containing, for example, 0.001 to 90% (e.g., 0.005 to 70% or 0.01 to 30%) of active ingredient in combination with a pharmaceutically acceptable carrier.

The following examples should not be construed as further limiting the above disclosure. Each and every patent application and publication and issued patent cited herein is incorporated herein by reference in its entirety.

### Examples

### Part I: Use of anti-ErbB3 Antibodies for Overcoming Resistance to ErbB Pathway Inhibitors

### Example 1: MM-121 Blocks Ligand Induced Activation of ErbB3

In this example, the ability of the anti-ErbB3 monoclonal antibody MM-121 (Ab #6, as disclosed in U.S. Patent No. 7,846,440) to inhibit ligand-induced activation of ErbB3 phosphorylation and signaling was examined in a series of *in vitro* experiments.

### In Vitro Signaling Studies Measured by ELISA

In the first set of experiments, three cancer cell lines (ACHN (renal cell adenocarcinoma, ATCC® #CRL-161™), Du145 (prostate carcinoma, ATCC® #HTB-81™) and OvCAR 8 (ovarian adenocarcinoma, ATCC® #HTB-161™) cell lines; obtained from the National Cancer Institute's Developmental Therapeutics Program) were seeded at 35,000 cells per well in 96-well plates and grown overnight (maintenance culture medium was RPMI-1640 media supplemented with 10% fetal calf serum, 2mM L-glutamine, and Pen-Strep; cells were grown in a humidified atmosphere at 5% CO₂, 95% air at 37° C). Cells were synchronized by 20-24 hour serum starvation. The cells were then pre-incubated with 4-fold serial dilutions, ranging from 2 mM to 7.6 pM, of MM-121 for 30 minutes. The cells then were stimulated with 25 nM heregulin (HRG)-1beta for 10 minutes, washed once with cold PBS and lysed in MPER lysis buffer (Pierce Chemical Co.).

For ELISA analysis of cell lysates, capture antibodies against ErbB 1 (R&D Systems AF231), ErbB2 (R&D Systems, MAB1129), ErbB3 (R&D Systems, MAB 3481) and AKT (Upstate, 05-591MG) were incubated in 384 well black flat-bottom polystyrene high-binding plates (Corning, 3708) overnight at room temperature. ELISAs were blocked with 2% bovine serum albumin (BSA) in phosphate buffered saline (PBS) for one hour and then incubated with lysates diluted in 2% BSA, 0.1% Tween-20 and PBS for two hours at room temperature. Between incubation steps, plates were washed three times with 0.5% Tween-20 in PBS. ELISAs measuring phospho-ErbB1, -ErbB2 and -ErbB3 were incubated with phosphor-tyrosine horseradish peroxidase (HRP) linked monoclonal antibody (R&D Systems, HAM1676) for two hours. ELISAs measuring phosphor-AKT were incubated with primary serine 473 specific anti-phospho AKT mouse monoclonal antibody (Cell Signaling Technologies, 5102) for two hours, then incubated with streptavidin-HRP (DY998) for 30 minutes. All ELISAs were visualized with SuperSignal® ELISA Pico Chemiluminescent Substrate (Pierce, Cat. #37069) and luminescent signal was measured using a luminometer.

ELISA analysis of the cell lysates is summarized in the graphs shown in Figure 1. The data demonstrate that MM-121 inhibits HRG-induced ErbB3, AKT and ERK phosphorylation in comparison to the 25 nM HRG control without MM-121. Inhibitor IC₅₀ values were calculated by least-squares fitting the dose response data with a sigmoidal curve (GraphPad Prism®, GraphPad Software, Inc., La Jolla, CA). In most cases, maximal inhibition of pErbB3, pAKT and pERK with MM-121 occurred near or below basal signaling levels as measured by the unstimulated control cells. Thus, the results of Figure 1 demonstrate that in ACHN, Du145 and OvCAR8 cells, MM-121 blocked the capacity of heregulin to stimulate ErbB3 and downstream AKT and ERK phosphorylation and reduced signaling levels to equal or below the unstimulated cells. **Example 2: MM-121 Overcomes Resistance to Erlotinib in *In-Vitro* Models of**

### EGFR-Wild-Type Non-Small Cell Lung Cancer

### Methods

Nine non-small cell lung cancer (NSCLC) cell lines were obtained from the American Type Culture collection: A549 (ATCC® #CCL-185™), EKVX (NCI vial 0502454), NCI-H2170 (ATCC® #CRL-5928™), NCI-H2347 (ATCC® #CRL-5942™), NCI-H322M (ATCC® # CRL-5806™), NCI-H358 (ATCC® # CRL-5806™), NCI-H441 (ATCC® #HTB-174™), NCI-H661 (ATCC® #HTB-183™) and SW-900 (ATCC® #HTB-59™). The cell lines bear no mutations within their EGFR genes and represent three distinct histological subtypes (see Table X below). Cells harboring Ras mutations are indicated.

Cells were seeded at 5,000 cells per well in a 96-well 3D-culture system (low-binding NanoCulture® plates, Scivax Corporation) and grown in RPMI-1640 medium supplemented with 10% fetal bovine serum and Pen-Strep at 37 °C. 48 h later, after which time spheroids had formed, serum was reduced to 2% and cells were incubated with ligands and drugs for 6 days at 37 °C. Relative live cell densities were then determined using the CellTiter Glo® reagent (Promega).

Two sets of experiments were performed: in one set, cells were treated with EGF (10 nM), heregulin1-β1 (HRG, 10 nM), a pool of the two ligands (10 nM each ligand), or in the absence of exogenous ligands, as well as with erlotinib (1 µM), MM-121 (1 µM), a combination of the two drugs (1 µM each drug), or in the absence of drugs. In a second set of experiments, cells were treated with doses of erlotinib ranging from 0 to 10 µM, in either the absence or presence of 10 nM heregulin -1β1, and in either the absence or presence of 1 µM MM 121.

**Table 1. EGFR wild-type NSCLC cell lines**

| **Cell Line** | **EGFR mutation status** | **K-Ras/H-Ras/N-Ras mutation status** | **Histological subtype** |
|---|---|---|---|
| **A549** | wild-type | K-Ras G12S | adenocarcinoma |
| **EKVX** | wild-type | WT | adenocarcinoma |
| **H2170** | wild-type | WT | squamous cell carcinoma |
| **H2347** | wild-type | N-Ras Q61R | adenocarcinoma |
| **H322M** | wild-type | WT | adenocarcinoma |
| **H358** | wild-type | K-Ras G12C | adenocarcinoma |
| **H441** | wild-type | K-Ras G12V | adenocarcinoma |
| **H661** | wild-type | WT | large cell carcinoma |
| **SW-900** | wild-type | K-Ras G12V | squamous cell carcinoma |

### Results

Erlotinib (Tarceva®) is indicated for treatment of patients with non-small cell lung cancer and has been shown to be effective in patients with tumors harboring EGFR mutations; however, patients with tumors having wild-type EGFR show poor response rates to erlotinib in the clinic, and some patients on EGFR therapy develop resistance due to a new T790M mutation appearing (Hammerman et al., (2009) Clinical Cancer Research 15(24), 7502-7509. *In vitro,* EGFR wild-type NSCLC cell lines are substantially less sensitive to erlotinib, having GI₅₀'s several orders of magnitude higher than cell lines bearing EGFR-activating mutations. Activating mutations in Ras oncogenes (H-, N-, and K-Ras) have also been shown to be associated with lung cancer, particularly K-Ras mutations in lung adenocarcinomas. NSCLC subtypes other than adenocarcinoma are also found to correlate with poor response to erlotinib in clinical trials.

In order to demonstrate the effectiveness of MM-121 in overcoming resistance to erlotinib in EGFR wild-type NSCLC cells having multiple histological subtypes, MM-121 was tested in nine EGFR wild-type NSCLC cell lines having adenocarcinoma, squamous cell carcinoma, or large cell carcinoma histological subtypes. The cell lines are either wild-type for the Ras oncogene or harbor K-Ras or N-Ras mutations as indicated in Table 1.

Five cell lines were each grown as spheroids and treated as described above with epidermal growth factor (EGF), heregulin1-β1 (HRG), a pool of ligands (EGF + HRG), or no exogenous ligands (medium), as well as with erlotinib, MM-121, a combination of erlotinib and MM-121, or no drug treatment. Tumor size was then determined by measuring live cell density. As shown in Figure 2, erlotinib inhibited cell growth in the absence of exogenous ligands and in the presence of EGF, but was less effective at inhibiting growth of cells grown in the presence of HRG. While the combination of MM-121 and erlotinib was in some cell lines more effective at inhibiting cell growth in the EGF-stimulated cells, MM-121 was able to greatly improve sensitivity of the cells to erlotinib in HRG-stimulated cells. This was true for all cell spheroids tested, including adenocarcinoma cell lines NCI-H322M, Figure 2A; EKVX, Figure 2B; A549, Figure 2C; H358, Figure 2D, and squamous cell line SW-900, Figure 2E. The mutation state of the K-Ras gene did not impact the effectiveness of MM-121 in increasing sensitivity of the cells to erlotinib.

Eight cell lines were then each grown as spheroids as described above and treated with doses of erlotinib ranging from 0 to 10 µM, in either the absence or presence of heregulin-1β1 (HRG), and in either the absence or presence of MM 121. As shown in Figure 3, while erlotinib was not able to inhibit cell growth in HRG-stimulated cells except at high concentrations; however, addition of MM-121 was able to restore sensitivity of the spheroids to erlotinib in the presence of HRG. This was true for all cell spheroids tested, including adenocarcinoma cell lines NCI-H322M (Figure 3A); EKVX (Figure 3B); A549 (Figure 3C); NCI-H358 (Figure 3D); NCI-H441 (Figure 3E), NCI-H2347 (Figure 3F); the squamous cell carcinoma cell line NCI-H2170 (Figure 3G), and the large cell carcinoma cell line NCI-H661 (Figure 3H). The mutation state of the K-Ras gene did not affect the effectiveness of MM-121 in increasing sensitivity of the cells to erlotinib.

These results demonstrate that HRG-mediated signaling may play a part in resistance to erlotinib in EGFR wild-type cells. The data further demonstrate that MM-121 is effective in restoring sensitivity of tumor cells to erlotinib in combination therapy.

### Example 3: Inhibition of pAKT production in human ovarian cancer cells in vitro

### Materials and Methods:

The A2780 human ovarian cancer cell line was originally established from tumor tissue from an untreated patient. The A2780cis cell line is cisplatin-resistant (Cat. # 93112517, Sigma). It was developed by chronic exposure of the parent cisplatin-sensitive A2780 cell line (Cat. # 93112519, Sigma) to increasing concentration of cisplatin. A2780cis is cross-resistant to melphalan, adriamycin and irradiation. In order to maintain resistance, cisplatin is added to the culture media every 2-3 passages, post-attachment.

Resistance to cisplatin is confirmed by treating A2780 and A2780cis cells for 72 hours with a serial dilution of cisplatin (0.01 to 10 µM). Cell viability is measured using the Cell Titer Glo assay (Cat. # G7570, Promega) according to the manufacturer's instructions.

The effect of cisplatin on the AKT pathway is determined by treating sensitive (A2780) and resistant (A2780cis) cells with a dose range of cisplatin (0.1 to 10 µM) *in vitro* for 1, 4, 24 or 72 hours. After incubation cell lysates are prepared and analyzed for pAKT (ser473) (Cat # 9271 Cell Signaling Technologies) by western blot.

The effect of MM-121 on the AKT pathway is determined by treating sensitive (A2780) and resistant (A2780cis) cells with a dose range of MM-121 ((0.01 to 1 µM) *in vitro* for 1, 4, 24 or 72 hours. After incubation cell lysates are prepared and analyzed for pAKT (ser473) (Cat. # 9271 Cell Signaling Technologies) by western blot.

### Results:

Resistance of the cell line A2780cis to treatment to cisplatin was evaluated by the methods described above or minor variations thereof. Resistant A2780cis and sensitive A2780 cells were plated and treated for 72 hours *in vitro* with a serial dilution of cisplatin (0.01 to 10 µM). Cell viability was measured by the Cell Titer Glo assay which measures metabolically active cells by quantitating ATP. As shown in Figure 4, the A2780cis cells had a much greater viability (shown as percentage of media control) than did the sensitive line, A2780.

In order to characterize the effect of cisplatin on the AKT pathway *in vitro,* A2780 and A2780cis cells were treated with a dose range of cisplatin as described above for 1, 4 (A2780cis only), 24, or 72 hours. Cells sensitive to cisplatin showed a decrease in pAKT production (Figure 8A) especially after 24 hours of treatment and/or at high cisplatin concentration (10 µM), whereas the cisplatin resistant cells showed no effect on pAKT production (figure 8B). As shown in Figure 8C, these cells did show a reduction in AKT phosphorylation after treatment with MM-121. A2780cis cells were treated with a dose range of MM-121 as described above for 1, 4, 24, or 72 hours. Cells showed a gradual decrease in pAKT production at all dose levels and increasing over time.

### Example 4: MM-121 rescues cisplatin resistance in a human ovarian cancer cell line in vitro

In order to determine whether MM-121 can rescue the cisplatin-resistant phenotype of A2780cis cells in vitro, resistant A2780cis and sensitive A2780 cells will be plated as described above and treated with a dose range of cisplatin, MM-121, or a combination thereof for 1, 4, 24, or 72 hours. Cell lysates are analyzed by western blot for pAKT. Cells sensitive to cisplatin will show a reduction in pAKT levels after treatment with cisplatin and MM-121, and an even greater decrease in pAKT for the cells treated with both cisplatin and MM-121. Cells resistant to cisplatin will show no reduction in pAKT after treatment with cisplatin, and a moderate amount of reduction of pAKT after treatment with MM-121. A2780cis cells treated with a combination of MM-121 and cisplatin will show a greater decrease in the amount of pAKT after treatment than cells treated with MM-121 alone, suggesting an additive effect on the inhibition PI3K/AKT signaling and a restoration of sensitivity to cisplatin.

### Part II: Use of bispecific anti-ErbB3, anti-ErbB2 Antibodies for Overcoming Resistance to ErbB Pathway Inhibitors

### Methods

### In vitro breast cancer model

BT474-M3 cells (see Noble, Cancer Chemother. Pharmacol. 2009 64:741-51) are treated with dose ranges of lapatinib, trastuzumab or MM-111 in the presence or absence of 5 nM heregulin. Viable cells are counted following 6 days of treatment. The effect of MM-111 combined with lapatinib or trastuzumab on inhibition of AKT phosphorylation is assessed in heregulin-stimulated BT474-M3 cells across a dose range.

### In vivo breast cancer xenograft model

BT474-M3 cells (2 x 10⁷ cells per mice) are inoculated subcutaneously into Nu/Nu immunodeficient mice, which are implanted with an estrogen pellet (0.72mg; 60-day release) one day before the experiment. Tumors are measured after seven days and mice are randomized into four groups: those treated with placebo, MM-111 (66 mg/kg, q7d), lapatinib (150 mg/kg q1d), or a combination of MM-111 and lapatinib. Tumors are measured twice a week and the experiment is ended at day 40. BT474-M3 breast tumor xenograft models are also treated with MM-111 (3 mg/kg q3d), trastuzumab (1 mg/kg q7d) or a combination of both drugs at these doses.

### Development of trastuzumab-resistance cell line

To establish trastuzumab resistant cells, BT474-M3 cells are cultured in RPMI1640 medium with 100nM trastuzumab for six months, and 200nM trastuzumab for two months, and then the dose level is increased to 500nM. Cells are assayed in cell proliferation periodically to check the resistance level to trastuzumab.

### Cell proliferation assay

BT474-M3 parental (wild-type) and trastuzumab-resistant cells are plated in 96-well plates (3000 cells/well). After overnight incubation, cells are treated with a series dilution of trastuzumab or MM-111. After five days of treatment, cell viability is measured by WST-1 (Roche, Cat. # 5015944001) according to manufacturer's instructions. Cells treated with control (RPMI1640 with 10% FBS) are set as 100%, other treatments are expressed as percentage of the control.

### Flow cytometry analysis

To determine the receptor status on BT474-M3 parental and trastuzumab-resistant cells, cells are trypsinized and washed with FACS buffer. Cells are then incubated with Alexa Fluor® 647-labeled mouse anti-ErbB2 antibody (BioLegend® Cat. # 324412), cetuximab antibody (anti-EGFR), and B 12 antibody (anti-ErbB3) for 1 hour at 4°C. Following washing with FACS buffer, cells were analyzed by FACSCalibur™ (BD bioscience).

### Spheroid assay

BT474-M3 wild type and trastuzumab-resistant cells (2000 cells/well) are plated in Ultra Low Cluster 96-well plates (Costar®, Corning, NY). After overnight incubation, a series dilution of trastuzumab or MM-111 is introduced to the plate. Cells are cultured for six days. Spheroids are then examined by Nikon microscope and analyzed by MetaMorph® Image Analysis Software (Molecular Devices). The spheroid size from cells cultured in medium containing 10% FBS is set as a control.

### Example 5: The activities of Her2-directed agents trastuzumab and lapatinib are attenuated by heregulin stimulated ErbB3 signaling, while MM-111 remains active

The ability of MM-111, lapatinib and trastuzumab to inhibit cell proliferation in the presence of heregulin was tested. Under basal conditions it was found that lapatinib (Figure 6A), trastuzumab (Figure 6B) and MM-111 (Figure 6C) inhibited BT474-M3 cell proliferation by 50%, 32% and 24%, respectively. When cells were cultured in the presence of 5 nM heregulin the effect of both lapatinib and trastuzumab was decreased so that inhibition of cell proliferation was reduced to 23% and 9%, respectively. Conversely, the inhibition of tumor cell growth by MM-111 was retained when heregulin was present, with 33% growth inhibition observed.

### Example 6: The addition of MM-111 to lapatinib or trastuzumab increases pAKT inhibition

The ability of the combination of MM-111 and lapatinib or MM-111 and trastuzumab to inhibit AKT phosphorylation (activation) in vivo was investigated. While it was found that lapatinib alone inhibited phosphorylation of AKT in the presence of heregulin, the combination of MM-111 and lapatinib was extremely effective, inhibiting phosphorylation of AKT well below basal levels at therapeutically relevant concentrations (Figure 7A). Trastuzumab did not inhibit AKT phosphorylation following heregulin stimulation (Figure 7B). However, the addition of MM-111 to trastuzumab improved the inhibition of heregulin-stimulated AKT phosphorylation that was observed for trastuzumab alone, with inhibition of pAKT almost to basal levels, suggesting an additive effect of the combination (Figure 7B).

### Example 7: The addition of MM-111 to lapatinib or trastuzumab potentiates in vivo activity

The combination of MM-111 with trastuzumab or lapatinib was investigated in vivo using the BT474-M3 breast cancer xenograft model. Sub-optimal monotherapy doses of MM-111 (3 mg/kg dosed every 3 days) and trastuzumab (1 mg/kg dosed weekly), were selected for combination experiments to allow observation of any differences in activity between monotherapy and combination groups. MM-111 administered at 3 mg/kg every 3 days provided similar exposure to a weekly dose of 1 mg/kg trastuzumab due to the different pharmacokinetic properties of each agent in mice. Tumor growth inhibition in groups dosed with the combination of 3 mg/kg MM-111 and 1 mg/kg trastuzumab was more potent, and reached statistical significance, compared to MM-111 alone and trastuzumab alone. Additionally, an increase in the number of completely regressed tumors was observed with combination treatment compared to the monotherapy treatment groups, in which no complete regressions were observed (Figure 8A). MM-111 and lapatinib were each dosed at an optimal efficacious dose weekly and every day, respectively. The combination of MM-111 and lapatinib provided more potency compared to either drug alone (Figure 8B).

### Example 8: MM-111 is active in a trastuzumab-resistant cell line

Flow cytometry analysis was performed to determine receptor status in wild-type and trastuzumab-resistant BT474-M3 cell lines. Wild-type or trastuzumab-resistant cells were stained with Alexa Fluor@ 647-labeled mouse anti-ErbB2 antibody, cetuximab antibody (anti-EGFR), or B12 antibody (anti-ErbB3). Trastuzumab-resistant cells had a slightly decreased ErbB2 level (Figure 9A) while EGFR (Figure 9B) and ErbB3 (Figure 9C) were unchanged.

To determine the efficacy of MM-111 in inhibiting trastuzumab-resistant BT474-M3 cell proliferation, parental wild-type and trastuzumab-resistant BT474-M3 cells were treated as described above with a series dilution of either MM-111 or trastuzumab. While trastuzumab significantly inhibited cell proliferation in the parental cells, its inhibitory effect was significantly reduced in trastuzumab-resistant cells (Figure 10A). In contrast, MM-111 maintained similar inhibitory activity in both parental and trastuzumab-resistant cells (Figure 10B), thus demonstrating that MM-111 is able to circumvent the resistance mechanisms developed by cells after repeated exposure to trastuzumab.

To further investigate the ability of MM-111 to inhibit cell growth in trastuzumab-resistant cells, multicellular spheroids of parental wild-type and trastuzumab-resistant BT474-M3 cells were prepared using the methods described above or minor variations thereof and treated with a series dilution of either MM-111 or trastuzumab. The inhibitory effect of trastuzumab was diminished in trastuzumab-resistant BT474-M3 cells, although it significantly inhibited spheroid growth of BT474-M3 parental cells (Figure 11A). In contrast, MM-111 significantly reduced spheroid growth of both trastuzumab-resistant and wild-type BT474-M3 cells (Figure 11B). Its inhibitory activity in trastuzumab-resistant cells was slightly improved when compared to its inhibitory activity in wild-type cells.

The data in the preceding Examples demonstrate that MM-111 is effective at inhibiting cell growth in cells that have developed resistance to trastuzumab.

### Example 9: MM-111 but not trastuzumab combines with anti-EGFR therapeutics in trastuzumab-resistant BT474-M3 cells

To compare the ability of MM-111 and trastuzumab to inhibit cell growth when in combination with EGFR inhibitors, spheroids of trastuzumab-resistant BT474-M3 cells were prepared using the methods described above and treated with a series of dilution of MM-111 and trastuzumab in the presence of either 300nM erlotinib (Figure 12A) or 100nM gefitinib (Figure 12B).". As shown in Figure 12A and 12B, MM-111 but not trastuzumab was able to combine with erlotinib or gefitinib to reduce cell growth in trastuzumab-resistant cell spheroids. These data demonstrate that the combination MM-111 and an EGFR inhibitor is effective at inhibiting cell growth in cells that have developed resistance to trastuzumab. Furthermore, the combination of trastuzumab with an EGFR inhibitor was not sufficient to overcome the resistance of the cells.

### Example 10: Combination therapy with MM-111, lapatinib and tamoxifen in BT474-M3 tumor xenografts with and without heregulin

### Methods

15x10⁶ BT474-M3 cells engineered to express GFP (BT474-M3-GFP) or BT474-M3 cells engineered to express GFP and heregulin 1 (BT474-M3-GFP-HRG) were implanted in to the mammary fat pads of estrogen supplemented (0.72mg 17β-estradiol in a 60-day slow release biodegradable carrier) female NCr/NU -mice (Taconic Farms, Inc). When tumor volumes reached on average 516 mm³ (BT474-M3-GFP-HRG on day 17 after tumor implantation) or 422 mm³ (BT474-M3-GFP on day 20 after tumor implantation), mice were segregated into 8 groups of 10-15 mice. Groups received either no treatment (Control), MM-111 (48 mpk q3d i.p. (dose every three days, intraperitoneally)), lapatinib (150 mpk qd p.o. (oral dose daily)), tamoxifen (5mg free base tamoxifen in a 60-day slow release biodegradable carrier), MM-111 and lapatinib, MM-111 and tamoxifen, lapatinib and tamoxifen or MM-111, lapatinib and tamoxifen. Tumors were measured twice a week with a digital caliber.

At the end of study (21 or 25 days after initiation of treatment for BT474-M3-GFP-HRG and BT474-M3-GFP, respectively) tumor samples were collected (24h after the last MM-111 and 6h after the last lapatinib dose) and analyzed for target and downstream signaling inhibition. Total and phosphorylated ErbB3 and Akt protein levels were analyzed from tumor lysates by suspension array technology (Luminex) and total and phosphorylated Erkl/2 levels by western blot using PCNA to normalize the results.

### Results

In order to demonstrate the effectiveness of MM-111 combination therapies to reduce tumor growth of heregulin (HRG) stimulated cells *in vivo,* combination therapies were tested in the BT474-M3-GFP and BT474-M3-GFP-HRG xenograft model according to the methods above. As shown in Figure 13A, BT474-M3-GFP and BT474-M3-GFP-HRG tumor-bearing mice were treated with MM-111 (48 mpk), lapatinib (150 mpk) and tamoxifen (5 mg) mono therapies. Tumoral HRG overexpression improved tamoxifen efficacy and modestly improved MM-111 efficacy. BT474-M3-GFP and BT474-M3-GFP-HRG tumor bearing mice were then treated with MM-111 (48 mpk) + lapatinib (150 mpk), MM-111 + tamoxifen (5 mg), and lapatinib + tamoxifen combination therapies. As shown in Figure 13B, tumoral HRG overexpression modestly improved efficacy of the MM-111 + tamoxifen combination. BT474-M3-GFP and BT474-M3-GFP-HRG tumor bearing mice were then treated with the combination of lapatinib + tamoxifen and MM-111 + lapatinib + tamoxifen combination therapies. As shown in Figure 13C, MM-111 greatly enhanced the efficacy of the lapatinib + tamoxifen combination therapy in both tumor models, demonstrating that MM-111 is required for the maximum anti-tumor efficacy regardless of the tumoral HRG expression.

BT474-M3-GFP and BT474-M3-GFP-HRG tumor bearing mice (Figure 14 left and right panels, respectively) were then treated with (from left to right) control (no treatment), MM-111, lapatinib, and tamoxifen monotherapies, the dual combinations of MM-111 + lapatinib, MM-111 + tamoxifen, and lapatinib + tamoxifen, and the triple combination. As shown in Figure 14A which shows phospho-ErbB3 (pErbB3), tumoral HRG expression increased the ErbB3 phosphoprotein levels and lead to an increased effectiveness of MM-111 monotherapy and MM-111 combination therapy in reducing levels of pErbB3. Tumoral HRG expression lead to a decreased lapatinib and lapatinib + tamoxifen activity in reducing pErbB3 levels. As shown in Figure 14B, MM-111 monotherapy and MM-111 combination therapies increased total ErbB3 expression in both tumor models. As shown in Figure 14C, which shows the ratio of pErbB3 to total ErbB3 (tErbB3), both the MM-111 monotherapy and MM-111 combination therapy decreased ErbB3 activity even in the presence of HRG, whereas lapatinib and lapatinib + tamoxifen effectiveness was reduced in the presence of HRG.

As shown in Figure 14F, which shows the ratio of phospho-Akt (pAkt, Figure 14D) to total Akt (tAkt or totAkt, Figure 14E), tumoral expression of HRG increased the Akt phosphoprotein/total protein levels and resulted in decreased effectiveness of the lapatinib and lapatinib + tamoxifen therapies, whereas the MM-111 monotherapy and combination therapies were effective at reducing pAkt production in the presence of HRG.

As shown in Figure 14I, which shows the ratio of phospho-ERK (ERKt, Figure 14G) to total Akt (totERK, Figure 14H), tumoral expression of HRG lead to an increase in ERK1/2 phosphoprotein/total protein levels and resulted in decreased effectiveness of the lapatinib and lapatinib + tamoxifen therapies, whereas the MM-111 monotherapy and combination therapies were effective at reducing pERK production in the presence of HRG.

### Example 11: MM-111 in combination with the ToGA -regimen in NCI-N87 tumor xenografts

The ToGA Study (Hoffmann-La Roche, ClinicalTrials.gov Identifier NCT01041404) was a study of trastuzumab in combination with chemotherapy compared with chemotherapy alone in patients with HER2-positive advanced gastric cancer. In that study, trastuzumab was administered as intravenous infusion of 6 mg/kg (loading dose 8 mg/kg) every 3 weeks. The chemotherapy consisted of a combination of 6 cycles of fluorouracil (800 mg/m²/day intravenous infusion every 3 weeks) and cisplatin (80 mg/m² intravenous infusion every 3 weeks), or capecitabine (1000 mg/m² p.o. twice daily for 14 days every 3 weeks) and cisplatin (80 mg/m² intravenous infusion every 3 weeks). Treatment with trastuzumab continued until disease progression. This treatment regimen was repeated in an NCI-N87 gastric cancer xenograft model according to the methods below.

### Methods

7.5 x 10⁶ NCI-N87 cells (ATCC® # CRL-5822™) were implanted subcutaneously in the flanks of female Nu/Nu (Charles River Laboratories, Inc.) mice. When tumor volumes reached on average 325 mm³ (on day 18 after tumor implantation) mice were segregated into 4 groups of 8-35 mice.

MM-111 was dosed either as a first line therapy at the initial treatment of the mice or as a second line therapy, wherein MM-111 was added to the treatment regimen (see arrows, Figure 15A). Groups received either no treatment (Control), trastuzumab (3.5 mpk q3d i.p.) + 5-FU (12 mpk qd, 5 times per week, i.p.), trastuzumab (3.5 mpk q3d i.p.) + 5-FU (12 mpk qd, 5 times per week, i.p.) + cisplatin (5 mpk q7d i.p.) or 1^{st} line MM-111 (96 mpk q3d i.p.) + trastuzumab (3.5 mpk q3d i.p.) + 5-FU (12 mpk qd, 5 times per week, i.p.). Tumors were measured twice a week with a digital caliber.

At the time of continued tumor growth on day 29, the trastuzumab + 5-FU treatment group was divided into 2 treatment groups receiving either a) trastuzumab + 5-FU or b) 2^{nd} line MM-111 + trastuzumab + 5-FU (see arrow, Day 29). Similarly, at the time of continued tumor growth on day 54, the trastuzumab + 5-FU + cisplatin treatment group was divided into two treatment groups receiving either a) trastuzumab + 5-FU + cisplatin or b) 2^{nd} line MM-111 + trastuzumab + 5-FU + cisplatin (see arrow, Day 54). Cisplatin administration had to be discontinued on day 52 and 5-FU administration had to be discontinued on day 64 due to animals showing signs of toxicities due to the chemotherapeutics. The discontinuation of the chemotherapies is indicated with arrows Figure 15A.

### Results

Figure 15A shows the tumor growth curves of NCI-N87 tumors treated as described above. Figures 15B-D each highlight a subset of the data shown in Figure 15A. As shown in Figure 15B, the addition of MM-111 as a second line therapy given to mice being treated with trastuzumab + 5-FU resulted in an increased efficacy on tumor cell growth inhibition in tumors that had progressed on treatment with trastuzumab + 5-FU alone. Similarly, as shown in Figure 15C, the addition of MM-111 as a second line therapy given to mice being treated with trastuzumab + 5-FU + cisplatin resulted in an increased efficacy on tumor cell growth inhibition in tumors that had progressed (resisted treatment) on treatment with trastuzumab + 5-FU + cisplatin alone. Finally, as shown in Figure 15D, treatment with MM-111 as a first line therapy in combination with trastuzumab and 5-FU prevented tumor growth for the first 60 days of the treatment, in contrast to treatment with the combination of trastuzumab + 5-FU wherein the tumor volume increased from the beginning of the treatment..

### Example 12: MM-111 in combination with trastuzumab and paclitaxel in NCI-N87 tumor xenografts

### Methods

7.5 x 10⁶ NCI-N87 cells were implanted subcutaneously in the flanks of female Nu/Nu mice. When tumor volumes reached on average 341 mm³ (on day 24 after tumor implantation) mice were segregated into 4 groups of 10 mice. Groups received either no treatment (Control), paclitaxel (20 mpk q7d i.p.), trastuzumab (3.5 mpk q3d i.p.) + paclitaxel or MM-111 (48 mpk q3d i.p.) + trastuzumab + paclitaxel. Tumors were measured twice a week with a digital caliper.

### Results

As shown in Figure 16, the combination of MM-111 with trastuzumab + paclitaxel resulted in an increased efficacy on tumor cell growth inhibition in NCI-N87 tumors and resulted in continued tumor regression in contrast to paclitaxel alone and trastuzumab + paclitaxel, which caused only tumor stasis at best.

### Example 13: MM-111/lapatinib/trastuzumab in BT474-M3 (±HRG) tumor xenografts

### Methods

15 x 10⁶ BT474-M3 cells engineered to express GFP (BT474-M3-GFP) or BT474-M3 cells engineered to express GFP and heregulin 1 (BT474-M3-GFP-HRG) were implanted in to the mammary fat pads of estrogen supplemented (0.72mg 17β-estradiol in a 60-day slow release biodegradable carrier) female NCR/NU mice. When tumor volumes reached on average 286 mm³ (BT474-M3-GFP-HRG on day 14 after tumor implantation) or 305 mm³ (BT474-M3-GFP on day 16 after tumor implantation), mice were segregated into 8 groups of 8 mice. Groups received either no treatment (Control), MM-111 (48 mpk q3d i.p.), lapatinib (150 mpk qd p.o.), trastuzumab (3.5 mpk q3d i.p.), MM-111 + lapatinib, MM-111 + trastuzumab, lapatinib and trastuzumab or MM-111, lapatinib and trastuzumab. Tumors were measured twice a week with a digital caliber.

### Results

BT474-M3-GFP and BT474-M3-GFP-HRG tumor bearing mice were treated with MM-111, lapatinib and trastuzumab monotherapies (Figure 17A), MM-111 + lapatinib, MM-111 + trastuzumab, and lapatinib + trastuzumab combination therapies (Figure 17B), and lapatinib + trastuzumab and MM-111 + lapatinib + trastuzumab combination therapies (Figure 17C). As shown in Figure 17B, tumoral HRG overexpression increased the efficacy of the MM-111 + trastuzumab combination and decreased the efficacy of trastuzumab + lapatinib combination.

In addition, as shown in Figure 17C, MM-111 greatly enhanced the efficacy of the lapatinib + trastuzumab combination therapy in the BT474-M3-GFP-HRG tumor model, demonstrating that MM-111 is required for the maximum anti-tumor efficacy when BT474-M3 tumors overexpress heregulin 1.

The results in the preceding Examples demonstrate the effectiveness of MM-111 in combination treatments, both as a 1^{st} line therapy to prevent development of resistance to other therapeutics and a 2^{nd} line therapy to re-sensitize tumor cells to treatment with other therapeutics.

### Equivalents

Those skilled in the art will recognize, or be able to ascertain using no more than routine experimentation, many equivalents of the specific embodiments of the invention(s) described herein. Such equivalents are intended to be encompassed by the following claims. Any combination of one or more of the embodiments disclosed in any independent claim and any of the dependent claims is also contemplated to be within the scope of the invention.

### Incorporation by Reference

Each and every patent, pending patent application, and publication referred to herein is hereby incorporated herein by reference in its entirety.

### SUMMARY OF SEQUENCE LISTING

| |
|---|
| MM-121 (Ab # 6) V_{H} amino acid sequence (SEQ ID NO:1) |
| |
| MM-121 (Ab # 6) V_{L} amino acid sequence (SEQ ID NO:2) |
| |
| MM-121 (Ab # 6) V_{H} CDR1 (SEQ ID NO:3) |
| HYVMA |
| MM-121 (Ab # 6) V_{H} CDR2 (SEQ ID NO:4) |
| SISSSGGWTLYADSVKG |
| MM-121 (Ab # 6) V_{H} CDR3 (SEQ ID NO:5) |
| GLKMATIFDY |
| MM-121 (Ab # 6) V_{L} CDR1 (SEQ ID NO:6) |
| TGTSSDVGSYNVVS |
| MM-121 (Ab # 6) V_{L} CDR2 (SEQ ID NO:7) |
| EVSQRPS |
| MM-121 (Ab # 6) V_{L} CDR3 (SEQ ID NO:8) |
| CSYAGSSIFVI |
| MM-121 Heavy Chain Amino Acid Sequence (SEQ ID NO:42) |
| 1 EVQLLESGGG LVQPGGSLRL SCAASGFTFS HYVMAWVRQA PGKGLEWVSS 51 ISSSGGWTLY ADSVKGRFTI SRDNSKNTLY LQMNSLRAED TAVYYCTRGL 101 KMATIFDYWG QGTLVTVSSA STKGPSVFPL APCSRSTSES TAALGCLVKD 151 YFPEPVTVSW NSGALTSGVH TFPAVLQSSG LYSLSSVVTV PSSNFGTQTY 201 TCNVDHKPSN TKVDKTVERK CCVECPPCPA PPVAGPSVFL FPPKPKDTLM 251 ISRTPEVTCV VVDVSHEDPE VQFNWYVDGV EVHNAKTKPR EEQFNSTFRV 301 VSVLTVVHQD WLNGKEYKCK VSNKGLPAPI EKTISKTKGQ PREPQVYTLP 351 PSREEMTKNQ VSLTCLVKGF YPSDIAVEWE SNGQPENNYK TTPPMLDSDG 401 SFFLYSKLTV DKSRWQQGNV FSCSVMHEAL HNHYTQKSLS LSPGK |
| MM-121 Light Chain Amino Acid Sequence (SEQ ID NO:43) |
| 1 QSALTQPASV SGSPGQSITI SCTGTSSDVG SYNVVSWYQQ HPGKAPKLII 51 YEVSQRPSGV SNRFSGSKSG NTASLTISGL QTEDEADYYC CSYAGSSIFV 101 IFGGGTKVTV LGQPKAAPSV TLFPPSSEEL QANKATLVCL VSDFYPGAVT 151 VAWKADGSPV KVGVETTKPS KQSNNKYAAS SYLSLTPEQW KSHRSYSCRV 201 THEGSTVEKT VAPAECS |
| MM-121 (Ab # 6) Heavy Chain Nucleotide Sequence (SEQ ID NO:45) |
| gaggtgcagc tgctggagag cggcggaggg ctggtccagc caggcggcag cctgaggctg tcctgcgccg ccagcggctt caccttcagc cactacgtga tggcctgggt gcggcaggcc ccaggcaagg gcctggaatg ggtgtccagc atcagcagca gcggcggctg gaccctgtac gccgacagcg tgaagggcag gttcaccatc agcagggaca acagcaagaa caccctgtac ctgcagatga acagcctgag ggccgaggac accgccgtgt actactgcac caggggcctg |
| aagatggcca ccatcttcga ctactggggc cagggcaccc tggtgaccgt gagcagc |
| MM-121 (Ab # 6) Light Chain Nucleotide Sequence (SEQ ID NO:46) |
| cagtccgccc tgacccagcc cgccagcgtg agcggcagcc caggccagag catcaccatc agctgcaccg gcaccagcag cgacgtgggc agctacaacg tggtgtcctg gtatcagcag caccccggca aggcccccaa gctgatcatc tacgaggtgt cccagaggcc cagcggcgtg agcaacaggt tcagcggcag caagagcggc aacaccgcca gcctgaccat cagcggcctg cagaccgagg acgaggccga ctactactgc tgcagctacg ccggcagcag catcttcgtg atcttcggcg gagggaccaa ggtgaccgtc cta |
| |
| Ab # 3 V_{H} amino acid sequence (SEQ ID NO:9) |
| EVQLLESGGGLVQPGGSLRLSCAASGFTFSAYNMRWVRQAPGKGLEWVSVIYPSGGAT RYADSVKGRFTISRDNSKNTLYLQMNSLRAEDTAVYYCARGYYYYGMDVWGQGTLV TVSS |
| Ab # 3 V_{L} amino acid sequence (SEO ID NO:10) |
| QSVLTQPPSASGTPGQRVTISCSGSDSNIGRNYIYWYQQFPGTAPKLLIYRNNQRPSGVP DRISGSKSGTSASLAISGLRSEDEAEYHCGTWDDSLSGPVFGGGTKLTVL |
| Ab # 3 V_{H} CDR1 (SEQ ID NO:11) |
| AYNMR |
| Ab # 3 V_{H} CDR2 (SEQ ID NO:12) |
| VIYPSGGATRYADSVKG |
| Ab # 3 V_{H} CDR3 (SEQ ID NO:13) |
| GYYYYGMDV |
| Ab # 3 V_{L} CDR1 (SEQ ID NO:14) |
| SGSDSNIGRNYIY |
| Ab # 3 V_{L} CDR2 (SEQ ID NO:15) |
| RNNQRPS |
| Ab # 3 V_{L} CDR3 (SEQ ID NO:16) |
| GTWDDSLSGPV |
| |
| Ab # 14 V_{H} amino acid sequence (SEQ ID NO:17) |
| EVQLLESGGGLVQPGGSLRLSCAASGFTFSAYGMGWVRQAPGKGLEWVSYISPSGGHT KYADSVKGRFTISRDNSKNTLYLQMNSLRAEDTAVYYCAKVLETGLLVDAFDIWGQGT MVTVSS |
| Ab # 14 V_{L} amino acid sequence (SEQ ID NO: 18) |
| QYELTQPPSVSVYPGQTASITCSGDQLGSKFVSWYQQRPGQSPVLVMYKDKRRPSEIPE RFSGSNSGNTATLTISGTQAIDEADYYCQAWDSSTYVFGTGTKVTVL |
| Ab # 14 V_{H} CDR1 (SEQ ID NO:19) |
| AYGMG |
| Ab # 14 V_{H} CDR2 (SEQ ID NO:20) |
| YISPSGGHTKYADSVKG |
| Ab # 14 V_{H} CDR3 (SEQ ID NO:21) |
| VLETGLLVDAFDI |
| Ab # 14 V_{L} CDR1 (SEQ ID NO:22) |
| SGDQLGSKFVS |
| Ab # 14 V_{L} CDR2 (SEQ ID NO:23) |
| YKDKRRPS |
| Ab # 14 V_{L} CDR3 (SEQ ID NO:24) |
| QAWDSSTYV |
| |
| Ab # 17 V_{H} amino acid sequence (SEQ ID NO:25) |
| EVQLLESGGGLVQPGGSLRLSCAASGFTFSWYGMGWVRQAPGKGLEWVSYISPSGGIT VYADSVKGRFTISRDNSKNTLYLQMNSLRAEDTAVYYCARLNYYYGLDVWGQGTTVT VSS |
| Ab # 17 V_{L} amino acid sequence (SEQ ID NO:26) |
| QDIQMTQSPSSLSASVGDRITITCQASQDIGDSLNWYQQKPGKAPRLLIYDASNLETGVP PRFSGSGSGTDFTFTFRSLQPEDIATYFCQQSANAPFTFGPGTKVDIK |
| Ab # 17 V_{H} CDR1 (SEQ ID NO:27) |
| WYGMG |
| Ab # 17 V_{H} CDR2 (SEQ ID NO:28) |
| YISPSGGITVYADSVKG |
| Ab # 17 V_{H} CDR3 (SEQ ID NO:29) |
| LNYYYGLDV |
| Ab # 17 V_{L} CDR1 (SEQ ID NO:30) |
| QASQDIGDSLN |
| Ab # 17 V_{L} CDR2 (SEQ ID NO:31) |
| DASNLET |
| Ab # 17 V_{L} CDR3 (SEQ ID NO:32) |
| QQSANAPFT |
| |
| Ab # 19 V_{H} amino acid sequence (SEQ ID NO:33) |
| EVQLLESGGGLVQPGGSLRLSCAASGFTFSRYGMWWVRQAPGKGLEWVSYIGSSGGPT YYVDSVKGRFTISRDNSKNTLYLQMNSLRAEDTAVYYCAGGRGTPYYFDSWGQGTLV TVSS |
| Ab # 19 V_{L} amino acid sequence (SEQ ID NO:34) |
| QYELTQPASVSGSPGQSITISCTGTSSDIGRWNIVSWYQQHPGKAPKLMIYDVSNRPSGV SNRF |
| SGSKSGNTASLTISGLQAEDEADYYCSSYTSSSTWVFGGGTKLTVL |
| Ab # 19 V_{H} CDR1 (SEQ ID NO:35) |
| RYGMW |
| Ab # 19 V_{H} CDR2 (SEQ ID NO:36) |
| YIGSSGGPTYYVDSVKG |
| Ab # 19 V_{H} CDR3 (SEQ ID NO:37) |
| GRGTPYYFDS |
| Ab # 19 V_{L} CDR1 (SEQ ID NO:38 |
| TGTSSDIGRWNIVS |
| Ab # 19 V_{L} CDR2 (SEQ ID NO:39) |
| DVSNRPS |
| Ab # 19 V_{L} CDR3 (SEQ ID NO:40) |
| SSYTSSSTWV |
| ErbB3 (SEQ ID NO:41) |
| SEVGNSQAVCPGTLNGLSVTGDAENQYQTLYKLYERCEVVMGNLEIVLTGHNADLSFL QWIREVTGYVLVAMNEFSTLPLPNLRVVRGTQVYDGKFAIFVMLNYNTNSSHALRQLR LTQLTEILSGGVYIEKNDKLCHMDTIDWRDIVRDRDAEIVVKDNGRSCPPCHEVCKGRC WGPGSEDCQTLTKTICAPQCNGHCFGPNPNQCCHDECAGGCSGPQDTDCFACRHFNDS GACVPRCPQPLVYNKLTFQLEPNPHTKYQYGGVCVASCPHNFVVDQTSCVRACPPDKM EVDKNGLKMCEPCGGLCPKACEGTGSGSRFQTVDSSNIDGFVNCTKILGNLDFLITGLNG DPWHKIPALDPEKLNVFRTVREITGYLNIQSWPPHMHNFSVFSNLTTIGGRSLYNRGFSLL IMKNLNVTSLGFRSLKEISAGRIYISANRQLCYHHSLNWTKVLRGPTEERLDIKHNRPRRD CVAEGKVCDPLCSSGGCWGPGPGQCLSCRNYSRGGVCVTHCNFLNGEPREFAHEAECFS CHPECQPMEGTATCNGSGSDTCAQCAHFRDGPHCVSSCPHGVLGAKGPIYKYPDVQNEC RPCHENCTQGCKGPELQDCLGQTLVLIGKTHLTMALTVIAGLVVIFMMLGGTFLYWRGR RIQNKRAMRRYLERGESIEPLDPSEKANKVLARIFKETELRKLKVLGSGVFGTVHKGVWI PEGESIKIPVCIKVIEDKSGRQSFQAVTDHMLAIGSLDHAHIVRLLGLCPGSSLQLVTQYLP LGSLLDHVRQHRGALGPQLLLNWGVQIAKGMYYLEEHGMVHRNLAARNVLLKSPSQV QVADFGVADLLPPDDKQLLYSEAKTPIKWMALESIHFGKYTHQSDVWSYGVTVWELMT FGAEPYAGLRLAEVPDLLEKGERLAQPQICTIDVYMVMVKCWMIDENIRPTFKELANEFT RMARDPPRYLVIKRESGPGIAPGPEPHGLTNKKLEEVELEPELDLDLDLEAEEDNLATTTL GSALSLPVGTLNRPRGSQSLLSPSSGYMPMNQGNLGESCQESAVSGSSERCPRPVSLHPMP RGCLASESSEGHVTGSEAELQEKVSMCRSRSRSRSPRPRGDSAYHSQRHSLLTPVTPLSPPG LEEEDVNGYVMPDTHLKGTPSSREGTLSSVGLSSVLGTEEEDEDEEYEYMNRRRRHSPPHP PRPSSLEELGYEYMDVGSDLSASLGSTQSCPLHPVPIMPTAGTTPDEDYEYMNRQRDGGGP GGDYAAMGACPASEQGYEEMRAFQGPGHQAPHVHYARLKTLRSLEATDSAFDNPDYWH SRLFPKANAQRT |
| |
| MM-111 amino acid sequence (SEQ ID NO:44) |
| QVQLQESGGGLVKPGGSLRLSCAASGFTFSSYWMSWVRQAPGKGLEWVANINRDGSA SYYVDSVKGRFTISRDDAKNSLYLQMNSLRAEDTAVYYCARDRGVGYFDLWGRGTLV TVSSASTGGGGSGGGGSGGGGSQSALTQPASVSGSPGQSITISCTGTSSDVGGYNFVSW YQQHPGKAPKLMIYDVSDRPSGVSDRFSGSKSGNTASLIISGLQADDEADYYCSSYGSSS |
| THVIFGGGTKVTVLGAASDAHKSEVAHRFKDLGEENFKALVLIAFAQYLQQSPFEDHV KLVNEVTEFAKTCVADESAENCDKSLHTLFGDKLCTVATLRETYGEMADCCAKQEPER NECFLQHKDDNPNLPRLVRPEVDVMCTAFHDNEETFLKKYLYEIARRHPYFYAPELLFF AKRYKAAFTECCQAADKAACLLPKLDELRDEGKASSAKQRLKCASLQKFGERAFKAW AVARLSQRFPKAEFAEVSKLVTDLTKVHTECCHGDLLECADDRADLAKYICENQDSISS KLKECCEKPLLEKSHCIAEVENDEMPADLPSLAADFVESKDVCKNYAEAKDVFLGMFL YEYARRHPDYSVVLLLRLAKTYETTLEKCCAAADPHECYAKVFDEFKPLVEEPQNLIKQ NCELFEQLGEYKFQNALLVRYTKKVPQVSTPTLVEVSRNLGKVGSKCCKHPEAKRMPC AEDYLSVVLNQLCVLHEKTPVSDRVTKCCTESLVNRRPCFSALEVDETYVPKEFQAETF TFHADICTLSEKERQIKKQTALVELVKHKPKATKEQLKAVMDDFAAFVEKCCKADDKE TCFAEEGKKLVAASQAALGLAAALQVQLVQSGAEVKKPGESLKISCKGSGYSFTSYWI AWVRQMPGKGLEYMGLIYPGDSDTKYSPSFQGQVTISVDKSVSTAYLQWSSLKPSDSA VYFCARHDVGYCTDRTCAKWPEWLGVWGQGTLVTVSSGGGGSSGGGSGGGGSQSVL TQPPSVSAAPGQKVTISCSGSSSNIGNNYVSWYQQLPGTAPKLLIYDHTNRPAGVPDRFS GSKSGTSASLAISGFRSEDEADYYCASWDYTLSGWVFGGGTKLTVLG |

The invention described herein also relates to the following aspects:
1. A method for overcoming resistance of a tumor in a subject to an ErbB pathway inhibitor, the method comprising:
   selecting a subject with a tumor exhibiting resistance to an ErbB pathway inhibitor; and
   administering to the subject (i) an ErbB3 inhibitor and (ii) the ErbB pathway inhibitor.
2. The method of aspect 1, wherein the resistance to the ErbB pathway inhibitor is acquired resistance.
3. The method of aspect 2, wherein the acquired resistance is acquired resistance to an EGFR inhibitor.
4. The method of aspect 3, wherein the acquired resistance is associated with a T790M EGFR mutation in tumor cells.
5. The method of aspect 3, wherein the acquired resistance is associated with a KRAS mutation in tumor cells.
6. The method of aspect 5, wherein the KRAS mutation is a G12S, G12C, or G12V KRAS mutation.
7. The method of aspect 5, wherein the KRAS mutation is a Q61R KRAS mutation.
8. The method of aspect 3, wherein the acquired resistance is resistance to gefitinib, trastuzumab, or lapatinib.
9. The method of aspect 2, wherein the acquired resistance is acquired resistance to a HER2 inhibitor.
10. The method of aspect 1, wherein the resistance is associated with reactivation of PI3K/AKT signaling in tumor cells in the subject.
11. The method of aspect 1, wherein the ErbB3 inhibitor administered to the subject is an anti-ErbB3 antibody.
12. The method of aspect 1, wherein the ErbB3 inhibitor administered to the subject is a bispecific anti-ErbB3, anti-ErbB2 antibody.
13. The method of aspect 11, wherein the anti-ErbB3 antibody comprises VH and VL sequences as shown in SEQ ID NOs: 1 and 2, respectively.
14. The method of aspect 12, wherein the anti-ErbB3 antibody comprises an antibody comprising VH CDR1, 2 and 3 sequences as shown in SEQ ID NOs: 3-5, respectively, and VL CDR1, 2 and 3 sequences as shown in SEQ ID NOs: 6-8, respectively.
15. The method of aspect 11, wherein the anti-ErbB3 antibody comprises VH and VL sequences as shown in SEQ ID NOs: 9 and 10, respectively.
16. The method of aspect 11, wherein the anti-ErbB3 antibody comprises VH and VL sequences as shown in SEQ ID NOs: 17 and 18, respectively.
17. The method of aspect 11, wherein the anti-ErbB3 antibody comprises VH and VL sequences as shown in SEQ ID NOs: 25 and 26, respectively.
18. The method of aspect 11, wherein the anti-ErbB3 antibody comprises VH and VL sequences as shown in SEQ ID NOs: 33 and 34, respectively.
19. The method of aspect 11, wherein the anti-ErbB3 antibody is selected from the group consisting of AV-203, mAb 1B4C3, mAb 2D1D12, AMG-888, mAb 205.10.1, mAb 205.10.2, mAb 205.10.3, and humanized mAb 8B8.
20. The method of aspect 1, wherein the ErbB3 inhibitor inhibits PI3K/AKT signaling.
21. The method of aspect 1, wherein the ErbB pathway inhibitor is an EGFR inhibitor.
22. The method of aspect 21, wherein the EGFR inhibitor is an anti-EGFR antibody.
23. The method of aspect 22, wherein the anti-EGFR antibody comprises cetuximab.
24. The method of aspect 22, wherein the anti-EGFR antibody is selected from the group consisting of MM-151, Sym004, matuzumab, panitumumab, nimotuzumab and mAb 806.
25. The method of aspect 21, wherein the EGFR inhibitor administered to the subject is a small molecule inhibitor of EGFR signaling.
26. The method of aspect 25, wherein the small molecule inhibitor of EGFR signaling is gefitinib.
27. The method of aspect 25, wherein the small molecule inhibitor of EGFR signaling is selected from the group consisting of lapatinib, canertinib, CI-1033 (PD 183805), erlotinib, PKI-166, PD-158780, pelitinib, EKB-569, and tyrphostin AG 1478.
28. The method of aspect 1, wherein the ErbB pathway inhibitor administered to the subject is a HER2 inhibitor.
29. The method of aspect 28, wherein the HER2 inhibitor an anti-HER2 antibody.
30. The method of aspect 29, wherein the anti-HER2 antibody is trastuzumab or pertuzumab.
31. The method of any one of aspects 1-30, wherein the tumor is a lung cancer.
32. The method of aspect 31, wherein the lung cancer is a non-small cell lung cancer (NSCLC).
33. The method of aspect 32, wherein the NSCLC is an adenocarcinoma.
34. The method of aspect 32, wherein the NSCLC is a squamous cell carcinoma.
35. The method of aspect 32, wherein the NSCLC is a large cell carcinoma.
36. The method of any one of aspects 1-30, wherein the subject has a cancer selected from the group consisting of colorectal cancer, head and neck cancer, pancreatic cancer and breast cancer.
37. A method of inhibiting growth, invasiveness or metastasis of a tumor, wherein the tumor comprises one or more of a T790M EGFR mutation, a G12S, G12C, or G12V KRAS mutation, and a Q61R KRAS mutation, the method comprising contacting the tumor with (i) an EGFR inhibitor; and (ii) an ErbB3 inhibitor.
38. The method of aspect 37, wherein the ErbB3 inhibitor comprises an anti-ErbB3 antibody.
39. The method of aspect 37, wherein the ErbB3 inhibitor comprises a bispecific anti-ErbB3, anti-ErbB2 antibody.
40. The method of aspect 38, wherein the anti-ErbB3 antibody comprises the VH and VL sequences set forth in SEQ ID NOs: 1 and 2, respectively.
41. The method of aspect 39, wherein the bispecific anti-ErbB3, anti-ErbB2 antibody comprises the amino acid sequence set forth in SEQ ID NO:44.
42. The method of aspect 38, wherein the anti-ErbB3 antibody is selected from the group consisting of mAb 1B4C3, mAb 2D1D12, AMG-888, Av-203, mAb 205.10.1, mAb 205.10.2, mAb 205.10.3, and humanized mAb 8B8.
43. The method of aspect 39, wherein the bispecific anti-ErbB3, anti-ErbB2 antibody is selected from the group comprising ALM, A5-HSA-ML3.9, A5-HSA-B1D2, B12-HSA-B1D2, A5-HSA-F5B6H2, H3-HSA-F5B6H2, and F4-HSA-F5B6H2.
44. The method of aspect 37, wherein the ErbB3 inhibitor inhibits PI3K/AKT signaling in the tumor.
45. The method of aspect 37, wherein the tumor is contacted with an EGFR inhibitor comprising an anti-EGFR antibody.
46. The method of aspect 45, wherein the anti-EGFR antibody comprises cetuximab.
47. The method of aspect 45, wherein the anti-EGFR antibody is selected from the group consisting of MM-151, Sym004, matuzumab, panitumumab, nimotuzumab and mAb 806.
48. The method of aspect 37, wherein the tumor is contacted with an EGFR inhibitor comprising a small molecule inhibitor of EGFR signaling.
49. The method of aspect 48, wherein the small molecule inhibitor of EGFR signaling is gefitinib.
50. The method of aspect 48, wherein the small molecule inhibitor of EGFR signaling is selected from the group consisting of afatinib, lapatinib, canertinib, erlotinib HCL, pelitinib, PKI-166, PD158780, and tyrphostin AG 1478.
51. The method of any one of aspects 37-50, wherein the tumor is a lung cancer tumor.
52. The method of aspect 51, wherein the lung cancer is non-small cell lung cancer (NSCLC).
53. The method of aspect 51, wherein the NSCLC is an adenocarcinoma.
54. The method of aspect 51, wherein the NSCLC is a squamous cell carcinoma.
55. The method of aspect 51, wherein the NSCLC is a large cell carcinoma.
56. The method of one of aspects 37-50, wherein the tumor is of a cancer selected from the group consisting of colorectal cancer, head and neck cancer, breast cancer, and pancreatic cancer.
57. A method of treating a tumor in a subject, the method comprising
   selecting a subject with a tumor comprising one or more of a T790M EGFR mutation, a G12S, G12C, or G12V KRAS mutation, and a Q61R KRAS mutation, and
   administering to the subject (i) an EGFR inhibitor; and (ii) an ErbB3 inhibitor.
58. The method of aspect 57, wherein the ErbB3 inhibitor administered to the subject is an anti-ErbB3 antibody.
59. The method of aspect 58, wherein the anti-ErbB3 antibody comprises the VH and VL sequences set forth in SEQ ID NOs: 1 and 2, respectively.
60. The method of aspect 58, wherein the anti-ErbB3 antibody comprises an antibody comprising VH CDR1, 2 and 3 sequences as shown in SEQ ID NOs: 3-5, respectively, and VL CDR1, 2 and 3 sequences as shown in SEQ ID NOs: 6-8, respectively.
61. The method of aspect 58, wherein the anti-ErbB3 antibody comprises VH and VL sequences as shown in SEQ ID NOs: 9 and 10, respectively.
62. The method of aspect 58, wherein the anti-ErbB3 antibody comprises VH and VL sequences as shown in SEQ ID NOs: 17 and 18, respectively.
63. The method of aspect 58, wherein the anti-ErbB3 antibody comprises VH and VL sequences as shown in SEQ ID NOs: 25 and 26, respectively.
64. The method of aspect 58, wherein the anti-ErbB3 antibody comprises VH and VL sequences as shown in SEQ ID NOs: 33 and 34, respectively.
65. The method of aspect 58, wherein the anti-ErbB3 antibody is selected from the group consisting of mAb 1B4C3, mAb 2D1D12, AMG-888, AV-203, mAb 205.10.1, mAb 205.10.2, mAb 205.10.3, and humanized mAb 8B8.
66. The method of aspect 57, wherein the ErbB3 inhibitor inhibits PI3K/AKT signaling in cells of the tumor.
67. The method of aspect 57, wherein the EGFR inhibitor administered to the subject is an anti-EGFR antibody.
68. The method of aspect 67, wherein the anti-EGFR antibody comprises cetuximab.
69. The method of aspect 67, wherein the anti-EGFR antibody is selected from the group consisting of MM-151, Sym004, matuzumab, panitumumab, nimotuzumab and mAb 806.
70. The method of aspect 57, wherein the EGFR inhibitor administered to the subject is a small molecule inhibitor of EGFR signaling.
71. The method of aspect 70, wherein the small molecule inhibitor of EGFR signaling is gefitinib.
72. The method of aspect 70, wherein the small molecule inhibitor of EGFR signaling is selected from the group consisting of afatinib, lapatinib, canertinib, erlotinib HCL, pelitinib, PKI-166, PD158780, and AG 1478.
73. The method of any one of aspects 57-72, wherein the tumor is a lung cancer.
74. The method of aspect73, wherein the lung cancer is a non-small cell lung cancer (NSCLC).
75. The method of aspect 74, wherein the NSCLC is an adenocarcinoma.
76. The method of aspect 74, wherein the NSCLC is a squamous cell carcinoma.
77. The method of aspect 74, wherein the NSCLC is a large cell carcinoma.
78. The method of any one of aspects 57-72, wherein the subject has a cancer selected from the group consisting of colorectal cancer, head and neck cancer, breast cancer, and pancreatic cancer.
79. Pharmaceutical compositions for overcoming resistance of a tumor to an ErbB pathway inhibitor, said compositions comprising an ErbB3 inhibitor, an ErbB pathway inhibitor and at least one pharmaceutical carrier.
80. The pharmaceutical compositions of aspect 79, wherein the ErbB3 inhibitor and the ErbB pathway inhibitor are formulated with the at least one pharmaceutical carrier into a single composition.
81. The pharmaceutical compositions of aspect 79, wherein the ErbB3 inhibitor is formulated with a pharmaceutical carrier to form a first composition, the ErbB pathway inhibitor is formulated with a pharmaceutical carrier to form a second composition, and the first and second composition are optionally packaged together.
82. The pharmaceutical compositions of any one of aspects 79-81, wherein the ErbB3 inhibitor is an anti-ErbB3 antibody.
83. The pharmaceutical compositions of aspect 82, wherein the ErbB3 inhibitor is an anti-ErbB3 antibody.
84. The pharmaceutical compositions of aspect 83, wherein the anti-ErbB3 antibody comprises the VH and VL sequences set forth in SEQ ID NOs: 1 and 2, respectively.
85. The pharmaceutical compositions of any one of aspects 79-81, wherein the ErbB3 inhibitor is a bispecific anti-ErbB3, anti-ErbB2 antibody.
86. The pharmaceutical compositions of 85, wherein the bispecific anti-ErbB3, anti-ErbB2 antibody comprises the amino acid sequence set forth in SEQ ID NO:44.
87. The pharmaceutical compositions of aspect 85, wherein the bispecific anti-ErbB3, anti-ErbB2 antibody exhibits an insignificant effect upon HER2 mediated signaling.
88. The pharmaceutical compositions of any one of aspects 79-81, wherein the ErbB3 inhibitor inhibits PI3K/AKT signaling.
89. The pharmaceutical compositions of any one of aspects 79-81, wherein the ErbB pathway inhibitor included in the composition is an anti-EGFR antibody.
90. The pharmaceutical compositions of aspect 89, wherein the anti-EGFR antibody comprises cetuximab.
91. The pharmaceutical compositions of any one of aspects 79-81, wherein the ErbB pathway inhibitor included in the composition is a small molecule inhibitor of EGFR signaling.
92. The pharmaceutical compositions of aspect 91, wherein the small molecule inhibitor of EGFR signaling is gefitinib.
93. The pharmaceutical compositions of any one of aspects 79-81, wherein the ErbB pathway inhibitor included in the composition is an anti-HER2 antibody.
94. The pharmaceutical compositions of aspect 93, wherein the anti-HER2 antibody comprises trastuzumab.
95. The pharmaceutical compositions of any one of aspects 79-81, wherein the tumor is a lung cancer.
96. The pharmaceutical compositions of aspect 95, wherein the lung cancer is a non-small cell lung cancer selected from an adenocarcinoma, a squamous cell carcinoma and a large cell carcinoma.
97. The pharmaceutical compositions of aspect 95, wherein the tumor comprises tumor cells comprising one or more of a T790M EGFR mutation, a G12S, G12C, or G12V KRAS mutation, and a Q61R KRAS mutation.
98. The pharmaceutical compositions of aspect 95, wherein the tumor is a non-small cell lung cancer selected from an adenocarcinoma, a squamous cell carcinoma and a large cell carcinoma and the tumor further comprises tumor cells comprising one or more of a T790M EGFR mutation, a G12S, G12C, or G12V KRAS mutation, and a Q61R KRAS mutation.

## Claims

1. A bispecific anti-ErbB3, anti-ErbB2 antibody for use in a method of overcoming resistance to trastuzumab in a patient which has a tumour that has acquired resistance to trastuzumab, said method comprising administering to the patient an effective amount of said bispecific antibody and trastuzumab.

2. A bispecific anti-ErbB3, anti-ErbB2 antibody for use in a method of overcoming resistance to an anti-EGFR antibody in a patient which has a tumour that has acquired resistance to said anti-EGFR antibody, said method comprising administering to the patient an effective amount of said bispecific antibody and said anti-EGFR antibody.

3. The bispecific antibody of claim 1 or 2 for use in the method of claim 1 or 2 wherein the bispecific antibody is MM-111, which comprises the amino acid sequence of SEQ ID NO:44.

4. The bispecific antibody of claim 2 or 3 for use in the method of claim 2 wherein the anti-EGFR antibody is selected from the group consisting of cetuximab, MM-151, Sym004, matuzumab, panitumumab, nimotuzumab and mAb 806.

5. The bispecific antibody of any one of the preceding claims for use in the method of any one of claims 1 or 2, wherein the method further comprises administering to the patient an effective amount of at least one chemotherapeutic agent.

6. The bispecific antibody of any one of the preceding claims for use in the method of claim 1, wherein the method further comprises administering to the patient an effective amount of paclitaxel.

7. The bispecific antibody of any one of claims 2 to 4 for use in the method of claim 2 wherein the method further comprises administering to the patient an effective amount of a small molecule EGFR inhibitor selected from afatinib, lapatinib, gefitinib, canertinib, CI-1033 (PD 183805), erlotinib, PKI-166, PD-158780, pelitinib, EKB-569, or tyrphostin AG 1478.

8. The bispecific antibody of any one of claims 2 to 4 for use in the method of claim 6 wherein the small molecule EGFR inhibitor is erlotinib, gefitinib, or lapatinib.

9. The bispecific antibody of any one of claims 2 to 4 for use in the method of claim 6, wherein the small molecule EGFR inhibitor is lapatinib and wherein the method further comprises administering to the patient an effective amount of tamoxifen.


SEQUENCE LISTING

<110> MERRIMACK PHARMACEUTICALS, INC.

<120> OVERCOMING RESISTANCE TO ERBB PATHWAY INHIBITORS

<130> N400857EP

<140> 12714108.3
<141> 2012-03-15

<150> 61/452,974
<151> 2011-03-15

<150> 61/452,976
<151> 2011-03-15

<160> 46

<170> PatentIn version 3.5

<210> 1
<211> 119
<212> PRT
<213> Artificial Sequence

<220>
<221> source
<223> /note="Description of Artificial Sequence: Synthetic
      polypeptide"

<400> 1

<210> 2
<211> 111
<212> PRT
<213> Artificial Sequence

<220>
<221> source
<223> /note="Description of Artificial Sequence: Synthetic
      polypeptide"

<400> 2


<210> 3
<211> 5
<212> PRT
<213> Artificial Sequence

<220>
<221> source
<223> /note="Description of Artificial Sequence: Synthetic
      peptide"

<400> 3
His Tyr Val Met Ala
1               5


<210> 4
<211> 17
<212> PRT
<213> Artificial Sequence

<220>
<221> source
<223> /note="Description of Artificial Sequence: Synthetic

peptide"

<400> 4
Ser Ile Ser Ser Ser Gly Gly Trp Thr Leu Tyr Ala Asp Ser Val Lys
1               5                   10                  15

Gly

<210> 5
<211> 10
<212> PRT
<213> Artificial Sequence

<220>
<221> source
<223> /note="Description of Artificial Sequence: Synthetic
      peptide"

<400> 5
Gly Leu Lys Met Ala Thr Ile Phe Asp Tyr
1               5                   10

<210> 6
<211> 14
<212> PRT
<213> Artificial Sequence

<220>
<221> source
<223> /note="Description of Artificial Sequence: Synthetic
      peptide"

<400> 6
Thr Gly Thr Ser Ser Asp Val Gly Ser Tyr Asn Val Val Ser
1               5                   10

<210> 7
<211> 7
<212> PRT
<213> Artificial Sequence

<220>
<221> source
<223> /note="Description of Artificial Sequence: Synthetic
      peptide"

<400> 7
Glu Val Ser Gln Arg Pro Ser
1               5

<210> 8
<211> 11
<212> PRT
<213> Artificial Sequence

<220>
<221> source

<223> /note="Description of Artificial Sequence: Synthetic
      peptide"

<400> 8
Cys Ser Tyr Ala Gly Ser Ser Ile Phe Val Ile
1               5                   10

<210> 9
<211> 118
<212> PRT
<213> Artificial Sequence

<220>
<221> source
<223> /note="Description of Artificial Sequence: Synthetic
      polypeptide"

<400> 9
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Asn Met Arg Trp Val Arg Gln Ala Pro Gly Lys Gly Leu Glu Trp Val
        35                  40                  45

Ser Val Ile Tyr Pro Ser Gly Gly Ala Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Tyr Tyr Tyr Tyr Gly Met Asp Val Trp Gly Gln Gly Thr
            100                 105                 110

Leu Val Thr Val Ser Ser
        115

<210> 10
<211> 110
<212> PRT
<213> Artificial Sequence

<220>
<221> source
<223> /note="Description of Artificial Sequence: Synthetic
      polypeptide"

<400> 10
Gln Ser Val Leu Thr Gln Pro Pro Ser Ala Ser Gly Thr Pro Gly Gln

1               5                   10                  15

Arg Val Thr Ile Ser Cys Ser Gly Ser Asp Ser Asn Ile Gly Arg Asn
            20                  25                  30

Tyr Ile Tyr Trp Tyr Gln Gln Phe Pro Gly Thr Ala Pro Lys Leu Leu
        35                  40                  45

Ile Tyr Arg Asn Asn Gln Arg Pro Ser Gly Val Pro Asp Arg Ile Ser
    50                  55                  60

Gly Ser Lys Ser Gly Thr Ser Ala Ser Leu Ala Ile Ser Gly Leu Arg
65                  70                  75                  80

Ser Glu Asp Glu Ala Glu Tyr His Cys Gly Thr Trp Asp Asp Ser Leu
                85                  90                  95

Ser Gly Pro Val Phe Gly Gly Gly Thr Lys Leu Thr Val Leu
            100                 105                 110

<210> 11
<211> 5
<212> PRT
<213> Artificial Sequence

<220>
<221> source
<223> /note="Description of Artificial Sequence: Synthetic
      peptide"

<400> 11
Ala Tyr Asn Met Arg
1               5

<210> 12
<211> 17
<212> PRT
<213> Artificial Sequence

<220>
<221> source
<223> /note="Description of Artificial Sequence: Synthetic
      peptide"

<400> 12
Val Ile Tyr Pro Ser Gly Gly Ala Thr Arg Tyr Ala Asp Ser Val Lys
1               5                   10                  15

Gly

<210> 13
<211> 9

<212> PRT
<213> Artificial Sequence

<220>
<221> source
<223> /note="Description of Artificial Sequence: Synthetic
      peptide"

<400> 13
Gly Tyr Tyr Tyr Tyr Gly Met Asp Val
1               5


<210> 14
<211> 13
<212> PRT
<213> Artificial Sequence

<220>
<221> source
<223> /note="Description of Artificial Sequence: Synthetic
      peptide"

<400> 14
Ser Gly Ser Asp Ser Asn Ile Gly Arg Asn Tyr Ile Tyr
1               5                   10


<210> 15
<211> 7
<212> PRT
<213> Artificial Sequence

<220>
<221> source
<223> /note="Description of Artificial Sequence: Synthetic
      peptide"

<400> 15
Arg Asn Asn Gln Arg Pro Ser
1               5


<210> 16
<211> 11
<212> PRT
<213> Artificial Sequence

<220>
<221> source
<223> /note="Description of Artificial Sequence: Synthetic
      peptide"

<400> 16
Gly Thr Trp Asp Asp Ser Leu Ser Gly Pro Val
1               5                   10


<210> 17
<211> 122
<212> PRT
<213> Artificial Sequence

<220>
<221> source
<223> /note="Description of Artificial Sequence: Synthetic
      polypeptide"

<400> 17
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Gly Met Gly Trp Val Arg Gln Ala Pro Gly Lys Gly Leu Glu Trp Val
        35                  40                  45

Ser Tyr Ile Ser Pro Ser Gly Gly His Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Leu Glu Thr Gly Leu Leu Val Asp Ala Phe Asp Ile Trp
            100                 105                 110

Gly Gln Gly Thr Met Val Thr Val Ser Ser
        115                 120


<210> 18
<211> 106
<212> PRT
<213> Artificial Sequence

<220>
<221> source
<223> /note="Description of Artificial Sequence: Synthetic
      polypeptide"

<400> 18
Gln Tyr Glu Leu Thr Gln Pro Pro Ser Val Ser Val Tyr Pro Gly Gln
1               5                   10                  15

Thr Ala Ser Ile Thr Cys Ser Gly Asp Gln Leu Gly Ser Lys Phe Val
            20                  25                  30

Ser Trp Tyr Gln Gln Arg Pro Gly Gln Ser Pro Val Leu Val Met Tyr
        35                  40                  45

Lys Asp Lys Arg Arg Pro Ser Glu Ile Pro Glu Arg Phe Ser Gly Ser
    50                  55                  60

Asn Ser Gly Asn Thr Ala Thr Leu Thr Ile Ser Gly Thr Gln Ala Ile
65                  70                  75                  80

Asp Glu Ala Asp Tyr Tyr Cys Gln Ala Trp Asp Ser Ser Thr Tyr Val
                85                  90                  95

Phe Gly Thr Gly Thr Lys Val Thr Val Leu
            100                 105

<210> 19
<211> 5
<212> PRT
<213> Artificial Sequence

<220>
<221> source
<223> /note="Description of Artificial Sequence: Synthetic
      peptide"

<400> 19
Ala Tyr Gly Met Gly
1               5

<210> 20
<211> 17
<212> PRT
<213> Artificial Sequence

<220>
<221> source
<223> /note="Description of Artificial Sequence: Synthetic
      peptide"

<400> 20
Tyr Ile Ser Pro Ser Gly Gly His Thr Lys Tyr Ala Asp Ser Val Lys
1               5                   10                  15

Gly

<210> 21
<211> 13
<212> PRT
<213> Artificial Sequence

<220>
<221> source
<223> /note="Description of Artificial Sequence: Synthetic
      peptide"

<400> 21
Val Leu Glu Thr Gly Leu Leu Val Asp Ala Phe Asp Ile
1               5                   10

<210> 22
<211> 11
<212> PRT
<213> Artificial Sequence

<220>
<221> source
<223> /note="Description of Artificial Sequence: Synthetic
      peptide"

<400> 22
Ser Gly Asp Gln Leu Gly Ser Lys Phe Val Ser
1               5                   10


<210> 23
<211> 8
<212> PRT
<213> Artificial Sequence

<220>
<221> source
<223> /note="Description of Artificial Sequence: Synthetic
      peptide"

<400> 23
Tyr Lys Asp Lys Arg Arg Pro Ser
1               5


<210> 24
<211> 9
<212> PRT
<213> Artificial Sequence

<220>
<221> source
<223> /note="Description of Artificial Sequence: Synthetic
      peptide"

<400> 24
Gln Ala Trp Asp Ser Ser Thr Tyr Val
1               5


<210> 25
<211> 118
<212> PRT
<213> Artificial Sequence

<220>
<221> source
<223> /note="Description of Artificial Sequence: Synthetic
      polypeptide"

<400> 25
Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Trp Tyr
            20                  25                  30

Gly Met Gly Trp Val Arg Gln Ala Pro Gly Lys Gly Leu Glu Trp Val
        35                  40                  45

Ser Tyr Ile Ser Pro Ser Gly Gly Ile Thr Val Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Asn Tyr Tyr Tyr Gly Leu Asp Val Trp Gly Gln Gly Thr
            100                 105                 110

Thr Val Thr Val Ser Ser
        115

<210> 26
<211> 108
<212> PRT
<213> Artificial Sequence

<220>
<221> source
<223> /note="Description of Artificial Sequence: Synthetic
      polypeptide"

<400> 26
Gln Asp Ile Gln Met Thr Gln Ser Pro Ser Ser Leu Ser Ala Ser Val
1               5                   10                  15

Gly Asp Arg Ile Thr Ile Thr Cys Gln Ala Ser Gln Asp Ile Gly Asp
            20                  25                  30

Ser Leu Asn Trp Tyr Gln Gln Lys Pro Gly Lys Ala Pro Arg Leu Leu
        35                  40                  45

Ile Tyr Asp Ala Ser Asn Leu Glu Thr Gly Val Pro Pro Arg Phe Ser
    50                  55                  60

Gly Ser Gly Ser Gly Thr Asp Phe Thr Phe Thr Phe Arg Ser Leu Gln
65                  70                  75                  80

Pro Glu Asp Ile Ala Thr Tyr Phe Cys Gln Gln Ser Ala Asn Ala Pro
                85                  90                  95

Phe Thr Phe Gly Pro Gly Thr Lys Val Asp Ile Lys
            100                 105

<210> 27
<211> 5
<212> PRT
<213> Artificial Sequence

<220>
<221> source
<223> /note="Description of Artificial Sequence: Synthetic
      peptide"

<400> 27
Trp Tyr Gly Met Gly
1               5


<210> 28
<211> 17
<212> PRT
<213> Artificial Sequence

<220>
<221> source
<223> /note="Description of Artificial Sequence: Synthetic
      peptide"

<400> 28
Tyr Ile Ser Pro Ser Gly Gly Ile Thr Val Tyr Ala Asp Ser Val Lys
1               5                   10                  15


Gly



<210> 29
<211> 9
<212> PRT
<213> Artificial Sequence

<220>
<221> source
<223> /note="Description of Artificial Sequence: Synthetic
      peptide"

<400> 29
Leu Asn Tyr Tyr Tyr Gly Leu Asp Val
1               5


<210> 30
<211> 11
<212> PRT
<213> Artificial Sequence

<220>
<221> source
<223> /note="Description of Artificial Sequence: Synthetic
      peptide"

<400> 30
Gln Ala Ser Gln Asp Ile Gly Asp Ser Leu Asn
1               5                   10

<210> 31
<211> 7
<212> PRT
<213> Artificial Sequence

<220>
<221> source
<223> /note="Description of Artificial Sequence: Synthetic
      peptide"

<400> 31
Asp Ala Ser Asn Leu Glu Thr
1               5


<210> 32
<211> 9
<212> PRT
<213> Artificial Sequence

<220>
<221> source
<223> /note="Description of Artificial Sequence: Synthetic
      peptide"

<400> 32
Gln Gln Ser Ala Asn Ala Pro Phe Thr
1               5


<210> 33
<211> 119
<212> PRT
<213> Artificial Sequence

<220>
<221> source
<223> /note="Description of Artificial Sequence: Synthetic
      polypeptide"

<400> 33
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Gly Met Trp Trp Val Arg Gln Ala Pro Gly Lys Gly Leu Glu Trp Val
        35                  40                  45

Ser Tyr Ile Gly Ser Ser Gly Gly Pro Thr Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Gly Arg Gly Thr Pro Tyr Tyr Phe Asp Ser Trp Gly Gln Gly
            100                 105                 110

Thr Leu Val Thr Val Ser Ser
        115

<210> 34
<211> 110
<212> PRT
<213> Artificial Sequence

<220>
<221> source
<223> /note="Description of Artificial Sequence: Synthetic
      polypeptide"

<400> 34
Gln Tyr Glu Leu Thr Gln Pro Ala Ser Val Ser Gly Ser Pro Gly Gln
1               5                   10                  15

Ser Ile Thr Ile Ser Cys Thr Gly Thr Ser Ser Asp Ile Gly Arg Trp
            20                  25                  30

Asn Ile Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Trp Val Phe Gly Gly Gly Thr Lys Leu Thr Val Leu
            100                 105                 110

<210> 35
<211> 5
<212> PRT
<213> Artificial Sequence

<220>
<221> source
<223> /note="Description of Artificial Sequence: Synthetic
      peptide"

<400> 35
Arg Tyr Gly Met Trp

1               5


<210> 36
<211> 17
<212> PRT
<213> Artificial Sequence

<220>
<221> source
<223> /note="Description of Artificial Sequence: Synthetic
      peptide"

<400> 36
Tyr Ile Gly Ser Ser Gly Gly Pro Thr Tyr Tyr Val Asp Ser Val Lys
1               5                   10                  15


Gly




<210> 37
<211> 10
<212> PRT
<213> Artificial Sequence

<220>
<221> source
<223> /note="Description of Artificial Sequence: Synthetic
      peptide"

<400> 37
Gly Arg Gly Thr Pro Tyr Tyr Phe Asp Ser
1               5                   10


<210> 38
<211> 14
<212> PRT
<213> Artificial Sequence

<220>
<221> source
<223> /note="Description of Artificial Sequence: Synthetic
      peptide"

<400> 38
Thr Gly Thr Ser Ser Asp Ile Gly Arg Trp Asn Ile Val Ser
1               5                   10


<210> 39
<211> 7
<212> PRT
<213> Artificial Sequence

<220>
<221> source
<223> /note="Description of Artificial Sequence: Synthetic
      peptide"

<400> 39

Asp Val Ser Asn Arg Pro Ser
1               5

<210> 40
<211> 10
<212> PRT
<213> Artificial Sequence

<220>
<221> source
<223> /note="Description of Artificial Sequence: Synthetic
      peptide"

<400> 40
Ser Ser Tyr Thr Ser Ser Ser Thr Trp Val
1               5                   10

<210> 41
<211> 1323
<212> PRT
<213> Artificial Sequence

<220>
<221> source
<223> /note="Description of Artificial Sequence: Synthetic
      polypeptide"

<400> 41
Ser Glu Val Gly Asn Ser Gln Ala Val Cys Pro Gly Thr Leu Asn Gly
1               5                   10                  15

Leu Ser Val Thr Gly Asp Ala Glu Asn Gln Tyr Gln Thr Leu Tyr Lys
            20                  25                  30

Leu Tyr Glu Arg Cys Glu Val Val Met Gly Asn Leu Glu Ile Val Leu
        35                  40                  45

Thr Gly His Asn Ala Asp Leu Ser Phe Leu Gln Trp Ile Arg Glu Val
    50                  55                  60

Thr Gly Tyr Val Leu Val Ala Met Asn Glu Phe Ser Thr Leu Pro Leu
65                  70                  75                  80

Pro Asn Leu Arg Val Val Arg Gly Thr Gln Val Tyr Asp Gly Lys Phe
                85                  90                  95

Ala Ile Phe Val Met Leu Asn Tyr Asn Thr Asn Ser Ser His Ala Leu
            100                 105                 110

Arg Gln Leu Arg Leu Thr Gln Leu Thr Glu Ile Leu Ser Gly Gly Val
        115                 120                 125

Tyr Ile Glu Lys Asn Asp Lys Leu Cys His Met Asp Thr Ile Asp Trp

130                 135                 140

Arg Asp Ile Val Arg Asp Arg Asp Ala Glu Ile Val Val Lys Asp Asn
145                 150                 155                 160

Gly Arg Ser Cys Pro Pro Cys His Glu Val Cys Lys Gly Arg Cys Trp
                165                 170                 175

Gly Pro Gly Ser Glu Asp Cys Gln Thr Leu Thr Lys Thr Ile Cys Ala
            180                 185                 190

Pro Gln Cys Asn Gly His Cys Phe Gly Pro Asn Pro Asn Gln Cys Cys
        195                 200                 205

His Asp Glu Cys Ala Gly Gly Cys Ser Gly Pro Gln Asp Thr Asp Cys
    210                 215                 220

Phe Ala Cys Arg His Phe Asn Asp Ser Gly Ala Cys Val Pro Arg Cys
225                 230                 235                 240

Pro Gln Pro Leu Val Tyr Asn Lys Leu Thr Phe Gln Leu Glu Pro Asn
                245                 250                 255

Pro His Thr Lys Tyr Gln Tyr Gly Gly Val Cys Val Ala Ser Cys Pro
            260                 265                 270

His Asn Phe Val Val Asp Gln Thr Ser Cys Val Arg Ala Cys Pro Pro
        275                 280                 285

Asp Lys Met Glu Val Asp Lys Asn Gly Leu Lys Met Cys Glu Pro Cys
    290                 295                 300

Gly Gly Leu Cys Pro Lys Ala Cys Glu Gly Thr Gly Ser Gly Ser Arg
305                 310                 315                 320

Phe Gln Thr Val Asp Ser Ser Asn Ile Asp Gly Phe Val Asn Cys Thr
                325                 330                 335

Lys Ile Leu Gly Asn Leu Asp Phe Leu Ile Thr Gly Leu Asn Gly Asp
            340                 345                 350

Pro Trp His Lys Ile Pro Ala Leu Asp Pro Glu Lys Leu Asn Val Phe
        355                 360                 365

Arg Thr Val Arg Glu Ile Thr Gly Tyr Leu Asn Ile Gln Ser Trp Pro
    370                 375                 380

Pro His Met His Asn Phe Ser Val Phe Ser Asn Leu Thr Thr Ile Gly
385                 390                 395                 400

Gly Arg Ser Leu Tyr Asn Arg Gly Phe Ser Leu Leu Ile Met Lys Asn
                405                 410                 415

Leu Asn Val Thr Ser Leu Gly Phe Arg Ser Leu Lys Glu Ile Ser Ala
            420                 425                 430

Gly Arg Ile Tyr Ile Ser Ala Asn Arg Gln Leu Cys Tyr His His Ser
        435                 440                 445

Leu Asn Trp Thr Lys Val Leu Arg Gly Pro Thr Glu Glu Arg Leu Asp
    450                 455                 460

Ile Lys His Asn Arg Pro Arg Arg Asp Cys Val Ala Glu Gly Lys Val
465                 470                 475                 480

Cys Asp Pro Leu Cys Ser Ser Gly Gly Cys Trp Gly Pro Gly Pro Gly
                485                 490                 495

Gln Cys Leu Ser Cys Arg Asn Tyr Ser Arg Gly Gly Val Cys Val Thr
            500                 505                 510

His Cys Asn Phe Leu Asn Gly Glu Pro Arg Glu Phe Ala His Glu Ala
        515                 520                 525

Glu Cys Phe Ser Cys His Pro Glu Cys Gln Pro Met Glu Gly Thr Ala
    530                 535                 540

Thr Cys Asn Gly Ser Gly Ser Asp Thr Cys Ala Gln Cys Ala His Phe
545                 550                 555                 560

Arg Asp Gly Pro His Cys Val Ser Ser Cys Pro His Gly Val Leu Gly
                565                 570                 575

Ala Lys Gly Pro Ile Tyr Lys Tyr Pro Asp Val Gln Asn Glu Cys Arg
            580                 585                 590

Pro Cys His Glu Asn Cys Thr Gln Gly Cys Lys Gly Pro Glu Leu Gln
        595                 600                 605

Asp Cys Leu Gly Gln Thr Leu Val Leu Ile Gly Lys Thr His Leu Thr
    610                 615                 620

Met Ala Leu Thr Val Ile Ala Gly Leu Val Val Ile Phe Met Met Leu
625                 630                 635                 640

Gly Gly Thr Phe Leu Tyr Trp Arg Gly Arg Arg Ile Gln Asn Lys Arg
                645                 650                 655

Ala Met Arg Arg Tyr Leu Glu Arg Gly Glu Ser Ile Glu Pro Leu Asp
            660                 665                 670

Pro Ser Glu Lys Ala Asn Lys Val Leu Ala Arg Ile Phe Lys Glu Thr
        675                 680                 685

Glu Leu Arg Lys Leu Lys Val Leu Gly Ser Gly Val Phe Gly Thr Val
    690                 695                 700

His Lys Gly Val Trp Ile Pro Glu Gly Glu Ser Ile Lys Ile Pro Val
705                 710                 715                 720

Cys Ile Lys Val Ile Glu Asp Lys Ser Gly Arg Gln Ser Phe Gln Ala
                725                 730                 735

Val Thr Asp His Met Leu Ala Ile Gly Ser Leu Asp His Ala His Ile
            740                 745                 750

Val Arg Leu Leu Gly Leu Cys Pro Gly Ser Ser Leu Gln Leu Val Thr
        755                 760                 765

Gln Tyr Leu Pro Leu Gly Ser Leu Leu Asp His Val Arg Gln His Arg
    770                 775                 780

Gly Ala Leu Gly Pro Gln Leu Leu Leu Asn Trp Gly Val Gln Ile Ala
785                 790                 795                 800

Lys Gly Met Tyr Tyr Leu Glu Glu His Gly Met Val His Arg Asn Leu
                805                 810                 815

Ala Ala Arg Asn Val Leu Leu Lys Ser Pro Ser Gln Val Gln Val Ala
            820                 825                 830

Asp Phe Gly Val Ala Asp Leu Leu Pro Pro Asp Asp Lys Gln Leu Leu
        835                 840                 845

Tyr Ser Glu Ala Lys Thr Pro Ile Lys Trp Met Ala Leu Glu Ser Ile
    850                 855                 860

His Phe Gly Lys Tyr Thr His Gln Ser Asp Val Trp Ser Tyr Gly Val
865                 870                 875                 880

Thr Val Trp Glu Leu Met Thr Phe Gly Ala Glu Pro Tyr Ala Gly Leu
                885                 890                 895

Arg Leu Ala Glu Val Pro Asp Leu Leu Glu Lys Gly Glu Arg Leu Ala
            900                 905                 910

Gln Pro Gln Ile Cys Thr Ile Asp Val Tyr Met Val Met Val Lys Cys
        915                 920                 925

Trp Met Ile Asp Glu Asn Ile Arg Pro Thr Phe Lys Glu Leu Ala Asn
    930                 935                 940

Glu Phe Thr Arg Met Ala Arg Asp Pro Pro Arg Tyr Leu Val Ile Lys
945                 950                 955                 960

Arg Glu Ser Gly Pro Gly Ile Ala Pro Gly Pro Glu Pro His Gly Leu
                965                 970                 975

Thr Asn Lys Lys Leu Glu Glu Val Glu Leu Glu Pro Glu Leu Asp Leu
            980                 985                 990

Asp Leu Asp Leu Glu Ala Glu Glu  Asp Asn Leu Ala Thr  Thr Thr Leu
        995                 1000                 1005

Gly Ser  Ala Leu Ser Leu Pro  Val Gly Thr Leu Asn  Arg Pro Arg
    1010                 1015                 1020

Gly Ser  Gln Ser Leu Leu Ser  Pro Ser Ser Gly Tyr  Met Pro Met
    1025                 1030                 1035

Asn Gln  Gly Asn Leu Gly Glu  Ser Cys Gln Glu Ser  Ala Val Ser
    1040                 1045                 1050

Gly Ser  Ser Glu Arg Cys Pro  Arg Pro Val Ser Leu  His Pro Met
    1055                 1060                 1065

Pro Arg  Gly Cys Leu Ala Ser  Glu Ser Ser Glu Gly  His Val Thr
    1070                 1075                 1080

Gly Ser  Glu Ala Glu Leu Gln  Glu Lys Val Ser Met  Cys Arg Ser
    1085                 1090                 1095

Arg Ser  Arg Ser Arg Ser Pro  Arg Pro Arg Gly Asp  Ser Ala Tyr
    1100                 1105                 1110

His Ser  Gln Arg His Ser Leu  Leu Thr Pro Val Thr  Pro Leu Ser
    1115                 1120                 1125

Pro Pro  Gly Leu Glu Glu Glu  Asp Val Asn Gly Tyr  Val Met Pro

1130                1135                1140

Asp Thr His Leu Lys Gly Thr Pro Ser Ser Arg Glu Gly Thr Leu
    1145                1150                1155

Ser Ser Val Gly Leu Ser Ser Val Leu Gly Thr Glu Glu Glu Asp
    1160                1165                1170

Glu Asp Glu Glu Tyr Glu Tyr Met Asn Arg Arg Arg Arg His Ser
    1175                1180                1185

Pro Pro His Pro Pro Arg Pro Ser Ser Leu Glu Glu Leu Gly Tyr
    1190                1195                1200

Glu Tyr Met Asp Val Gly Ser Asp Leu Ser Ala Ser Leu Gly Ser
    1205                1210                1215

Thr Gln Ser Cys Pro Leu His Pro Val Pro Ile Met Pro Thr Ala
    1220                1225                1230

Gly Thr Thr Pro Asp Glu Asp Tyr Glu Tyr Met Asn Arg Gln Arg
    1235                1240                1245

Asp Gly Gly Gly Pro Gly Gly Asp Tyr Ala Ala Met Gly Ala Cys
    1250                1255                1260

Pro Ala Ser Glu Gln Gly Tyr Glu Glu Met Arg Ala Phe Gln Gly
    1265                1270                1275

Pro Gly His Gln Ala Pro His Val His Tyr Ala Arg Leu Lys Thr
    1280                1285                1290

Leu Arg Ser Leu Glu Ala Thr Asp Ser Ala Phe Asp Asn Pro Asp
    1295                1300                1305

Tyr Trp His Ser Arg Leu Phe Pro Lys Ala Asn Ala Gln Arg Thr
    1310                1315                1320

<210> 42
<211> 445
<212> PRT
<213> Artificial Sequence

<220>
<221> source
<223> /note="Description of Artificial Sequence: Synthetic
      polypeptide"

<400> 42
Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser His Tyr
            20                  25                  30

Val Met Ala Trp Val Arg Gln Ala Pro Gly Lys Gly Leu Glu Trp Val
        35                  40                  45

Ser Ser Ile Ser Ser Ser Gly Gly Trp Thr Leu Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Gly Leu Lys Met Ala Thr Ile Phe Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Cys Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Met Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 43
<211> 217
<212> PRT
<213> Artificial Sequence

<220>
<221> source
<223> /note="Description of Artificial Sequence: Synthetic
      polypeptide"

<400> 43
Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Ser Tyr
            20                  25                  30

Asn Val Val Ser Trp Tyr Gln Gln His Pro Gly Lys Ala Pro Lys Leu
        35                  40                  45

Ile Ile Tyr Glu Val Ser Gln Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Thr Glu Asp Glu Ala Asp Tyr Tyr Cys Cys Ser Tyr Ala Gly Ser
                85                  90                  95

Ser Ile Phe Val Ile Phe Gly Gly Gly Thr Lys Val Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Val Ser Asp Phe
    130                 135                 140

Tyr Pro Gly Ala Val Thr Val Ala Trp Lys Ala Asp Gly Ser Pro Val
145                 150                 155                 160

Lys Val Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Arg Val Thr His Glu Gly Ser Thr Val Glu
        195                 200                 205

Lys Thr Val Ala Pro Ala Glu Cys Ser
    210                 215

<210> 44
<211> 1095
<212> PRT
<213> Homo sapiens

<400> 44
Gln Val Gln Leu Gln Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr

20                  25                  30

Trp Met Ser Trp Val Arg Gln Ala Pro Gly Lys Gly Leu Glu Trp Val
        35                  40                  45

Ala Asn Ile Asn Arg Asp Gly Ser Ala Ser Tyr Tyr Val Asp Ser Val
    50                  55                  60

Lys Gly Arg Phe Thr Ile Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Arg Gly Val Gly Tyr Phe Asp Leu Trp Gly Arg Gly Thr
            100                 105                 110

Leu Val Thr Val Ser Ser Ala Ser Thr Gly Gly Gly Gly Ser Gly Gly
        115                 120                 125

Gly Gly Ser Gly Gly Gly Gly Ser Gln Ser Ala Leu Thr Gln Pro Ala
    130                 135                 140

Ser Val Ser Gly Ser Pro Gly Gln Ser Ile Thr Ile Ser Cys Thr Gly
145                 150                 155                 160

Thr Ser Ser Asp Val Gly Gly Tyr Asn Phe Val Ser Trp Tyr Gln Gln
                165                 170                 175

His Pro Gly Lys Ala Pro Lys Leu Met Ile Tyr Asp Val Ser Asp Arg
            180                 185                 190

Pro Ser Gly Val Ser Asp Arg Phe Ser Gly Ser Lys Ser Gly Asn Thr
        195                 200                 205

Ala Ser Leu Ile Ile Ser Gly Leu Gln Ala Asp Asp Glu Ala Asp Tyr
    210                 215                 220

Tyr Cys Ser Ser Tyr Gly Ser Ser Ser Thr His Val Ile Phe Gly Gly
225                 230                 235                 240

Gly Thr Lys Val Thr Val Leu Gly Ala Ala Ser Asp Ala His Lys Ser
                245                 250                 255

Glu Val Ala His Arg Phe Lys Asp Leu Gly Glu Glu Asn Phe Lys Ala
            260                 265                 270

Leu Val Leu Ile Ala Phe Ala Gln Tyr Leu Gln Gln Ser Pro Phe Glu
        275                 280                 285

Asp His Val Lys Leu Val Asn Glu Val Thr Glu Phe Ala Lys Thr Cys
    290                 295                 300

Val Ala Asp Glu Ser Ala Glu Asn Cys Asp Lys Ser Leu His Thr Leu
305                 310                 315                 320

Phe Gly Asp Lys Leu Cys Thr Val Ala Thr Leu Arg Glu Thr Tyr Gly
                325                 330                 335

Glu Met Ala Asp Cys Cys Ala Lys Gln Glu Pro Glu Arg Asn Glu Cys
            340                 345                 350

Phe Leu Gln His Lys Asp Asp Asn Pro Asn Leu Pro Arg Leu Val Arg
        355                 360                 365

Pro Glu Val Asp Val Met Cys Thr Ala Phe His Asp Asn Glu Glu Thr
    370                 375                 380

Phe Leu Lys Lys Tyr Leu Tyr Glu Ile Ala Arg Arg His Pro Tyr Phe
385                 390                 395                 400

Tyr Ala Pro Glu Leu Leu Phe Phe Ala Lys Arg Tyr Lys Ala Ala Phe
                405                 410                 415

Thr Glu Cys Cys Gln Ala Ala Asp Lys Ala Ala Cys Leu Leu Pro Lys
            420                 425                 430

Leu Asp Glu Leu Arg Asp Glu Gly Lys Ala Ser Ser Ala Lys Gln Arg
        435                 440                 445

Leu Lys Cys Ala Ser Leu Gln Lys Phe Gly Glu Arg Ala Phe Lys Ala
    450                 455                 460

Trp Ala Val Ala Arg Leu Ser Gln Arg Phe Pro Lys Ala Glu Phe Ala
465                 470                 475                 480

Glu Val Ser Lys Leu Val Thr Asp Leu Thr Lys Val His Thr Glu Cys
                485                 490                 495

Cys His Gly Asp Leu Leu Glu Cys Ala Asp Asp Arg Ala Asp Leu Ala
            500                 505                 510

Lys Tyr Ile Cys Glu Asn Gln Asp Ser Ile Ser Ser Lys Leu Lys Glu
        515                 520                 525

Cys Cys Glu Lys Pro Leu Leu Glu Lys Ser His Cys Ile Ala Glu Val
    530                 535                 540

Glu Asn Asp Glu Met Pro Ala Asp Leu Pro Ser Leu Ala Ala Asp Phe
545                 550                 555                 560

Val Glu Ser Lys Asp Val Cys Lys Asn Tyr Ala Glu Ala Lys Asp Val
                565                 570                 575

Phe Leu Gly Met Phe Leu Tyr Glu Tyr Ala Arg Arg His Pro Asp Tyr
            580                 585                 590

Ser Val Val Leu Leu Leu Arg Leu Ala Lys Thr Tyr Glu Thr Thr Leu
        595                 600                 605

Glu Lys Cys Cys Ala Ala Ala Asp Pro His Glu Cys Tyr Ala Lys Val
    610                 615                 620

Phe Asp Glu Phe Lys Pro Leu Val Glu Glu Pro Gln Asn Leu Ile Lys
625                 630                 635                 640

Gln Asn Cys Glu Leu Phe Glu Gln Leu Gly Glu Tyr Lys Phe Gln Asn
                645                 650                 655

Ala Leu Leu Val Arg Tyr Thr Lys Lys Val Pro Gln Val Ser Thr Pro
            660                 665                 670

Thr Leu Val Glu Val Ser Arg Asn Leu Gly Lys Val Gly Ser Lys Cys
        675                 680                 685

Cys Lys His Pro Glu Ala Lys Arg Met Pro Cys Ala Glu Asp Tyr Leu
    690                 695                 700

Ser Val Val Leu Asn Gln Leu Cys Val Leu His Glu Lys Thr Pro Val
705                 710                 715                 720

Ser Asp Arg Val Thr Lys Cys Cys Thr Glu Ser Leu Val Asn Arg Arg
                725                 730                 735

Pro Cys Phe Ser Ala Leu Glu Val Asp Glu Thr Tyr Val Pro Lys Glu
            740                 745                 750

Phe Gln Ala Glu Thr Phe Thr Phe His Ala Asp Ile Cys Thr Leu Ser
        755                 760                 765

Glu Lys Glu Arg Gln Ile Lys Lys Gln Thr Ala Leu Val Glu Leu Val
    770                 775                 780

Lys His Lys Pro Lys Ala Thr Lys Glu Gln Leu Lys Ala Val Met Asp
785                 790                 795                 800

Asp Phe Ala Ala Phe Val Glu Lys Cys Cys Lys Ala Asp Asp Lys Glu
                805                 810                 815

Thr Cys Phe Ala Glu Glu Gly Lys Lys Leu Val Ala Ala Ser Gln Ala
            820                 825                 830

Ala Leu Gly Leu Ala Ala Ala Leu Gln Val Gln Leu Val Gln Ser Gly
        835                 840                 845

Ala Glu Val Lys Lys Pro Gly Glu Ser Leu Lys Ile Ser Cys Lys Gly
    850                 855                 860

Ser Gly Tyr Ser Phe Thr Ser Tyr Trp Ile Ala Trp Val Arg Gln Met
865                 870                 875                 880

Pro Gly Lys Gly Leu Glu Tyr Met Gly Leu Ile Tyr Pro Gly Asp Ser
                885                 890                 895

Asp Thr Lys Tyr Ser Pro Ser Phe Gln Gly Gln Val Thr Ile Ser Val
            900                 905                 910

Asp Lys Ser Val Ser Thr Ala Tyr Leu Gln Trp Ser Ser Leu Lys Pro
        915                 920                 925

Ser Asp Ser Ala Val Tyr Phe Cys Ala Arg His Asp Val Gly Tyr Cys
    930                 935                 940

Thr Asp Arg Thr Cys Ala Lys Trp Pro Glu Trp Leu Gly Val Trp Gly
945                 950                 955                 960

Gln Gly Thr Leu Val Thr Val Ser Ser Gly Gly Gly Gly Ser Ser Gly
                965                 970                 975

Gly Gly Ser Gly Gly Gly Gly Ser Gln Ser Val Leu Thr Gln Pro Pro
            980                 985                 990

Ser Val Ser Ala Ala Pro Gly Gln  Lys Val Thr Ile Ser  Cys Ser Gly
        995                 1000                1005

Ser Ser  Ser Asn Ile Gly Asn  Asn Tyr Val Ser Trp  Tyr Gln Gln
    1010                1015                1020

Leu Pro  Gly Thr Ala Pro Lys  Leu Leu Ile Tyr Asp  His Thr Asn

1025                 1030                 1035

Arg Pro Ala Gly Val Pro Asp Arg Phe Ser Gly Ser Lys Ser Gly
    1040                 1045                 1050

Thr Ser Ala Ser Leu Ala Ile Ser Gly Phe Arg Ser Glu Asp Glu
    1055                 1060                 1065

Ala Asp Tyr Tyr Cys Ala Ser Trp Asp Tyr Thr Leu Ser Gly Trp
    1070                 1075                 1080

Val Phe Gly Gly Gly Thr Lys Leu Thr Val Leu Gly
    1085                 1090                 1095

<210> 45
<211> 357
<212> DNA
<213> Artificial Sequence

<220>
<221> source
<223> /note="Description of Artificial Sequence: Synthetic
      polynucleotide"

<400> 45
gaggtgcagc tgctggagag cggcggaggg ctggtccagc caggcggcag cctgaggctg      60

tcctgcgccg ccagcggctt caccttcagc cactacgtga tggcctgggt gcggcaggcc     120

ccaggcaagg gcctggaatg ggtgtccagc atcagcagca gcggcggctg gaccctgtac     180

gccgacagcg tgaagggcag gttcaccatc agcagggaca acagcaagaa caccctgtac     240

ctgcagatga acagcctgag ggccgaggac accgccgtgt actactgcac caggggcctg     300

aagatggcca ccatcttcga ctactggggc cagggcaccc tggtgaccgt gagcagc        357

<210> 46
<211> 333
<212> DNA
<213> Artificial Sequence

<220>
<221> source
<223> /note="Description of Artificial Sequence: Synthetic
      polynucleotide"

<400> 46
cagtccgccc tgacccagcc cgccagcgtg agcggcagcc caggccagag catcaccatc      60

agctgcaccg gcaccagcag cgacgtgggc agctacaacg tggtgtcctg gtatcagcag     120

caccccggca aggcccccaa gctgatcatc tacgaggtgt cccagaggcc cagcggcgtg     180

agcaacaggt tcagcggcag caagagcggc aacaccgcca gcctgaccat cagcggcctg     240

cagaccgagg acgaggccga ctactactgc tgcagctacg ccggcagcag catcttcgtg     300

atcttcggcg gagggaccaa ggtgaccgtc cta 333